# EUROPEAN PATENT SPECIFICATION

(11) **EP 3 897 845 B1**
(45) Date of publication and mention of the grant of the patent: **14.01.2026**
(21) Application number: 19818165.3
(22) Date of filing: 17.12.2019
(51) Int. Cl.: A61P 35/00, C07K 16/28, C07K 19/00

(54) **BIFUNCTIONAL MOLECULE DIRECTED AGAINST HUMAN PD-1**
BIFUNKTIONELLES, GEGEN HUMAN PD-1 GERICHTETES MOLEKÜL
MOLÉCULE BIFONCTIONNELLE DIRIGÉE CONTRE LE PD-1 HUMAIN

(30) Priority: 21.12.2018 EP 18306799
(43) Date of publication of application: 27.10.2021
(73) Proprietor: OSE Immunotherapeutics, 44200 Nantes (FR)
(72) Inventor: POIRIER, Nicolas, 44200 Nantes (FR); MARY, Caroline, 44200 Nantes (FR); THEPENIER, Virginie, 44200 Nantes (FR); MORELLO, Aurore, 44200 Nantes (FR)
(74) Representative: Cabinet Becker et Associés
(86) International application number: PCT/EP2019/085781
(87) International publication number: WO 2020/127369

(56) References cited:
- WO-A1-2017/025498
- WO-A1-2018/184964
- WO-A2-2017/134302
- CN-A- 105 111 314
- CN-A- 108 250 303
- CYNTHIA CHALLENER: "Fusion Proteins Pose Manufacturability Challenges", BIOPHARMA INTERNATIONAL, vol. 30, no. 5, 1 May 2017 (2017-05-01), pages 31 - 31, XP055593788

## Description

### FIELD OF THE INVENTION

The invention pertains to the field of immunotherapy. The present invention provides a bifunctional molecule that comprises a humanized anti-PD1 antibody linked to an immunotherapeutic agent and uses thereof.

### BACKGROUND OF THE INVENTION

The approach of targeting T cell inhibition checkpoints for dis-inhibition with therapeutic antibodies is an area of intense investigation (for a review, see Pardoll, Nat Rev Cancer. 2012; 12:253-264). Targeting immune checkpoints of the adaptive immunity has shown great therapeutic efficacy to fight numerous cancers, but in a limited proportion of patients. Immune checkpoint on innate myeloid cells (macrophages, dendritic cells, MDSC, PMN) remain poorly studied while these cells represent the most abundant immune cell type in many solid tumors and are often associated with a poor outcome. Combining immune checkpoint therapies targeting both innate (mediated by myeloid cells) and adaptive (mediated by T cells) immune responses has demonstrated great efficiency in preclinical models but remains a challenge in the clinic.

Immune cells activation is governed by the integration of balance co-stimulatory and co-inhibitory signals. T cell receptor (TCR)-mediated T cell activation is modulated by both co-stimulatory and co-inhibitory signals. The antigen-independent second signal modifies first signal, provided by interaction of antigenic peptide-MHC complex with the TCR, which confers specificity to the response. T cell co-stimulatory and co-inhibitory pathways have a broad immunoregulatory functions, controlling effector, memory and regulatory T cells, as well as naive T cells. Therapeutic modulation of those pathways is translating to effective new strategies for treating cancer (For review, see Schildberg et al., 44(5), Immunity, 2016). Ongoing studies on regulation of the immune responses have led to the identification of multiple immunologic pathways that may be targeted for the development of cancer therapies. Those molecules are referred herein as immune checkpoint co-activators or co-inhibitors (see review Sharma et al., Cell, 161(2), 2015 and Pardoll, Nature Reviews Cancer, 12(4), 2012).

Programmed cell death protein 1 (PD-1, also known as CD279) is a cell surface protein molecule that belongs to the immunoglobulin superfamily. It is expressed on T and B lymphocytes and macrophages, and plays a role in cell fate and differentiation. Particularly, PD-1, functioning as an immune checkpoint, plays an important role in down-regulating the immune system by preventing the activation of T-cells, which in turn reduces autoimmunity and promotes self-tolerance. Two ligands for PD-1 have been identified, PD-L1 and PD-L2, that have been shown to downregulate T cell activation upon binding to PD-1 (Freeman et al. (2000) J Exp Med 192: 1027-34; Latchman et al. (2001) Nat Immunol 2:261-8; Carter et al. (2002) Eur J Immunol 32:634-43). The interaction between PD-1 and its ligand results in a decrease in tumor infiltrating lymphocytes, a decrease in T-cell receptor mediated proliferation, and immune evasion by the cancerous cells. Particularly, PD1 ligation reduces signals downstream of TCR stimulation on T cells, inhibiting T cell response and resulting in decreased activation and cytokine production.

Both strategies using anti-PD1 and anti-PDL1 inhibitor to disrupt their interaction was a success in cancer therapy (Brahmer et al., N Eng J Med, 366(26), 2012; Powles et al., Nature, 515(7528), 2014; Topalian et al., N Eng J Med, 366(26), 2012; Ansell, Curr Opin Hematol, 22(4), 2015). However, the accumulation of immunosuppressive and hypo-stimulatory myeloid cells within tumor microenvironment limits the efficiency of T-cell responses and the efficacy of immunotherapies, in particular those targeting at immune checkpoint such as PD-1/PD-L1. In parallel, immunotherapies targeting innate immune checkpoint have shown limited efficacy alone, since T-cell responses remained blocked mainly by absence of co-stimulation within tumor-microenvironment and/or the engagement of co-inhibitory molecules with the ligand expressed by tumor cells or antigen-presenting cells. Combining immunotherapies targeting at both immune checkpoint of adaptive (T-cells) and innate (myeloid cells) cells have demonstrated potent efficacy at preclinical levels.

Bifunctional molecules have been disclosed in the art. WO2017/025498 discloses fusion polypeptides binding to PD-1 and LAG-3. WO2018/184964 discloses immunoconjugates of an anti-PD-1 antibody with a mutant IL-2 or with IL-15.

However, the validation and development of combined immunotherapies are strongly limited by the cost of biotherapies and the limited access to such immunotherapies. There remains therefore a significant need in the art for new and improved agents for safe immunotherapy, notably against cancer, targeting innate myeloid immune cells with an effective positive impact on adaptive immune response, in particular T cell immune responses. The present inventors have made a significant step forward with the invention disclosed herein.

### SUMMARY OF THE INVENTION

The inventors provide a bifunctional molecule comprising a humanized anti-hPD-1 antibody and an immunotherapeutic agent promising for numerous therapeutic applications, in particular for the treatment of cancer. The present invention is based on the development of a humanized antibody specifically targeting human PD-1 which shows high binding affinity to PD-1 and strong competition with its ligands PD-L1 and PD-L2. Surprisingly, this humanized antibody presents a high humanness score and allows high production yields when produced as a bifunctional molecule bound to an immunotherapeutic agent. Indeed, it has been engineered to present a high manufacturability in mammalian cell-based production systems, particularly when fused with an active protein domain in the C-terminal end of its heavy or light chains, a situation usually associated with poor manufacturability.

The object of the present invention is defined by the claims.

In a first aspect, the bifunctional molecule comprises or consists of:
(a) a humanized anti-human PD-1 antibody, which comprises:
   (i) a heavy chain variable domain (VH) comprising or consisting of an amino acid sequence selected from the group consisting of SEQ ID NO: 19, 22 or 24, and (ii) a light chain variable domain (VL) comprising or consisting of an amino acid sequence of SEQ ID NO: 28,
   and
(b) an immunotherapeutic agent or a fragment thereof, wherein the immunotherapeutic agent or a fragment thereof is a peptide, a polypeptide or a protein and has a size comprised between 10 kDa and 50 kDa; wherein the immunotherapeutic agent or fragment thereof is selected from the list consisting of cytokines, cytokines receptors, chemokines, chemokines receptors, and human transmembrane immune protein of type I or II, preferably the extracellular domain thereof,
wherein the C-terminal end of the heavy and/or light chain(s) of the antibody is covalently linked to the N-terminal end of the immunotherapeutic agent as a fusion protein, preferably by a peptide linker. Preferably, the antibody is an antagonist of the binding of human PDL-1 and/or PD-L2 to human PD1. In a particular aspect, the immunotherapeutic agent is a human transmembrane immune protein of type I or a fragment thereof, selected from the group consisting of ICOSL, CD86, B7H4, B7H3, CD28H, PDL2, PDL1, DNAM, CTLA-4, Lag-3, TIGIT, 2B4, BTLA, HVEM, CD101, nectin-1, nectin-2, nectin-3, NELC-5, TLT-2, LFA-3, TIM3, TIM4, LAIR1, SIRPG, IL10R, IL6RA, IL-1R1, IL-1RAcP, IL6RB, TGFBRII, CSF1R, IL22R, VEGFR1, VEGFR2, VEGFR3, CD111, CD112, CD155, CD113, VISTA, CD244, OX40, SIRPalpha, CD80, CD24, Siglec-10, Fas, IL15RA, SIRB1, SIRB2, LTBR, IL21R and GITR.

In another aspect, the immunotherapeutic agent is a human transmembrane immune protein of type II or a fragment thereof, selected from the group consisting of CD40L, OX40L, FasL, TRAIL, TNF, LIGHT, APRIL, GITRL, CD30, CD70, CD40, CD27, CD30, CD153, RANK, CD96, CLEC1, CLEC2 /CLE1B, CLEC3A, CLEC4A, CLEC4E, CLEC4L, CLEC51, CLEC6, CLEC7A, NKG2D, BTL-II, TGFRII, DECTIN-1, DC-SIGN, LT-alpha, LT-beta, 4-1BBL and MINCLE, preferably a member of the TNF family.

In another aspect, the immunotherapeutic agent is a cytokine or a fragment thereof selected from the group consisting of TGFβ, IL-1, IL-2, IL-6, IL-7, IL-10, IL-12A, IL12B, IL-15, IL-21, IL-4 and IL-18.

In a very particular aspect, the immunotherapeutic agent is a human IL-2 or a mutant thereof, in particular an IL-2 mutant having the sequence as set forth in SEQ ID NO: 58 with the substitutions F42A, Y45A and L72G, preferably T3A, F42A, Y45A, L72G and C125A.

In a particular aspect, the antibody comprises a light chain constant domain derived from a human kappa light chain constant domain and a heavy chain constant domain derived from a human IgG1, IgG2, IgG3 or IgG4 heavy chain constant domain.

In a more specific aspect, the antibody comprises a light chain constant domain derived from a human kappa light chain constant domain and a heavy chain constant domain derived from a human IgG1 heavy chain constant domain, optionally with a substitution or a combination of substitutions selected from the group consisting of T250Q/M428L; M252Y/S254T/T256E + H433K/N434F; E233P/L234V/L235A/G236A + A327G/A330S/P331S; E333A; S239D/A330L/1332E; P257I/Q311; K326W/E333S; S239D/I332E/G236A; N297A; L234A/L235A; N297A + M252Y/S254T/T256E; K444A, and K322A, preferably selected from the group consisting of N297A optionally in combination with M252Y/S254T/T256E, and L234A/L235A, wherein the numbering of the residues is according to EU numbering.

In another more specific aspect, the antibody comprises a light chain constant domain derived from a human kappa light chain constant domain and a heavy chain constant domain derived from a human IgG4 heavy chain constant domain, optionally with a substitution or a combination of substitutions selected from the group consisting of S228P; L234A/L235A, S228P + M252Y/S254T/T256E, and K444A, wherein the numbering of the residues is according to EU numbering.

The invention also concerns an isolated nucleic acid sequence or a group of isolated nucleic acid molecules encoding the bifunctional molecule as disclosed herein, a vector, comprising the nucleic acid or group of nucleic acid molecules, and a host cell, comprising the vector or the nucleic acid or group of nucleic acid molecules disclosed herein.

In one aspect, the invention relates to a method for producing the bifunctional molecule, comprising a step of culturing a host cell as disclosed herein and optionally a step of isolating the bifunctional molecule.

In another aspect, the invention concerns a pharmaceutical composition comprising the bifunctional molecule, the nucleic acid or group of nucleic acid molecules, the vector as disclosed herein and a pharmaceutically acceptable carrier.

Optionally, the pharmaceutical composition further comprises an additional therapeutic agent, preferably selected in the group consisting of alkylating agents, angiogenesis inhibitors, antibodies, antimetabolites, antimitotics, antiproliferatives, antivirals, aurora kinase inhibitors, apoptosis promoters (for example, Bcl-2 family inhibitors), activators of death receptor pathway, Bcr-Abl kinase inhibitors, BiTE (Bi-Specific T cell Engager) antibodies, antibody drug conjugates, biologic response modifiers, Bruton's tyrosine kinase (BTK) inhibitors, cyclin-dependent kinase inhibitors, cell cycle inhibitors, cyclooxygenase-2 inhibitors, DVDs, leukemia viral oncogene homolog (ErbB2) receptor inhibitors, growth factor inhibitors, heat shock protein (HSP)-90 inhibitors, histone deacetylase (HDAC) inhibitors, hormonal therapies, immunologicals, inhibitors of inhibitors of apoptosis proteins (IAPs), intercalating antibiotics, kinase inhibitors, kinesin inhibitors, Jak2 inhibitors, mammalian target of rapamycin inhibitors, microRNAs, mitogen-activated extracellular signal-regulated kinase inhibitors, multivalent binding proteins, non-steroidal anti-inflammatory drugs (NSAIDs), poly ADP (adenosine diphosphate)-ribose polymerase (PARP) inhibitors, platinum chemotherapeutics, polo-like kinase (Plk) inhibitors, phosphoinositide-3 kinase (PI3K) inhibitors, proteasome inhibitors, purine analogs, pyrimidine analogs, receptor tyrosine kinase inhibitors, retinoids/deltoids plant alkaloids, small inhibitory ribonucleic acids (siRNAs), topoisomerase inhibitors, ubiquitin ligase inhibitors, hypomethylating agents, checkpoints inhibitors, peptide vaccine and the like, epitopes or neoepitopes from tumor antigens, as well as combinations of one or more of these agents.

The invention finally relates to a pharmaceutical composition, a bifunctional molecule, a nucleic acid or group of nucleic acid molecules, a vector, or a host cell as disclosed herein for use as a medicament. In a particular aspect, the pharmaceutical composition, bifunctional molecule, nucleic acid or group of nucleic acid molecules, vector, or host cell as disclosed herein is for use in the treatment of cancer. Preferably, the cancer is selected from the group consisting of a hematologic malignancy or a solid tumor with expression of PD-1 and/or PD-L1 such as a cancer selected from the group consisting of hematolymphoid neoplasms, angioimmunoblastic T cell lymphoma, myelodysplastic syndrome, and acute myeloid leukemia, a cancer induced by virus or associated with immunodeficiency such as a cancer selected from the group consisting of Kaposi sarcoma (e.g., associated with Kaposi sarcoma herpes virus); cervical, anal, penile and vulvar squamous cell cancer and oropharyngeal cancers (e.g., associated with human papilloma virus); B cell non-Hodgkin lymphomas (NHL) including diffuse large B-cell lymphoma, Burkitt lymphoma, plasmablastic lymphoma, primary central nervous system lymphoma, HHV-8 primary effusion lymphoma, classic Hodgkin lymphoma, and lymphoproliferative disorders (e.g., associated with Epstein-Barr virus (EBV) and/or Kaposi sarcoma herpes virus); hepatocellular carcinoma (e.g., associated with hepatitis B and/or C viruses); Merkel cell carcinoma (e.g., associated with Merkel cell polyoma virus (MPV)); and cancer associated with human immunodeficiency virus infection (HIV) infection, and a cancer selected from the group consisting of metastatic or not metastatic, Melanoma, malignant mesothelioma, Non-Small Cell Lung Cancer, Renal Cell Carcinoma, Hodgkin's Lymphoma, Head and Neck Cancer, Urothelial Carcinoma, Colorectal Cancer, Hepatocellular Carcinoma, Small Cell Lung Cancer, Metastatic Merkel Cell Carcinoma, Gastric or Gastroesophageal cancers and Cervical Cancer.

In another particular aspect, the pharmaceutical composition, bifunctional molecule, nucleic acid or group of nucleic acid molecules, vector, or host cell as disclosed herein is for use for treating infectious disease, preferably chronic infectious disease, even more preferably chronic viral infections, preferably caused by a virus selected from the group consisting of HIV, hepatitis virus, herpes virus, adenovirus, influenza virus, flaviviruses, echovirus, rhinovirus, coxsackie virus, coronavirus, respiratory syncytial virus, mumps virus, rotavirus, measles virus, rubella virus, parvovirus, vaccinia virus, HTLV virus, dengue virus, papillomavirus, molluscum virus, poliovirus, rabies virus, JC virus and arboviral encephalitis virus.

Optionally, the bifunctional molecule, the pharmaceutical composition, the isolated nucleic acid molecule or the group of isolated nucleic acid molecules, the vector, or the host cell is for use in combination with radiotherapy or an additional therapeutic agent, preferably selected in the group consisting of alkylating agents, angiogenesis inhibitors, antibodies, antimetabolites, antimitotics, antiproliferatives, antivirals, aurora kinase inhibitors, apoptosis promoters (for example, Bcl-2 family inhibitors), activators of death receptor pathway, Bcr-Abl kinase inhibitors, BiTE (Bi-Specific T cell Engager) antibodies, antibody drug conjugates, biologic response modifiers, Bruton's tyrosine kinase (BTK) inhibitors, cyclin-dependent kinase inhibitors, cell cycle inhibitors, cyclooxygenase-2 inhibitors, DVDs, leukemia viral oncogene homolog (ErbB2) receptor inhibitors, growth factor inhibitors, heat shock protein (HSP)-90 inhibitors, histone deacetylase (HDAC) inhibitors, hormonal therapies, immunologicals, inhibitors of inhibitors of apoptosis proteins (IAPs), intercalating antibiotics, kinase inhibitors, kinesin inhibitors, Jak2 inhibitors, mammalian target of rapamycin inhibitors, microRNAs, mitogen-activated extracellular signal-regulated kinase inhibitors, multivalent binding proteins, non-steroidal anti-inflammatory drugs (NSAIDs), poly ADP (adenosine diphosphate)-ribose polymerase (PARP) inhibitors, platinum chemotherapeutics, polo-like kinase (Plk) inhibitors, phosphoinositide-3 kinase (PI3K) inhibitors, proteasome inhibitors, purine analogs, pyrimidine analogs, receptor tyrosine kinase inhibitors, retinoids/deltoids plant alkaloids, small inhibitory ribonucleic acids (siRNAs), topoisomerase inhibitors, ubiquitin ligase inhibitors, hypomethylating agents, checkpoints inhibitors, peptide vaccine and the like, epitopes or neoepitopes from tumor antigens, as well as combinations of one or more of these agents.

### BRIEF DESCRIPTION OF THE DRAWINGS

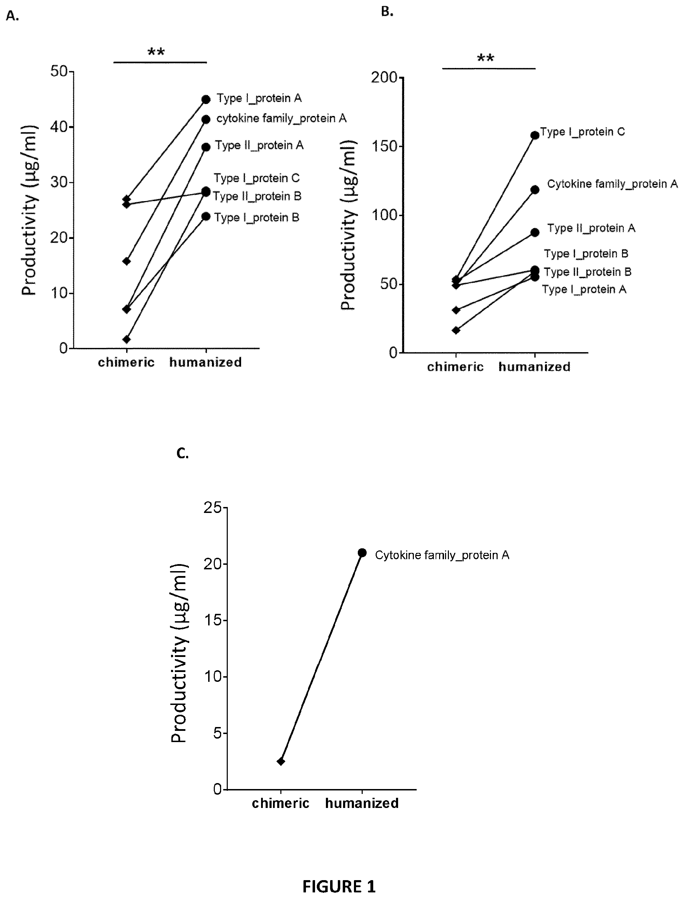

**Figure 1****: Productivity of chimeric versus humanized anti PD1 Bifunctional antibodies fused to a Type I Ig-like, Type II TNF family or cytokine protein in the C-terminal of the heavy chains (A), of the light chains (B) or both heavy and light chains (C).** HEK freestyle were transiently transfected with lipofectamine and DNA plasmid encoding for humanized or chimeric form of bifunctional antibodies. Supernatants containing the antibodies were harvested Day 3 following transfection and concentration was measured by sandwich ELISA using an anti-human IgG Fc as capture antibody and an anti-human IgK for detection. Mann Whitney test was used for statistical analysis **p<0,05. As example, in this figure, the cytokine fused to anti PD-1 antibody was an IL-7; the type I protein fused protein A corresponds to CD86, protein B corresponds to CD80 ; protein C corresponds to SIRPa (i.e., SIRPalpha); type II protein A corresponds to OX40L and protein B corresponds to 4-1BBL. In this experiment, the bifunctional molecule comprises a humanized anti-PD1 antibody having a heavy chain variable domain as disclosed in SEQ ID NO:19 and a light chain variable domain as disclosed in SEQ ID NO:28.

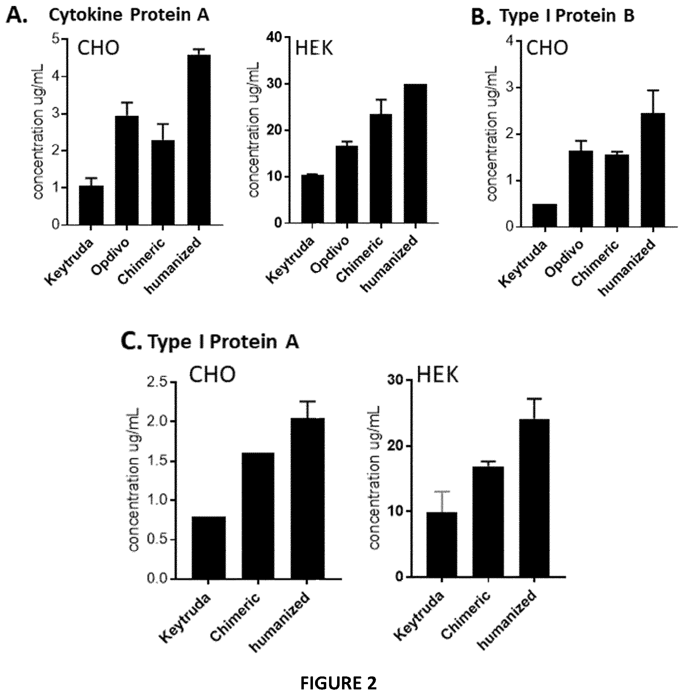

**Figure 2****: Productivity of bifunctional proteins with chimeric, humanized or other anti PD-1 backbones.** HEK freestyle or adherent CHO cells were transiently transfected with DNA plasmid encoding for humanized or chimeric form of anti PD-1 antibody of the present invention or nivol²mab or pembrolizumab anti PD-1 sequences fused in the C-terminal of the heavy chain to a cytokine or type I protein. Shaking-flask or 12-well plate was used for the HEK or CHO production respectively. Supernatants containing the antibodies were harvested Day 3 following transfection and concentration was measured by sandwich ELISA using an anti-human IgG Fc as capture antibody and an anti-human IgK for detection. **Figure 2A****:** Productivity of the bifunctional anti PD-1 fused to the IL-7 cytokine. **Figure 2B****:** Productivity of the bifunctional anti PD-1 fused to the CD80 protein I fused protein. **Figure 2C****:** Productivity of the bifunctional anti PD-1 fused to the SIRPa protein I fused protein. In this experiment, the bifunctional molecule comprises a humanized anti-PD1 antibody having a heavy chain variable domain as disclosed in SEQ ID NO:24 and a light chain variable domain as disclosed in SEQ ID NO:28.

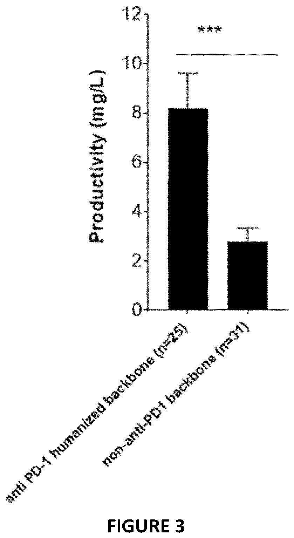

**Figure 3****: Productivity of bifunctional proteins with humanized anti PD-1 backbone versus bifunctional protein with other non anti PD-1 backbone.** HEK freestyle were transiently transfected with DNA plasmid encoding for multiple bifunctional proteins with humanized anti PD-1 backbone or non-anti-PD-1 backbone. Supernatants containing the antibodies were harvested Day 3 following transfection and concentration was measured by sandwich ELISA using an anti-human IgG Fc as capture antibody and an anti-human IgK for detection. In this experiment, the bifunctional molecule comprises a humanized anti-PD1 antibody having a heavy chain variable domain as disclosed in SEQ ID NO:19, 22 or 24 and a light chain variable domain as disclosed in SEQ ID NO:28.

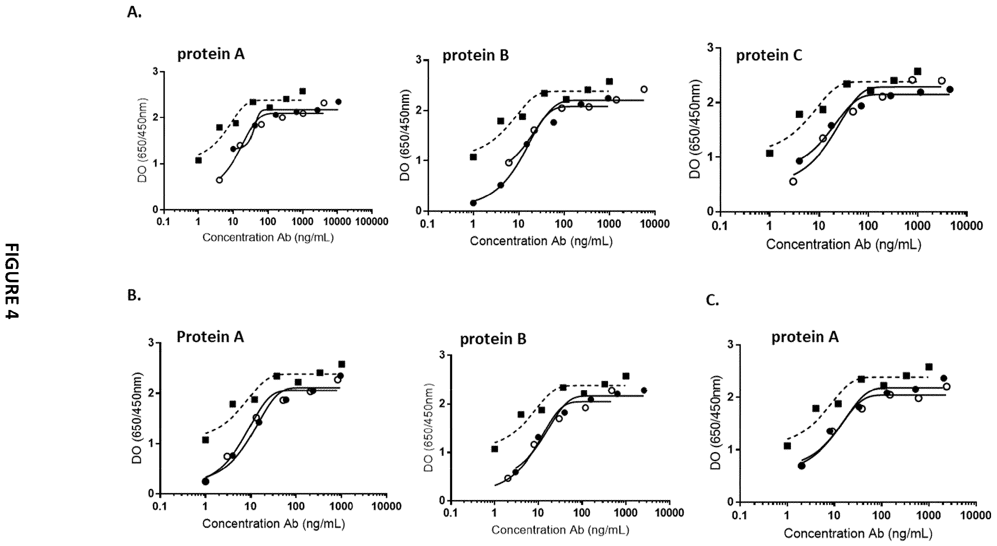

**Figure 4****: PD-1 binding ELISA assay.** Human recombinant PD-1 protein was immobilized and anti-PD-1 bifunctional antibodies were added at different concentrations. Revelation was performed with an anti-human Fc antibody coupled to peroxidase. Colorimetry was determined at 450nm using TMB substrate. **(A)** Data of anti PD-1 chimeric antibodies unfused (■) or fused to 3 Type I Ig like family proteins A, B or C on the VL domain (o) or VH domain (e); (B) Data of anti PD-1 chimeric antibodies unfused (■) or fused to 2 Type II TNF family proteins A, B or C on the VL domain (o) or VH domain (•);.**(C)** Data of anti PD-1 chimeric antibodies unfused (■) or fused to cytokine family protein A on the VL domain (o) or VH domain (e). In this figure, the cytokine fused to anti PD-1 antibody is an IL-7; the type I protein fused protein A corresponds to CD86, protein B corresponds to CD80; protein C corresponds to SIRPa; type II protein A corresponds to OX40L and protein B corresponds to 4-1BBL. In this experiment, the bifunctional molecule comprises a humanized anti-PD1 antibody having a heavy chain variable domain as disclosed in SEQ ID NO:19 and a light chain as disclosed in SEQ ID NO:28.

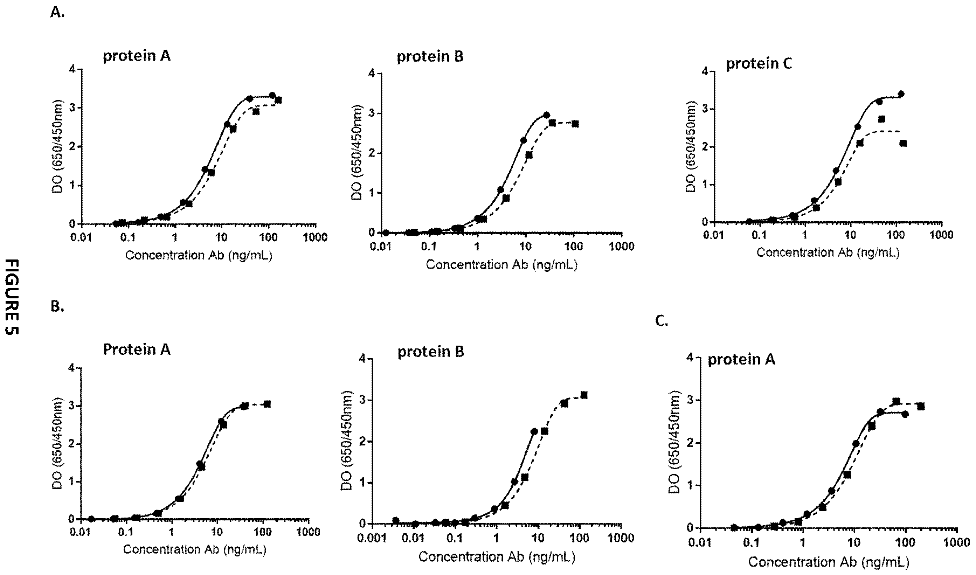

**Figure 5****: PD-1 binding ELISA assay of chimeric vs humanized bifunctional Anti-PD-1 antibodies fused to the heavy chains with type I (A) type II (B) and cytokine (C) proteins.** Human recombinant PD-1 protein was immobilized and anti-PD-1 bifunctional antibodies were added at different concentrations. Revelation was performed with an anti-human Fc antibody coupled to peroxidase. Colorimetry was determined at 450nm using TMB substrate. **(A)** Data of anti PD-1 chimeric (•) versus humanized (■) antibodies fused to 3 Type I Ig like family proteins A, B or C; **(B)** Data of anti PD-1 chimeric (•) versus humanized (■) antibodies fused to Type II TNF family proteins A or B. **(C)** Data of anti PD-1 chimeric (•) versus humanized (■) antibodies fused to cytokine family protein A. In this figure, the cytokine fused to anti PD-1 antibody was an IL-7; the type I protein fused protein A corresponds to CD86, protein B corresponds to CD80; protein C corresponds to SIRPa; type II protein A corresponds to OX40L and protein B corresponds to 4-1BBL. In this experiment, the bifunctional molecule comprises a humanized anti-PD1 antibody having a heavy chain variable domain as disclosed in SEQ ID NO:19 and a light chain variable domain as disclosed in SEQ ID NO:28.

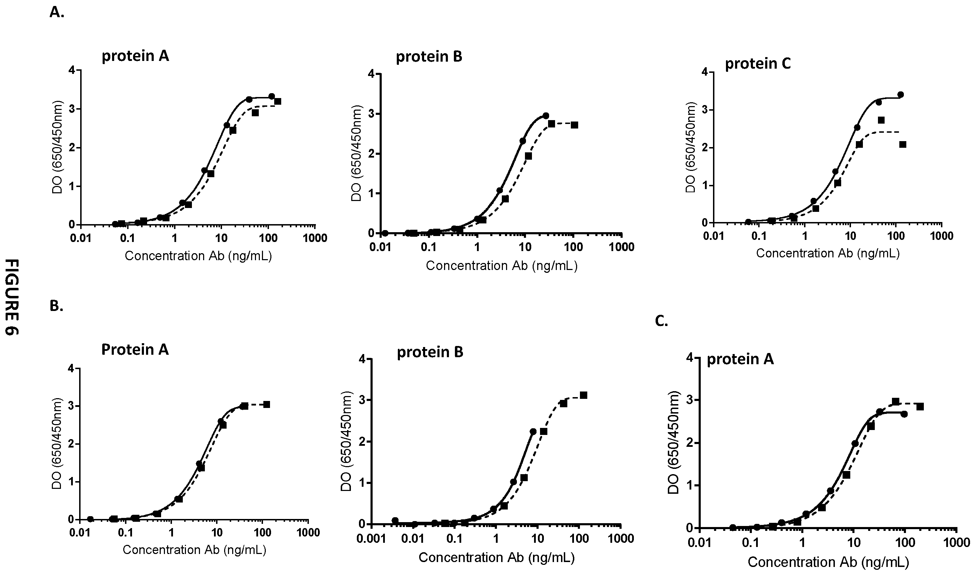

**Figure 6****: PD-1 binding ELISA assay of chimeric vs humanized bifunctional Anti-PD-1 antibodies fused to the light chains with type I (A) type II (B) and cytokine (C) proteins.** Human recombinant PD-1 protein was immobilized and anti-PD-1 bifunctional antibodies were added at different concentrations. Revelation was performed with an anti-human Fc antibody coupled to peroxidase. Colorimetry was determined at 450nm using TMB substrate. (A) Data of anti PD-1 chimeric (•) versus humanized (■) antibodies fused to 3 Type I Ig like family proteins A, B or C; (B) Data of anti PD-1 chimeric (•) versus humanized (■) antibodies fused to Type II TNF family proteins A or B. (C) Data of anti PD-1 chimeric (•) versus humanized (■) antibodies fused to cytokine family protein A. In this figure, the cytokine fused to anti PD-1 antibody is an IL-7; the type I protein fused protein A corresponds to CD86, protein B corresponds to CD80; protein C corresponds to SIRPa; type II protein A corresponds to OX40L and protein B corresponds to 4-1BBL. In this experiment, the bifunctional molecule comprises a humanized anti-PD1 antibody having a heavy chain variable domain as disclosed in SEQ ID NO:19 and a light chain variable domain as disclosed in SEQ ID NO:28.

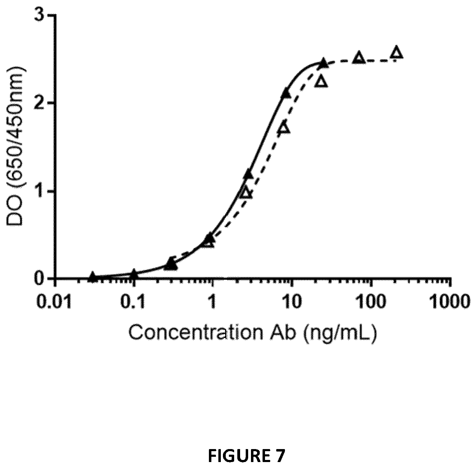

**Figure 7****: PD-1 binding ELISA assay of chimeric vs humanized bifunctional Anti-PD-1 antibodies fused to both heavy and light chains with cytokine protein A.** Human recombinant PD-1 protein was immobilized and anti-PD-1 Ab chimeric (▲) versus humanized (△) fused to cytokine A (IL-7) of both its heavy and lights chains were added at different concentrations. Revelation was performed with an anti-human Fc antibody coupled to peroxidase. Colorimetry was determined at 450nm using TMB substrate. In this experiment, the bifunctional molecule comprises a humanized anti-PD1 antibody having a heavy chain variable domain as disclosed in SEQ ID NO:19 and a light chain variable domain as disclosed in SEQ ID NO:28.

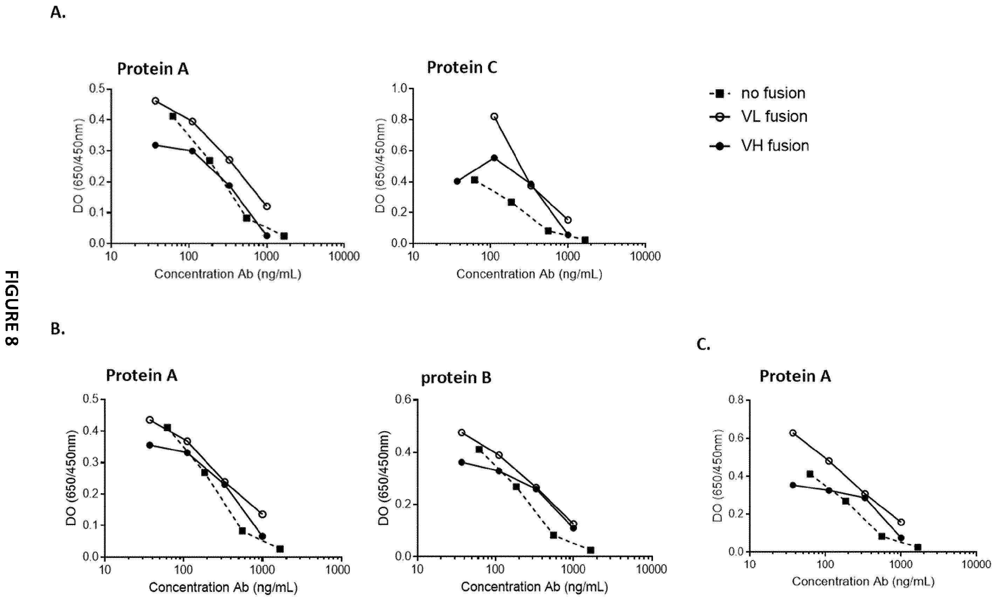

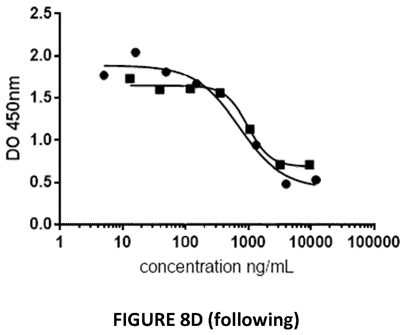

**Figure 8****: Competition PD-1/PD-L1 or PD-L2 ELISA assay.** A: PD-1/PD-L1 antagonist activity: PD-L1 is immobilized and complex antibody+biotinylated recombinant human PD-1 was added. Different concentrations of Anti-PD-1 antibody was tested and recombinant biotinylated PD1 protein was added at 0,6 µg/mL. Revelation was performed with streptavidin peroxidase to detect PD1 molecule and revealed by colorimetry at 450nm using TMB substrate. **(A)** Data of anti PD-1_antibodies fused to 3 Type I Ig like family proteins A or C; **(B)** Data of anti PD-1 antibodies fused to Type II TNF family proteins A or B **(C)** Data of anti PD-1 antibodies fused cytokine family protein A. In this figure, the cytokine fused to anti PD-1 antibody is an IL-7; the type I protein fused protein A corresponds to CD86; protein C corresponds to SIRPa; type II protein A corresponds to OX40L and protein B corresponds to 4-1BBL. **(D):** PD-1/PD-L2 antagonist activity. PD-L2 was immobilized and a complex biotinylated recombinant human PD-1 + anti PD-1 VH IL-7 (•) or anti PD-1 VH CD80 (■) was added. Similar protocol as PD-L1/PD-1 assay was used for revelation. In this experiment, the bifunctional molecule comprises a humanized anti-PD1 antibody having a heavy chain variable domain as disclosed in SEQ ID NO: 24 and a light chain variable domain as disclosed in SEQ ID NO:28.

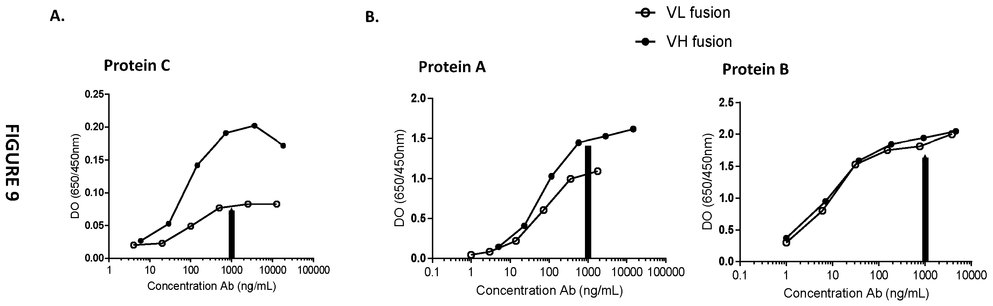

**Figure 9****: Bridging ELISA Binding assay. (A) and (B)** PD1-His recombinant protein was immobilized and Bifunctional anti PD-1 antibody (protein type I or II fused to VL domain (o) to VH domain (•)) were added at serial concentrations. Soluble recombinant receptor ligands of Protein C, A or B were then added at 1ug/mL. Detection was performed with a receptor specific mouse antibody + an anti IgG mouse antibody coupled to peroxidase. ELISA was revealed by colorimetry at 450nm using TMB substrate. Histograms represent recombinant protein A, B or C immobilized on the plate and were used as positive control for ELISA. In this figure, protein A corresponds to OX40L, protein B corresponds to 4-1BBLand protein C corresponds to SIRPalpha.

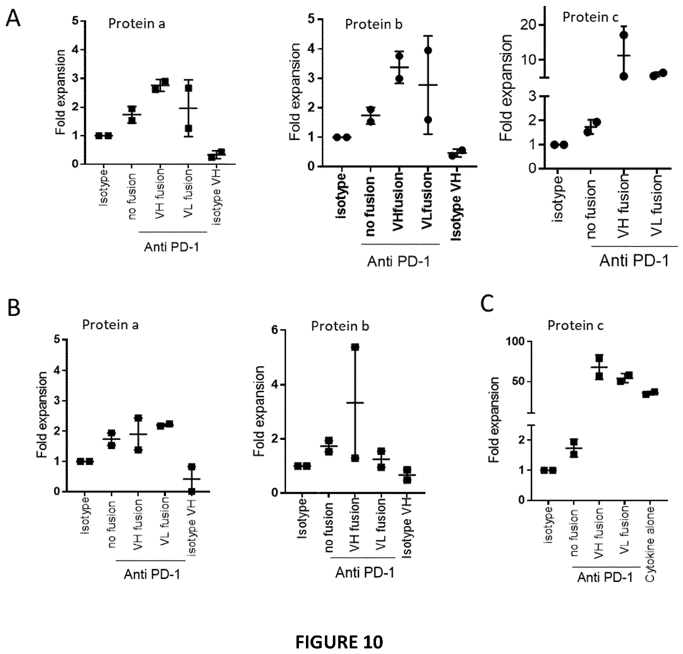

**Figure 10****: T cell proliferation stimulated by bifunctional anti-PD1- molecules.** CD3 CD28 pre-activated T cells were re-stimulated on CD3/PDL1 coated plate in the presence of anti PD-1 bifunctional antibody fused to VH or VL domain with Type I **(A)** Type II **(B)** or **(C)** Cytokine proteins (10ug/mL). Unfused anti PD-1 antibody, or isotype VH fused antibody are used as control in the experiment. T cell proliferation was assessed by H3 thymidine incorporation at Day 6. Data are represented in fold change with isotype treatment used as control. Each point represents data from one donor. In this figure, the cytokine fused to anti PD-1 antibody is an IL-7; the type I protein fused protein A corresponds to CD86, protein B corresponds to CD80; protein C corresponds to SIRPa; type II protein A corresponds to OX40L and protein B corresponds to 4-1BBL.

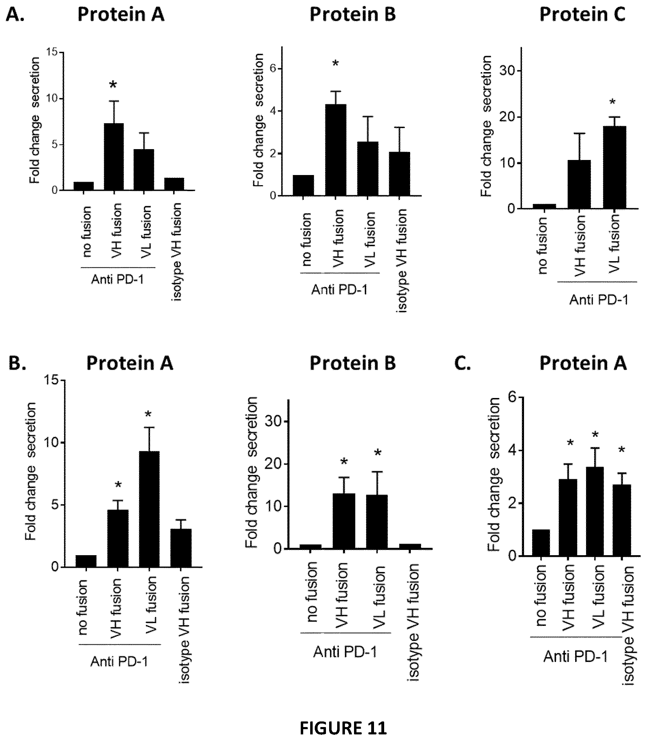

**Figure 11****: IFNγ secretion by T cells treated with anti PD1- bifunctional antibodies.** CD3 CD28 pre-activated T cells were re-stimulated on CD3/PDL1 coated plate in the presence of anti PD-1 bifunctional antibody fused to VH or VL domain with (A) Type I, (B) Type II or (C) Cytokine proteins (10ug/mL). Unfused anti PD-1 antibody, or isotype VH fused antibody are used as control in the experiment. IFN gamma secretion was assessed by ELISA in the supernatant collected at Day 5. Data are represented in fold change with anti PD1 no fusion treatment used as control. Mann Whitney test was used for statistical analysis *p<0,05. In this figure, the cytokine fused to anti PD-1 antibody is an IL-7; the type I protein fused protein A corresponds to CD80, protein B corresponds to CD86; protein C corresponds to SIRPa; type II protein A corresponds to OX40L and protein B corresponds to 4-1BBL.

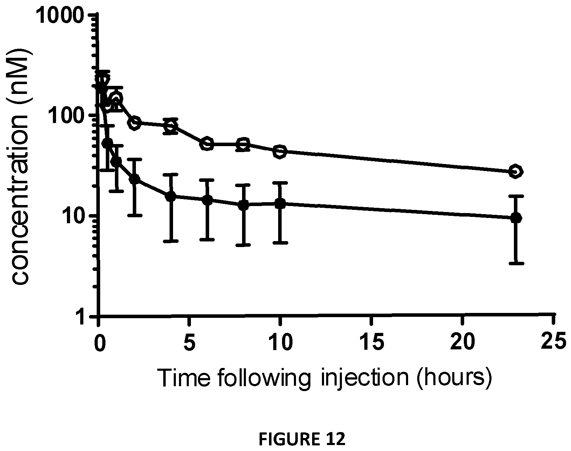

**Figure 12****: Pharmacokinetics of bifunctional humanized PD-1 antibody mice following a single injection.** Balb/C Mice were intravenously with bifunctional antibody humanized variant with IgG4 S228P isotype (•) or IgG1 N298A isotype (○). Plasma drug concentration was determined by ELISA using an immobilized anti-human light chain antibody (clone NaM76-5F3) diluted serum containing anti-PD-1 antibody was added. Detection was performed with a peroxidase-labeled donkey anti-human IgG. In this experiment, the bifunctional molecule comprises a humanized anti-PD1 antibody having a heavy chain variable domain as disclosed in SEQ ID NO:24 and a light chain variable domain as disclosed in SEQ ID NO:28.

### DETAILED DESCRIPTION OF THE INVENTION

### Introduction

The humanized antibodies of the invention are bifunctional since they combine the specific anti-PD-1 effects and the effects of an immunotherapeutic agent engrafted to the humanized anti-PD1 antibody. Indeed, the present invention relates to a bifunctional molecule comprising a particular humanized anti-PD-1 antibody with an immunotherapeutic agent. More particularly, it relates to a bifunctional molecule comprising a humanized anti-PD-1 antibody with an immunotherapeutic agent, the immunotherapeutic agent being covalently linked to a polypeptide chain of the humanized anti-PD-1 antibody, either the light chain or the heavy chain of the antibody or both. More specifically, the chain of the humanized anti-PD-1 antibody and the immunotherapeutic agent are prepared as a fusion protein. In this particular aspect, the N terminal end of the immunotherapeutic agent is linked to the C terminal end of the chain of the humanized anti-PD-1 antibody, optionally through a peptide linker. The bifunctional molecules of the invention have in particular one or several of the following advantages:
- They present a high manufacturability and high productivity yield when they are produced in mammalian cells (e.g., COS, CHO) compared to a chimeric antibody. Furthermore, the same improved productivity does not exist when considering other anti-PD1 antibodies. As illustrated by Figures 1 to 3, the bifunctional molecules including the particular humanized anti-PD-1 of the present invention have surprisingly a better production compared to the chimeric antibody or to two anti-PD1 antibodies of reference which are already clinically approved, namely pembrolizumab and Nivolumab. In addition, this improved production has been demonstrated with three different kinds of immunotherapeutic agents and six different immunotherapeutic agents, namely a cytokine (i.e., IL-7), a protein of type I (i.e., CD80, CD86 and SIRPalpha), and a protein of type II (i.e., OX40L and 4-1BBL). Furthermore, the Inventors have observed that, when the immunotherapeutic agent is a Type I transmembrane protein, this immunotherapeutic agent remains functional when grafted to the C terminus of the humanized anti-hPD1 antibody. This is both surprising and of great interest because Type I transmembrane proteins are characterized by an N-terminus effecting extracellular domain so that, up to now, N-terminus grafting of Type I transmembrane proteins generally does not enable the functionality of such proteins. In addition, the improved productivity has been observed if the immunotherapeutic agent is fused to the C terminal end of the heavy chain or of the light chain. Even more, the improved productivity has been observed if the immunotherapeutic agent is fused both to the C terminal end of the heavy chain and to the C terminal end of the light chain (Figure 1). Therefore, the better production is closely associated to the humanized anti-PD1 antibodies of the invention. This property is highly surprising and cannot be anticipated.
- They are fully functional and activate innate and adaptive immune responses. Indeed, as shown in Figures 4-7, the binding of the bifunctional molecules to PD-1 is not substantially modified. The bifunctional molecules substantially retain the same antagonistic activity as illustrated in Figures 8-9. It has been shown with 1) three different kinds of immunotherapeutic agents and six different immunotherapeutic agents, namely a cytokine (i.e., IL-7), a protein of type I (i.e., CD80, CD86 and SIRPalpha), and a protein of type II (i.e., OX40L and 4-1BBL); and 2) if the immunotherapeutic agent is fused to the C terminal end of the heavy chain or of the light chain or to both light and heavy chains. Finally, the bifunctional molecules induce a T cell proliferation at least as good as the anti-PD1 antibody alone or even better than the anti-PD1 antibody alone (Figure 10). They show a better efficiency on T cell activation than anti-PD1 antibody alone (Figure 11). This capacity to activate T cell better than the anti-PD1 antibody alone is a surprising property of the bifunction molecules of the invention in view of the slight loss of binding to the ligand of PD-1, namely PD-L1.
- Due to the targeting, the bifunctional molecules avoid hematological toxicity due to restricted expression of PD-1 (no binding to human Red Blood Cells (RBC) and platelets);
- They reduce tumor growth and modify tumor microenvironment;
- They enable human T cell immune responses, being selective antagonist of PD-1/PD-L1 interaction and/or PD-1/PD-L2 interaction;
- They may show additional or synergistic effects by combination of the anti-PD-1 and the immunotherapeutic agent into one molecule (in particular regarding the secretion of IFNy and proliferation of T cells in the Examples).

The humanized anti-PD-1 antibodies of the invention have CDRs sequences with a very low similarity with other anti-PD-1 antibodies, including pembrolizumab (also called Keytruda) and nivolumab (also called Opdivo). Thus, unpredictably, and maybe in relation to such difference of the CDRs sequences, the applicant has succeeded to obtain a bifunctional exhibiting the advantageous effects as described in the application. The humanized anti-PD-1 antibody used the bifunctional molecules has the strongly unexpected capacity to be produced with a high efficiency whatever is the immunotherapeutic agent coupled to the antibody. Then, a scaffold of bifunctional molecules suitable for preparing various bifunctional molecules at an industrial scale. This is of great help both for reglementary and safety processes because of the produced amounts compatible with a drug development and of the reproducibility.

### Definitions

In order that the present invention may be more readily understood, certain terms are defined hereafter. Additional definitions are set forth throughout the detailed description.

Unless otherwise defined, all terms of art, notations and other scientific terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a difference over what is generally understood in the art. The techniques and procedures described or referenced herein are generally well understood and commonly employed using conventional methodologies by those skilled in the art

As used herein, the terms "Programmed Death 1", "Programmed Cell Death 1", "PD1", "PD-1", "PDCD1", "PD-1 antigen", "human PD-1", "hPD-1" and "hPD1" are used interchangeably and refer to the Programmed Death-1 receptor, also known as CD279, and include variants and isoforms of human PD-1, and analogs having at least one common epitope with PD-1. PD-1 is a key regulator of the threshold of immune response and peripheral immune tolerance. It is expressed on activated T cells, B cells, monocytes, and dendritic cells and binds to its ligands PD-L1 and PD-L2. Human PD-1 is encoded by the PDCD1 gene. As an example, the amino acid sequence of a human PD-1 is disclosed under GenBank accession number NP_005009. PD1 has four splice variants expressed on human Peripheral blood mononuclear cells (PBMC). Accordingly, PD-1 proteins include full-length PD-1, as well as alternative splice variants of PD- 1, such as PD-1Aex2, PD-1Aex3, PD-1Aex2,3 and PD-1Aex2,3,4. Unless specified otherwise, the terms include any variant and isoform of human PD-1 that are naturally expressed by PBMC, or that are expressed by cells transfected with a PD-1 gene.

As used herein, the term "antibody" describes a type of immunoglobulin molecule and is used in its broadest sense. In particular, antibodies include immunoglobulin molecules. Immunoglobulin molecules can be of any type (e.g., IgG, IgE, IgM, IgD, IgA and IgY), class (e.g., IgG1, IgG2, IgG3, IgG4, IgA1 and IgA2) or subclass. The heavy-chain constant domains that correspond to the different classes of immunoglobulins are called alpha, delta, epsilon, gamma, and mu, respectively.

As used herein, an "antigen-binding fragment" of an antibody means a part of an antibody, i.e. a molecule corresponding to a portion of the structure of the antibody of the disclosure, that exhibits antigen-binding capacity for PD-1, possibly in its native form; such fragment especially exhibits the same or substantially the same antigen-binding specificity for said antigen compared to the antigen-binding specificity of the corresponding four-chain antibody. Advantageously, the antigen-binding fragments have a similar binding affinity as the corresponding 4-chain antibodies. However, antigen-binding fragment that have a reduced antigen-binding affinity with respect to corresponding 4-chain antibodies are also encompassed within the disclosure but are not claimed. The antigen-binding capacity can be determined by measuring the affinity between the antibody and the target fragment. These antigen-binding fragments may also be designated as "functional fragments" of antibodies. Antigen-binding fragments of antibodies are fragments which comprise their hypervariable domains designated CDRs (Complementary Determining Regions) or part(s) thereof encompassing the recognition site for the antigen, *i.e*. the extracellular domain of PD1, thereby defining antigen recognition specificity.

A "Fab" fragment contains the constant domain of the light chain and the first constant domain (CH1) of the heavy chain. Fab' fragments differ from Fab fragments by the addition of a few residues at the carboxyl terminus of the heavy chain CH1 domain including one or more cysteines from the antibody hinge region. F(ab') fragments are produced by cleavage of the disulfide bond at the hinge cysteines of the F(ab')2 pepsin digestion product. Additional chemical couplings of antibody fragments are known to those of ordinary skill in the art. Fab and F(ab')2 fragments lack the Fc fragment of an intact antibody, clear more rapidly from the circulation of animals, and may have less non-specific tissue binding than an intact antibody (see, e.g. Wahl et al, 1983, J. Nucl. Med. 24:316).

An "Fv" fragment is the minimum fragment of an antibody that contains a complete target recognition and binding site. This region consists of a dimer of one heavy and one light chain variable domain in a tight, non-covalent association (VH-VL dimer). It is in this configuration that the three CDRs of each variable domain interact to define a target binding site on the surface of the VH-VL dimer. Often, the six CDRs confer target binding specificity to the antibody. However, in some instances even a single variable domain (or half of an Fv comprising only three CDRs specific for a target) can have the ability to recognize and bind target, although at a lower affinity than the entire binding site.

"Single-chain Fv" or "scFv" antibody binding fragments comprise the VH and VL domains of an antibody, where these domains are present in a single polypeptide chain. Generally, the Fv polypeptide further comprises a polypeptide linker between the VH and VL domains which enables the scFv to form the desired structure for target binding.

"Single domain antibodies" are composed of a single VH or VL domains which exhibit sufficient affinity to PD-1. In a specific aspect, which is not claimed, the single domain antibody is a camelized antibody {See, e.g., Riechmann, 1999, Journal of Immunological Methods 231 :25-38).

In terms of structure, an antibody may have heavy (H) chains and light (L) chains interconnected by disulfide bonds. There are two types of light chain, lambda (λ) and kappa (κ). Each heavy and light chain contains a constant region and a variable region (or "domain"). Light and heavy chain variable regions contain a "framework" region interrupted by three hypervariable regions, also called "complementarity-determining regions" or "CDRs". The extent of the framework region and CDRs have been defined (see, Kabat et al., Sequences of Proteins of Immunological Interest, and U.S. Department of Health and Human Services, 1991). Preferably, the CDRs are defined according to Kabat method. The framework regions act to form a scaffold that provides, for positioning the CDRs in correct orientation by inter-chain, non-covalent interactions. The CDRs are primarily responsible for binding to an epitope of an antigen. The CDRs of each chain are typically referred to as "Complementarity Determining Region 1" or "CDR1", "CDR2", and "CDR3", numbered sequentially starting from the N-terminus. The VL and VH domain of the antibody according to the invention may comprise four framework regions or "FR's", which are referred to in the art and herein as "Framework region 1 " or "FR1", "FR2", "FR3", and "FR4", respectively. These framework regions and complementary determining regions are preferably operably linked in the following order: FR1-CDR1-FR2-CDR2-FR3-CDR3-FR4 (from amino terminus to carboxy terminus).

An "antibody heavy chain" as used herein, refers to the larger of the two types of polypeptide chains present in antibody conformations. The CDRs of the antibody heavy chain are typically referred to as "HCDR1", "HCDR2" and "HCDR3". The framework regions of the antibody heavy chain are typically referred to as "HFR1", "HFR2", "HFR3" and "HFR4".

An "antibody light chain," as used herein, refers to the smaller of the two types of polypeptide chains present in antibody conformations; κ and λ light chains refer to the two major antibody light chain isotypes. The CDRs of the antibody light chain are typically referred to as "LCDR1", "LCDR2" and "LCDR3". The framework regions of the antibody light chain are typically referred to as "LFR1", "LFR2", "LFR3" and "LFR4".

With regard to the binding of an antibody to a target molecule, the terms "bind" or "binding" refer to peptides, polypeptides, proteins, fusion proteins, molecules and antibodies (including antibody fragments, which are not claimed) that recognize and contact an antigen. Preferably, it refers to an antigen-antibody type interaction. The terms "specific binding", "specifically binds to," "specific for," "selectively binds" and "selective for" a particular antigen (e.g., PD-1) or an epitope on a particular antigen (e.g., PD-1) mean that the antibody recognizes and binds a specific antigen, but does not substantially recognize or bind other molecules in a sample. For example, an antibody that specifically (or preferentially) binds to PD-1 or to a PD-1 epitope is an antibody that binds this PD-1 epitope for example with greater affinity, avidity, more readily, and/or with greater duration than it binds to other PD-1 epitopes or non-PD-1 epitopes. Preferably, the term "specific binding" means the contact between an antibody and an antigen with a binding affinity equal or lower than 10⁻⁷ M. In certain aspects, antibodies bind with affinities equal or lower than 10⁻⁸ M, 10⁻⁹ M or 10⁻¹⁰ M.

As used herein "PD-1 antibody," "anti-PD-1 antibody," "PD-1 Ab," "PD-1-specific antibody" or "anti-PD-1 Ab" or "humanized anti-PD-1 antibody" are used interchangeably and refer to an antibody, as described herein, which specifically binds to PD-1, preferably human PD-1. In some embodiments, the antibody binds to the extracellular domain of PD- 1. Particularly, an anti-PD-1 antibody is an antibody capable of binding to a PD-1 antigen and inhibits the PD-1-mediated signaling pathway, thereby enhancing immune responses such as T cell activation.

As used herein, the term "bifunctional molecule", "bifunctional compound", "bifunctional protein", "Bicki", "Bicki antibody", "bifunctional antibody" and "bifunctional checkpoint inhibitors molecule" have the same meanings and can be interchangeably used. These terms refer to an antibody that recognizes one antigen by virtue of possessing at least one region (e.g. derived from a variable region of an antibody) that is specific for this antigen, and at least a second region that is a polypeptide. More specifically, the bifunctional molecule is a fusion protein of an antibody with another polypeptide or polypeptide fragment thereof.

The term "chimeric antibody" as used herein, means an antibody or antigen-binding fragment, having a portion of heavy and/or light chain derived from one species, and the rest of the heavy and/or light chain derived from a different species. In an illustrative example, a chimeric antibody may comprise a constant region derived from human and a variable region from a non-human species, such as from a mouse.

As used herein, the term "humanized antibody" is intended to refer to antibodies in which CDR sequences derived from the germline of another mammalian species, such as a mouse, have been grafted onto human framework sequences (e.g. chimeric antibodies that contain minimal sequence derived from a non-human antibody). A "humanized form" of an antibody, e.g., a non-human antibody, also refers to an antibody that has undergone humanization. A humanized antibody is generally a human immunoglobulin (recipient antibody) in which residues from one or more CDRs are replaced by residues from at least one CDR of a non-human antibody (donor antibody) while maintaining the desired specificity, affinity, and capacity of the original antibody. The donor antibody can be any suitable non-human antibody, such as a mouse, rat, rabbit, chicken, or non-human primate antibody having a desired specificity, affinity, or biological effect. In some instances, selected framework region residues of the recipient antibody are replaced by framework region residues from the donor antibody. Alternatively, selected framework region residues of the donor antibody are replaced by framework region residues from a human or humanized antibody. Additional framework region modifications may be made within the human framework sequences. Humanized antibodies thus may also comprise residues that are not found in either the recipient antibody or the donor antibody. Such amino acid modifications may be made to further refine antibody function and/or increased the humanization process. By "amino acid change" or "amino acid modification" is meant herein a change in the amino acid sequence of a polypeptide. "Amino acid modifications" include substitution, insertion and/or deletion in a polypeptide sequence. By "amino acid substitution" or "substitution" herein is meant the replacement of an amino acid at a particular position in a parent polypeptide sequence with another amino acid. By "amino acid insertion" or "insertion" is meant the addition of an amino acid at a particular position in a parent polypeptide sequence. By "amino acid deletion" or "deletion" is meant the removal of an amino acid at a particular position in a parent polypeptide sequence. The amino acid substitutions may be conservative. A conservative substitution is the replacement of a given amino acid residue by another residue having a side chain ("R-group") with similar chemical properties (e.g., charge, bulk and/or hydrophobicity). As used herein, "amino acid position" or "amino acid position number" are used interchangeably and refer to the position of a particular amino acid in an amino acids sequence, generally specified with the one letter codes for the amino acids. The first amino acid in the amino acids sequence (i.e. starting from the N terminus) should be considered as having position 1.

A conservative substitution is the replacement of a given amino acid residue by another residue having a side chain ("R-group") with similar chemical properties (e.g., charge, bulk and/or hydrophobicity). In general, a conservative amino acid substitution will not substantially change the functional properties of a protein. Conservative substitutions and the corresponding rules are well-described in the state of the art. For instance, conservative substitutions can be defined by substitutions within the groups of amino acids reflected in the following tables:

**Table A - Amino Acid Residue**

| Amino Acid groups | Amino Acid Residues |
|---|---|
| Acidic Residues | ASP and GLU |
| Basic Residues | LYS, ARG, and HIS |
| Hydrophilic Uncharged Residues | SER, THR, ASN, and GLN |
| Aliphatic Uncharged Residues | GLY, ALA, VAL, LEU, and ILE |
| Non-polar Uncharged Residues | CYS, MET, and PRO |
| Aromatic Residues | PHE, TYR, and TRP |

**Table B - Alternative Conservative Amino Acid Residue Substitution Groups**

| | | | |
|---|---|---|---|
| 1 | Alanine (A) | Serine (S) | Threonine (T) |
| 2 | Aspartic acid (D) | Glutamic acid (E) | |
| 3 | Asparagine (N) | Glutamine (Q) | |
| 4 | Arginine (R) | Lysine (K) | |
| 5 | Isoleucine (I) | Leucine (L) | Methionine (M) |
| 6 | Phenylalanine (F) | Tyrosine (Y) | Tryptophan (W) |

**Table C - Further Alternative Physical and Functional Classifications of Amino Acid Residues**

| | |
|---|---|
| Alcohol group-containing residues | S and T |
| Aliphatic residues | I, L, V, and M |
| Cycloalkenyl-associated residues | F, H, W, and Y |
| Hydrophobic residues | A, C, F, G, H, I, L, M, R, T, V, W, and Y |
| Negatively charged residues | D and E |
| Polar residues | C, D, E, H, K, N, Q, R, S, and T |
| Small residues | A, C, D, G, N, P, S, T, and V |
| Very small residues | A, G, and S |
| Residues involved in turn formation | A, C, D, E, G, H, K, N, Q, R, S, P, and T |
| Flexible residues | E, Q, T, K, S, G, P, D, E, and R |

As used herein, an "isolated antibody" is an antibody that has been separated and/or recovered from a component of its natural environment. An isolated antibody includes an antibody in situ within recombinant cells, since at least one component of the antibody's natural environment is not present. In some embodiments, an antibody is purified to homogeneity and/or to greater than 90%, 95% or 99% purity as determined by, for example, electrophoretic (e.g., SDS-PAGE, isoelectric focusing (IEF), capillary electrophoresis) or chromatographic (e.g., ion exchange or reverse phase HPLC) under reducing or non-reducing conditions.

The terms "derive from" and "derived from" as used herein refers to a compound having a structure derived from the structure of a parent compound or protein and whose structure is sufficiently similar to those disclosed herein and based upon that similarity, would be expected by one skilled in the art to exhibit the same or similar properties, activities and utilities as the claimed compounds. For example, a humanized antibody derived from a murine antibody refers to an antibody or antibody fragment that shares similar properties with the murine antibody, e.g. recognizes the same epitope, shares similar VH and VL with modified residues that participate and/or increased the humanization of the antibody.

The term "treatment" refers to any act intended to ameliorate the health status of patients such as therapy, prevention, prophylaxis and retardation of the disease or of the symptoms of the disease. It designates both a curative treatment and/or a prophylactic treatment of a disease. A curative treatment is defined as a treatment resulting in cure or a treatment alleviating, improving and/or eliminating, reducing and/or stabilizing a disease or the symptoms of a disease or the suffering that it causes directly or indirectly. A prophylactic treatment comprises both a treatment resulting in the prevention of a disease and a treatment reducing and/or delaying the progression and/or the incidence of a disease or the risk of its occurrence. In certain embodiments, such a term refers to the improvement or eradication of a disease, a disorder, an infection or symptoms associated with it. In other embodiments, this term refers to minimizing the spread or the worsening of cancers. Treatments according to the present invention do not necessarily imply 100% or complete treatment. Rather, there are varying degrees of treatment of which one of ordinary skill in the art recognizes as having a potential benefit or therapeutic effect. Preferably, the term "treatment" refers to the application or administration of a composition including one or more active agents to a subject, who has a disorder/disease, for instance associated with the signaling pathway mediated by PD-1.

As used herein, the terms "disorder" or "disease" refer to the incorrectly functioning organ, part, structure, or system of the body resulting from the effect of genetic or developmental errors, infection, poisons, nutritional deficiency or imbalance, toxicity, or unfavorable environmental factors. Preferably, these terms refer to a health disorder or disease e.g. an illness that disrupts normal physical or mental functions. More preferably, the term disorder refers to immune and/or inflammatory diseases that affect animals and/or humans, such as cancer.

The term "immune disease", as used herein, refers to a condition in a subject characterized by cellular, tissue and/or organ injury caused by an immunologic reaction of the subject to its own cells, tissues and/or organs. The term "inflammatory disease" refers to a condition in a subject characterized by inflammation, e.g., chronic inflammation. Autoimmune disorders may or may not be associated with inflammation. Moreover, inflammation may or may not be caused by an autoimmune disorder.

The term "cancer" as used herein is defined as disease characterized by the rapid and uncontrolled growth of aberrant cells. Cancer cells can spread locally or through the bloodstream and lymphatic system to other parts of the body.

As used herein, the term "disease associated with or related to PD-1", "PD-1 positive cancer" or "PD-1 positive infectious disease" is intended to refer to the cancer or infectious disease (e.g. caused by a virus and/or bacteria) which is resulted from PD-1 expression or has the symptom/characteristic of PD-1 expression, i.e. any condition that is caused by, exacerbated by, or otherwise linked to increased or decreased expression or activities of PD-1.

As used herein, the term "subject", "host", "individual," or "patient" refers to human, including adult and child.

As used herein, a "pharmaceutical composition" refers to a preparation of one or more of the active agents, such as comprising a bifunctional molecule according to the invention, with optional other chemical components such as physiologically suitable carriers and excipients. The purpose of a pharmaceutical composition is to facilitate administration of the active agent to an organism. Compositions of the present invention can be in a form suitable for any conventional route of administration or use. In one embodiment, a "composition" typically intends a combination of the active agent, e.g., compound or composition, and a naturally-occurring or non-naturally-occurring carrier, inert (for example, a detectable agent or label) or active, such as an adjuvant, diluent, binder, stabilizer, buffers, salts, lipophilic solvents, preservative, adjuvant or the like and include pharmaceutically acceptable carriers. An "acceptable vehicle" or "acceptable carrier" as referred to herein, is any known compound or combination of compounds that are known to those skilled in the art to be useful in formulating pharmaceutical compositions.

"An effective amount" or a "therapeutic effective amount" as used herein refers to the amount of active agent required to confer therapeutic effect on the subject, either alone or in combination with one or more other active agents, e.g. the amount of active agent that is needed to treat the targeted disease or disorder, or to produce the desired effect. The "effective amount" will vary depending on the agent(s), the disease and its severity, the characteristics of the subject to be treated including age, physical condition, size, gender and weight, the duration of the treatment, the nature of concurrent therapy (if any), the specific route of administration and like factors within the knowledge and expertise of the health practitioner. These factors are well known to those of ordinary skill in the art and can be addressed with no more than routine experimentation. It is generally preferred that a maximum dose of the individual components or combinations thereof be used, that is, the highest safe dose according to sound medical judgment.

As used herein, the term "medicament" refers to any substance or composition with curative or preventive properties against disorders or diseases.

The term "in combination" as used herein refers to the use of more than one therapy (e.g., prophylactic and/or therapeutic agents). The use of the term "in combination" does not restrict the order in which therapies (e.g., prophylactic and/or therapeutic agents) are administered to a subject with a disease or disorder.

The terms "polynucleotide", "nucleic acid" and "nucleic acid sequence" are equivalent and refer to a polymeric form of nucleotide of any length, for example RNA or DNA or analogs thereof. Nucleic acids (e.g., components, or portions, of the nucleic acids) of the present invention may be naturally occurring, modified or engineered, isolated and/or non-natural. Engineered nucleic acids include recombinant nucleic acids and synthetic nucleic acids. "Isolated nucleic acid encoding an anti-PD1 antibody" refers to one or more nucleic acid molecules encoding antibody heavy and light chains (or fragments thereof, which are not claimed), including such nucleic acid molecule(s) in a single vector or separate vectors, and such nucleic acid molecule(s) present at one or more locations in a host cell.

As used herein, the terms "nucleic acid construct", "plasmid", and "vector" are equivalent and refer to a nucleic acid molecule that serves to transfer a passenger nucleic acid sequence, such as DNA or RNA, into a host cell.

As used herein, the term "host cell" is intended to include any individual cell or cell culture that can be or has been recipient of vectors, exogenous nucleic acid molecules, and polynucleotides encoding the antibody construct of the present invention; and/or recipients of the antibody construct itself. The introduction of the respective material into the cell can be carried out by way of transformation, transfection and the like. The term "host cell" is also intended to include progeny or potential progeny of a single cell. Host cells include for example bacterial, microbial, plant and animal cells.

"Immune cells" as used herein refers to cells involved in innate and adaptive immunity for example such as white blood cells (leukocytes) which are derived from hematopoietic stem cells (HSC) produced in the bone marrow, lymphocytes (T cells, B cells, natural killer (NK) cells and Natural Killer T cells (NKT) and myeloid-derived cells (neutrophil, eosinophil, basophil, monocyte, macrophage, dendritic cells). In particular, the immune cell can be selected in the non-exhaustive list comprising B cells, T cells, in particular CD4+ T cells and CD8+ T cells, NK cells, NKT cells, APC cells, dendritic cells and monocytes. "T cell" as used herein includes for example CD4 + T cells, CD8 + T cells, T helper 1 type T cells, T helper 2 type T cells, T helper 17 type T cells and inhibitory T cells.

As used herein, the term "T effector cell", "T eff" or "effector cell" describes a group of immune cells that includes several T cell types that actively respond to a stimulus, such as co-stimulation. It particularly includes T cells which function to eliminate antigen (e.g., by producing cytokines which modulate the activation of other cells or by cytotoxic activity). It notably includes CD4+, CD8+, Treg cells, cytotoxic T cells and helper T cells (Th1 and Th2).

As used herein, the term "regulatory T cell", Treg cells" or "T reg" refers to a subpopulation of T cells that modulate the immune system, maintain tolerance to self-antigens, and prevent autoimmune disease. Tregs are immunosuppressive and generally suppress or downregulate induction and proliferation of effector T cells. Tregs express the biomarkers CD4, FOXP3, and CD25 and are thought to be derived from the same lineage as naïve CD4 cells.

The term "exhausted T cell" refers to a population of T cell in a state of dysfunction (i.e. "exhaustion"). T cell exhaustion is characterized by progressive loss of function, changes in transcriptional profiles and sustained expression of inhibitory receptors. Exhausted T cells lose their cytokines production capacity, their high proliferative capacity and their cytotoxic potential, which eventually leads to their deletion. Exhausted T cells typically indicate higher levels of CD43, CD69 and inhibitory receptors combined with lower expression of CD62L and CD127.

The term "immune response" refers to the action of, for example, lymphocytes, antigen presenting cells, phagocytic cells, granulocytes, and soluble macromolecules produced by the above cells or the liver (including antibodies, cytokines, and complements) that results in selective damage to, destruction of, or elimination from the human body of invading pathogens, cells or tissues infected with pathogens, cancerous cells, or, in cases of autoimmunity or pathological inflammation, normal human cells or tissues.

The term "antagonist" as used herein, refers to a substance that block or reduces the activity or functionality of another substance. Particularly, this term refers to an antibody that binds to a cellular receptor (e.g. PD-1) as a reference substance (e.g. PD-L1 and/or PD-L2), preventing it from producing all or part of its usual biological effects (e.g. the creation of an immune suppressive microenvironment). The antagonist activity of a humanized antibody according to the invention may be assessed by competitive ELISA.

As used herein, the term "isolated" indicates that the recited material (e.g., antibody, polypeptide, nucleic acid, etc.) is substantially separated from, or enriched relative to, other materials with which it occurs in nature. Particularly, an "isolated" antibody is one which has been identified and separated and/or recovered from a component of its natural environment. For example, the isolated antibody is purified (1) to greater than 75% by weight of antibody as determined by the Lowry method, or (2) to homogeneity by SDS-PAGE under reducing or non-reducing conditions. Isolated antibody includes the antibody in situ within recombinant cells since at least one component of the antibody's natural environment will not be present. Ordinarily, however, isolated antibody will be prepared by at least one purification step.

The term "and/or" as used herein is to be taken as specific disclosure of each of the two specified features or components with or without the other. For example, "A and/or B" is to be taken as specific disclosure of each of (i) A, (ii) B and (iii) A and B, just as if each is set out individually.

The term "a" or "an" can refer to one of or a plurality of the elements it modifies (e.g., "a reagent" can mean one or more reagents) unless it is contextually clear either one of the elements or more than one of the elements is described.

The term "about" as used herein in connection with any and all values (including lower and upper ends of numerical ranges) means any value having an acceptable range of deviation of up to +/- 10% (e.g., +/- 0.5%, +/-1 %, +/-1 .5%, +/- 2%, +/- 2.5%, +/- 3%, +/- 3.5%, +/- 4%, +/- 4.5%, +/- 5%, +/- 5.5%, +/- 6%, +/- 6.5%, +/- 7%, +/- 7.5%, +/- 8%, +/- 8.5%, +/- 9%, +/-9.5%). The use of the term "about" at the beginning of a string of values modifies each of the values (i.e. "about 1, 2 and 3" refers to about 1, about 2 and about 3). Further, when a listing of values is described herein (e.g. about 50%, 60%, 70%, 80%, 85% or 86%) the listing includes all intermediate and fractional values thereof (e.g., 54%, 85.4%).

### Anti-PD-1 Antibody

The bifunctional molecule according to the invention comprises a first entity that comprises a humanized anti-hPD-1 antibody.

Provided herein are humanized antibodies that bind to human PD-1. In some aspects, the humanized antibody specifically binds to human PD-1, preferably to the extracellular domain of human PD-1. In some aspects, the humanized antibody selectively binds to one or more of full-length human PD-1, PD-1Aex2, PD-1Aex3, PD-1Aex2,3 and PD-1Aex2,3,4.

In some aspects, the humanized anti-PD1 antibody is an isolated antibody, particularly a non-natural isolated antibody. Such isolated humanized anti-PD1 antibody can be prepared by at least one purification step. In some embodiments, an isolated antibody is purified to at least 80%, 85%, 90%, 95% or 99% by weight. In some embodiments, an anti-PD1 isolated antibody is provided as a solution comprising at least 85%, 90%, 95%, 98%, 99% to 100% by weight of an antibody, the remainder of the weight comprising the weight of other solutes dissolved in the solvent.

Preferably, such antibody has the ability to block or inhibit the interaction between PD-1 and at least one of its ligands (e.g. PD-L1 and/or PD-L2). The ability to "block binding" or "block interaction" or "inhibit interaction" as used herein refers to the ability of an antibody or antigen-binding fragment to prevent the binding interaction between two molecules (e.g. PD-1 and its ligand PD-L1 and/or PD-L2) to any detectable degree.

Preferably, the anti-PD1 antibody is an antagonist of the binding of human PD-L1 and/or PD-L2 to human PD-1, more preferably of human PD-L1 and PD-L2 to human PD-1.

In certain embodiments, the anti-hPD1 antibody inhibits the binding interaction between PD-1 and at least one of its ligands (e.g. PD-L1 and/or PD-L2, preferably PD-L1 and PD-L2) by at least 50%. In certain embodiments, this inhibition may be greater than 60%, greater than 70%, greater than 80%, or greater than 90%.

Humanized forms of anti-PD1 antibodies according to this invention may comprise immunoglobulins, immunoglobulin of any class, such as IgD, IgE, IgG, IgA, or IgM (or sub-class thereof), immunoglobulin chains which contain minimal sequence derived from a non-human (e.g. murine) immunoglobulin targeting human PD-1. Preferably, the humanized anti-hPD-1 antibody according to the invention derives from IgG1, IgG2, IgG3 or IgG4, preferably from an IgG4.

A humanized antibody typically comprises one or more variable domains in which CDRs (or portions thereof) are derived from a non-human antibody, and FRs (or portions thereof) are derived from human or humanized antibody sequences. Alternatively, some FR residues can be substituted to restore or improve antibody specificity, affinity and/or humanization. A humanized antibody optionally will also comprise at least a portion of a human or humanized constant region (Fc). Methods of antibodies humanization are well known in the art see for example, Winter and Milstein, Nature, 1991, 349:293-299; Riechmann et al., Nature, 332, pp. 323 (1988); Verhoeyen et al., Science, 239, pp. 1534 (1988), Rader et al, Proc. Nat. Acad. Sci. U.S.A., 1998, 95:8910-8915; Steinberger et al, J. Biol. Chem., 2000, 275:36073-36078; Queen et al, Proc. Natl. Acad. Sci. U.S.A., 1989, 86: 10029-10033; Almagro, J.C. and Fransson, J., Front. Biosci. 13 (2008) 1619-1633; Kashmiri, S.V. et al, Methods 36 (2005) 25-34 (describing SDR (a-CDR) grafting); Padlan, E.A., Mol. Immunol. 28 (1991) 489-498 (describing "resurfacing"); Dall'Acqua, W.F. et al, Methods 36 (2005) 43-60 (describing "FR shuffling"); and Osbourn, J. et al, Methods 36 (2005) 61-68 and Klimka, A. et al, Br. J. Cancer 83 (2000) 252-260 (describing the "guided selection" approach to FR shuffling) and U.S. Patent Nos. 5,585,089, 5,693,761, 5,693,762, 5,821,337, 7,527,791, 6,982,321, and 7,087,409; and 6,180,370.

Preferably, the humanized antibody against human PD-1 is a monoclonal antibody.

### CDRs

"Complementarity determining regions" or "CDRs" are known in the art as referring to non-contiguous sequences of amino acids within antibody variable regions, which confer antigen specificity and binding affinity. The precise amino acid sequence boundaries of a given CDR can be readily determined using any of a number of well-known schemes, including those described by Kabat et al., (Sequences of Proteins of Immunological Interest 5th ed. (1991) "Kabat" numbering scheme); Al-Lazikani et al., 1997, J. Mol. Biol, 273:927-948 ("Chothia" numbering scheme); MacCallum et al, 1996, J. Mol. Biol. 262:732-745 ("Contact" numbering scheme); Lefranc et al., Dev. Comp. Immunol., 2003, 27:55-77 ("IMGT" numbering scheme); and Honegge and Pluckthun, J. Mol. Biol, 2001, 309:657-70 ("AHo" numbering scheme). Unless otherwise specified, the numbering scheme used for identification of a particular CDR herein is the Kabat numbering scheme.

The CDRs regions of the humanized antibody are derived from a murine antibody and have been optimized to i) provide a safe humanized antibody with a very high level of humanization (superior to 85%) and stability ; and ii) increase the antibody properties, more particularly a higher manufacturability when produced in mammalian cells and a higher production yield in mammal cells such as COS and HCO cells while preserving an antagonist activity and inhibition of the binding of human PD-L1 to human PD-1, as they have a binding affinity (KD) for a human PD-1 less than 10⁻⁷ M, preferably less than 10⁻⁸ M.

In one aspect that is not claimed, the antigen-binding fragment of an antibody comprises a heavy chain comprising a heavy chain variable domain comprising HCDR1, HCDR2 and HCDR3 and a light chain comprising a variable domain comprising LCDR1, LCDR2 and LCDR3, and a fragment of a heavy chain constant domain. By a fragment of a heavy chain constant domain, it should be understood that the antigen-binding fragment therefore comprises at least a portion of a full heavy chain constant domain. As examples, a heavy chain constant domain may comprise or consist of at least the C_{H}1 domain of a heavy chain, or at least the C_{H}1 and the C_{H}2 domains of a heavy chain, or at least the C_{H}1, C_{H}2 and C_{H}3 domains of a heavy chain. A fragment of a heavy chain constant domain may also be defined as comprising at least a portion of the Fc domain of the heavy chain. Accordingly, antigen-binding fragment of an antibody encompasses the Fab portion of a full antibody, the F(ab')₂ portion of a full antibody, the Fab' portion of a full antibody. The heavy chain constant domain may also comprise or consist in a full heavy chain constant domain, for example illustrated in the present description, wherein several full heavy chain constant domains are described. In a particular aspect of the disclosure which is not claimed, and when the antigen-binding fragment of an antibody comprises a fragment of a heavy chain constant domain comprising or consisting in a portion of a full heavy chain constant domain, the heavy chain constant domain fragment may consist of at least 10 amino acid residues; or may consist of 10 to 300 amino acid residues, in particular 210 amino acid residues.

In one aspect, the bifunctional molecule comprises a humanized anti-hPD-1 antibody that comprises:
(i) a heavy chain variable domain comprising HCDR1, HCDR2 and HCDR3, and
(ii) a light chain variable domain comprising LCDR1, LCDR2 and LCDR3,
wherein:
- the heavy chain CDR1 (HCDR1) comprises or consists of an amino acid sequence of SEQ ID NO: 1, optionally with one, two or three modification(s) selected from substitution(s), addition(s), deletion(s) and any combination thereof at any position but position 3 of SEQ ID NO: 1;
- the heavy chain CDR2 (HCDR2) comprises or consists of an amino acid sequence of SEQ ID NO: 2, optionally with one, two or three modification(s) selected from substitution(s), addition(s), deletion(s) and any combination thereof at any position but positions 13, 14 and 16 of SEQ ID NO: 2;
- the heavy chain CDR3 (HCDR3) comprises or consists of an amino acid sequence of SEQ ID NO: 3 wherein X1 is D or E and X2 is selected from the group consisting of T, H, A, Y, N, E and S, preferably in the group consisting of H, A, Y, N, E; optionally with one, two or three modification(s) selected from substitution(s), addition(s), deletion(s) and any combination thereof at any position but positions 2, 3, 7 and 8 of SEQ ID NO: 3;
- the light chain CDR1 (LCDR1) comprises or consists of an amino acid sequence of SEQ ID NO: 12 wherein X is G or T, optionally with one, two or three modification(s) selected from substitution(s), addition(s), deletion(s) and any combination thereof at any position but positions 5, 6, 10, 11 and 16 of SEQ ID NO: 12;
- the light chain CDR2 (LCDR2) comprises or consists of an amino acid sequence of SEQ ID NO: 15, optionally with one, two or three modification(s) selected from substitution(s), addition(s), deletion(s) and any combination thereof; and
- the light chain CDR3 (LCDR3) comprises or consists of an amino acid sequence of SEQ ID NO:16, optionally with one, two or three modification(s) selected from substitution(s), addition(s), deletion(s) and any combination thereof at any position but positions 1, 4 and 6 of SEQ ID NO: 16.

In another aspect, the bifunctional molecule comprises a humanized anti-hPD-1 antibody that comprises the HCDR1, HCDR2, LCDR2 and LCDR3 as specified above and : a heavy chain CDR3 (HCDR3) comprising or consisting of an amino acid sequence of SEQ ID NO: 3 wherein either X1 is D and X2 is selected from the group consisting of T, H, A, Y, N, E, and S preferably in the group consisting of H, A, Y, N, E; or X1 is E and X2 is selected from the group consisting of T, H, A, Y, N, E and S, preferably in the group consisting of H, A, Y, N, E and S; optionally with one, two or three modification(s) selected from substitution(s), addition(s), deletion(s) and any combination thereof at any position but positions 2, 3, 7 and 8 of SEQ ID NO: 3; and a light chain CDR1 (LCDR1) comprising or consisting of an amino acid sequence of SEQ ID NO: 12 wherein X is G or T, optionally with one, two or three modification(s) selected from substitution(s), addition(s), deletion(s) and any combination thereof at any position but positions 5, 6, 10, 11 and 16 of SEQ ID NO: 12.

In another aspect, the bifunctional molecule comprises a humanized anti-hPD-1 antibody that comprises the HCDR1, HCDR2, LCDR2 and LCDR3 as specified above and : a heavy chain CDR3 (HCDR3) comprising or consisting of an amino acid sequence of SEQ ID NO: 4, 5, 6, 7, 8, 9, 10 or 11 optionally with one, two or three modification(s) selected from substitution(s), addition(s), deletion(s) and any combination thereof at any position but positions 2, 3, 7 and 8 of SEQ ID NO: 4, 5, 6, 7, 8, 9, 10 or 11; and a light chain CDR1 (LCDR1) comprising or consisting of an amino acid sequence of SEQ ID NO: 13 or SEQ ID NO:14, optionally with one, two or three modification(s) selected from substitution(s), addition(s), deletion(s) and any combination thereof at any position but positions 5, 6, 10, 11 and 16 of SEQ ID NO: 13 or SEQ ID NO:14.

In another aspect, the bifunctional molecule comprises a humanized anti-hPD-1 antibody that comprises the HCDR1, HCDR2, LCDR2 and LCDR3 as specified above and:
- a heavy chain CDR3 (HCDR3) comprising or consisting of an amino acid sequence of SEQ ID NO: 4, optionally with one, two or three modification(s) selected from substitution(s), addition(s), deletion(s) and any combination thereof at any position but positions 2, 3, 7 and 8 of SEQ ID NO: 4; and a light chain CDR1 (LCDR1) comprising or consisting of an amino acid sequence of SEQ ID NO: 13, optionally with one, two or three modification(s) selected from substitution(s), addition(s), deletion(s) and any combination thereof at any position but positions 5, 6, 10, 11 and 16 of SEQ ID NO: 13; or
- a heavy chain CDR3 (HCDR3) comprising or consisting of an amino acid sequence of SEQ ID NO: 5, optionally with one, two or three modification(s) selected from substitution(s), addition(s), deletion(s) and any combination thereof at any position but positions 2, 3, 7 and 8 of SEQ ID NO: 5; and a light chain CDR1 (LCDR1) comprising or consisting of an amino acid sequence of SEQ ID NO: 13, optionally with one, two or three modification(s) selected from substitution(s), addition(s), deletion(s) and any combination thereof at any position but positions 5, 6, 10, 11 and 16 of SEQ ID NO: 13; or
- a heavy chain CDR3 (HCDR3) comprising or consisting of an amino acid sequence of SEQ ID NO: 6, optionally with one, two or three modification(s) selected from substitution(s), addition(s), deletion(s) and any combination thereof at any position but positions 2, 3, 7 and 8 of SEQ ID NO: 6; and a light chain CDR1 (LCDR1) comprising or consisting of an amino acid sequence of SEQ ID NO: 13, optionally with one, two or three modification(s) selected from substitution(s), addition(s), deletion(s) and any combination thereof at any position but positions 5, 6, 10, 11 and 16 of SEQ ID NO: 13; or
- a heavy chain CDR3 (HCDR3) comprising or consisting of an amino acid sequence of SEQ ID NO: 7, optionally with one, two or three modification(s) selected from substitution(s), addition(s), deletion(s) and any combination thereof at any position but positions 2, 3, 7 and 8 of SEQ ID NO:7; and a light chain CDR1 (LCDR1) comprising or consisting of an amino acid sequence of SEQ ID NO: 13, optionally with one, two or three modification(s) selected from substitution(s), addition(s), deletion(s) and any combination thereof at any position but positions 5, 6, 10, 11 and 16 of SEQ ID NO: 13; or
- a heavy chain CDR3 (HCDR3) comprising or consisting of an amino acid sequence of SEQ ID NO: 8 optionally with one, two or three modification(s) selected from substitution(s), addition(s), deletion(s) and any combination thereof at any position but positions 2, 3, 7 and 8 of SEQ ID NO: 8; and a light chain CDR1 (LCDR1) comprising or consisting of an amino acid sequence of SEQ ID NO: 13, optionally with one, two or three modification(s) selected from substitution(s), addition(s), deletion(s) and any combination thereof at any position but positions 5, 6, 10, 11 and 16 of SEQ ID NO: 13; or
- a heavy chain CDR3 (HCDR3) comprising or consisting of an amino acid sequence of SEQ ID NO: 9 optionally with one, two or three modification(s) selected from substitution(s), addition(s), deletion(s) and any combination thereof at any position but positions 2, 3, 7 and 8 of SEQ ID NO: 9; and a light chain CDR1 (LCDR1) comprising or consisting of an amino acid sequence of SEQ ID NO: 13, optionally with one, two or three modification(s) selected from substitution(s), addition(s), deletion(s) and any combination thereof at any position but positions 5, 6, 10, 11 and 16 of SEQ ID NO: 13; or
- a heavy chain CDR3 (HCDR3) comprising or consisting of an amino acid sequence of SEQ ID NO: 10 optionally with one, two or three modification(s) selected from substitution(s), addition(s), deletion(s) and any combination thereof at any position but positions 2, 3, 7 and 8 of SEQ ID NO: 10; and a light chain CDR1 (LCDR1) comprising or consisting of an amino acid sequence of SEQ ID NO: 13, optionally with one, two or three modification(s) selected from substitution(s), addition(s), deletion(s) and any combination thereof at any position but positions 5, 6, 10, 11 and 16 of SEQ ID NO: 13; or
- a heavy chain CDR3 (HCDR3) comprising or consisting of an amino acid sequence of SEQ ID NO: 11 optionally with one, two or three modification(s) selected from substitution(s), addition(s), deletion(s) and any combination thereof at any position but positions 2, 3, 7 and 8 of SEQ ID NO: 11; and a light chain CDR1 (LCDR1) comprising or consisting of an amino acid sequence of SEQ ID NO: 13, optionally with one, two or three modification(s) selected from substitution(s), addition(s), deletion(s) and any combination thereof at any position but positions 5, 6, 10, 11 and 16 of SEQ ID NO: 13; or
- a heavy chain CDR3 (HCDR3) comprising or consisting of an amino acid sequence of SEQ ID NO: 4, optionally with one, two or three modification(s) selected from substitution(s), addition(s), deletion(s) and any combination thereof at any position but positions 2, 3, 7 and 8 of SEQ ID NO: 4; and a light chain CDR1 (LCDR1) comprising or consisting of an amino acid sequence of SEQ ID NO: 14, optionally with one, two or three modification(s) selected from substitution(s), addition(s), deletion(s) and any combination thereof at any position but positions 5, 6, 10, 11 and 16 of SEQ ID NO: 14; or
- a heavy chain CDR3 (HCDR3) comprising or consisting of an amino acid sequence of SEQ ID NO: 5, optionally with one, two or three modification(s) selected from substitution(s), addition(s), deletion(s) and any combination thereof at any position but positions 2, 3, 7 and 8 of SEQ ID NO: 5; and a light chain CDR1 (LCDR1) comprising or consisting of an amino acid sequence of SEQ ID NO: 14, optionally with one, two or three modification(s) selected from substitution(s), addition(s), deletion(s) and any combination thereof at any position but positions 5, 6, 10, 11 and 16 of SEQ ID NO: 14; or
- a heavy chain CDR3 (HCDR3) comprising or consisting of an amino acid sequence of SEQ ID NO: 6, optionally with one, two or three modification(s) selected from substitution(s), addition(s), deletion(s) and any combination thereof at any position but positions 2, 3, 7 and 8 of SEQ ID NO: 6; and a light chain CDR1 (LCDR1) comprising or consisting of an amino acid sequence of SEQ ID NO: 14, optionally with one, two or three modification(s) selected from substitution(s), addition(s), deletion(s) and any combination thereof at any position but positions 5, 6, 10, 11 and 16 of SEQ ID NO: 14; or
- a heavy chain CDR3 (HCDR3) comprising or consisting of an amino acid sequence of SEQ ID NO: 7, optionally with one, two or three modification(s) selected from substitution(s), addition(s), deletion(s) and any combination thereof at any position but positions 2, 3, 7 and 8 of SEQ ID NO:7; and a light chain CDR1 (LCDR1) comprising or consisting of an amino acid sequence of SEQ ID NO: 14, optionally with one, two or three modification(s) selected from substitution(s), addition(s), deletion(s) and any combination thereof at any position but positions 5, 6, 10, 11 and 16 of SEQ ID NO: 14; or
- a heavy chain CDR3 (HCDR3) comprising or consisting of an amino acid sequence of SEQ ID NO: 8 optionally with one, two or three modification(s) selected from substitution(s), addition(s), deletion(s) and any combination thereof at any position but positions 2, 3, 7 and 8 of SEQ ID NO: 8; and a light chain CDR1 (LCDR1) comprising or consisting of an amino acid sequence of SEQ ID NO: 14, optionally with one, two or three modification(s) selected from substitution(s), addition(s), deletion(s) and any combination thereof at any position but positions 5, 6, 10, 11 and 16 of SEQ ID NO: 14; or
- a heavy chain CDR3 (HCDR3) comprising or consisting of an amino acid sequence of SEQ ID NO: 9 optionally with one, two or three modification(s) selected from substitution(s), addition(s), deletion(s) and any combination thereof at any position but positions 2, 3, 7 and 8 of SEQ ID NO: 9; and a light chain CDR1 (LCDR1) comprising or consisting of an amino acid sequence of SEQ ID NO: 14, optionally with one, two or three modification(s) selected from substitution(s), addition(s), deletion(s) and any combination thereof at any position but positions 5, 6, 10, 11 and 16 of SEQ ID NO: 14; or
- a heavy chain CDR3 (HCDR3) comprising or consisting of an amino acid sequence of SEQ ID NO: 10 optionally with one, two or three modification(s) selected from substitution(s), addition(s), deletion(s) and any combination thereof at any position but positions 2, 3, 7 and 8 of SEQ ID NO: 10; and a light chain CDR1 (LCDR1) comprising or consisting of an amino acid sequence of SEQ ID NO: 14, optionally with one, two or three modification(s) selected from substitution(s), addition(s), deletion(s) and any combination thereof at any position but positions 5, 6, 10, 11 and 16 of SEQ ID NO: 14; or
- a heavy chain CDR3 (HCDR3) comprising or consisting of an amino acid sequence of SEQ ID NO: 11 optionally with one, two or three modification(s) selected from substitution(s), addition(s), deletion(s) and any combination thereof at any position but positions 2, 3, 7 and 8 of SEQ ID NO: 11; and a light chain CDR1 (LCDR1) comprising or consisting of an amino acid sequence of SEQ ID NO: 14, optionally with one, two or three modification(s) selected from substitution(s), addition(s), deletion(s) and any combination thereof at any position but positions 5, 6, 10, 11 and 16 of SEQ ID NO: 14.

In a particular aspect, the modifications are substitutions, in particular conservative substitutions.

In one aspect, the anti-human-PD-1 antibody comprises or consists of:
(i) a heavy chain comprising a CDR1 of SEQ ID NO: 1, a CDR2 of SEQ ID NO: 2 and a CDR3 of SEQ ID NO: 3 wherein X1 is D or E and X2 is selected from the group consisting of T, H, A, Y, N, E and S, preferably in the group consisting of H, A, Y, N and E; and (ii) a light chain comprising a CDR1 of SEQ ID NO: 12 wherein X is G or T, a CDR2 of SEQ ID NO: 15 and a CDR3 of SEQ ID NO: 16; or
(i) a heavy chain comprising a CDR1 of SEQ ID NO: 1, a CDR2 of SEQ ID NO: 2 and a CDR3 of SEQ ID NO: 3 wherein X1 is D and X2 is selected from the group consisting of T, H, A, Y, N and E, preferably in the group consisting of H, A, Y, N and E; or wherein X1 is E and X2 is selected from the group consisting of T, H, A, Y, N, E, and S, preferably in the group consisting of H, A, Y, N, E and S; and (ii) a light chain comprising a CDR1 of SEQ ID NO: 12 wherein X is G or T, a CDR2 of SEQ ID NO: 15 and a CDR3 of SEQ ID NO: 16; or
(i) a heavy chain comprising a CDR1 of SEQ ID NO: 1, a CDR2 of SEQ ID NO: 2 and a CDR3 of SEQ ID NO: 3 wherein X1 is D and X2 is selected from the group consisting of T, H, A, Y, N and E, preferably in the group consisting of H, A, Y, N and E; and (ii) a light chain comprising a CDR1 of SEQ ID NO: 12 wherein X is G or T, a CDR2 of SEQ ID NO: 15 and a CDR3 of SEQ ID NO: 16; or
(i) a heavy chain comprising a CDR1 of SEQ ID NO: 1, a CDR2 of SEQ ID NO: 2 and a CDR3 of SEQ ID NO: 3 wherein X1 is E and X2 is selected from the group consisting of T, H, A, Y, N, E, and S, preferably in the group consisting of H, A, Y, N, E and S; and (ii) a light chain comprising a CDR1 of SEQ ID NO: 12 wherein X is G or T, a CDR2 of SEQ ID NO: 15 and a CDR3 of SEQ ID NO: 16.

In another aspect, the anti-human-PD-1 antibody comprises or consists essentially of (i) a heavy chain comprising a CDR1 of SEQ ID NO: 1, a CDR2 of SEQ ID NO: 2 and a CDR3 of SEQ ID NO: 4, 5, 6, 7, 8, 9, 10 or 11; and (ii) a light chain comprising a CDR1 of SEQ ID NO: 13 or SEQ ID NO:14, a CDR2 of SEQ ID NO: 15 and a CDR3 of SEQ ID NO: 16.

In another aspect, the anti-human-PD-1 antibody comprises or consists essentially of:
(i) a heavy chain comprising a CDR1 of SEQ ID NO: 1, a CDR2 of SEQ ID NO: 2 and a CDR3 of SEQ ID NO: 4; and (ii) a light chain comprising a CDR1 of SEQ ID NO: 13, a CDR2 of SEQ ID NO: 15 and a CDR3 of SEQ ID NO: 16; or
(i) a heavy chain comprising a CDR1 of SEQ ID NO: 1, a CDR2 of SEQ ID NO: 2 and a CDR3 of SEQ ID NO: 5; and (ii) a light chain comprising a CDR1 of SEQ ID NO: 13, a CDR2 of SEQ ID NO: 15 and a CDR3 of SEQ ID NO: 16; or
(i) a heavy chain comprising a CDR1 of SEQ ID NO: 1, a CDR2 of SEQ ID NO: 2 and a CDR3 of SEQ ID NO: 6; and (ii) a light chain comprising a CDR1 of SEQ ID NO: 13, a CDR2 of SEQ ID NO: 15 and a CDR3 of SEQ ID NO: 16; or
(i) a heavy chain comprising a CDR1 of SEQ ID NO: 1, a CDR2 of SEQ ID NO: 2 and a CDR3 of SEQ ID NO: 7; and (ii) a light chain comprising a CDR1 of SEQ ID NO: 13, a CDR2 of SEQ ID NO: 15 and a CDR3 of SEQ ID NO: 16; or
(i) a heavy chain comprising a CDR1 of SEQ ID NO: 1, a CDR2 of SEQ ID NO: 2 and a CDR3 of SEQ ID NO: 8; and (ii) a light chain comprising a CDR1 of SEQ ID NO: 13, a CDR2 of SEQ ID NO: 15 and a CDR3 of SEQ ID NO: 16; or
(i) a heavy chain comprising a CDR1 of SEQ ID NO: 1, a CDR2 of SEQ ID NO: 2 and a CDR3 of SEQ ID NO: 9; and (ii) a light chain comprising a CDR1 of SEQ ID NO: 13, a CDR2 of SEQ ID NO: 15 and a CDR3 of SEQ ID NO: 16; or
(i) a heavy chain comprising a CDR1 of SEQ ID NO: 1, a CDR2 of SEQ ID NO: 2 and a CDR3 of SEQ ID NO: 10; and (ii) a light chain comprising a CDR1 of SEQ ID NO: 13, a CDR2 of SEQ ID NO: 15 and a CDR3 of SEQ ID NO: 16; or
(i) a heavy chain comprising a CDR1 of SEQ ID NO: 1, a CDR2 of SEQ ID NO: 2 and a CDR3 of SEQ ID NO: 11; and (ii) a light chain comprising a CDR1 of SEQ ID NO: 13, a CDR2 of SEQ ID NO: 15 and a CDR3 of SEQ ID NO: 16; or
(i) a heavy chain comprising a CDR1 of SEQ ID NO: 1, a CDR2 of SEQ ID NO: 2 and a CDR3 of SEQ ID NO: 4; and (ii) a light chain comprising a CDR1 of SEQ ID NO: 14, a CDR2 of SEQ ID NO: 15 and a CDR3 of SEQ ID NO: 16; or
(i) a heavy chain comprising a CDR1 of SEQ ID NO: 1, a CDR2 of SEQ ID NO: 2 and a CDR3 of SEQ ID NO: 5; and (ii) a light chain comprising a CDR1 of SEQ ID NO: 14, a CDR2 of SEQ ID NO: 15 and a CDR3 of SEQ ID NO: 16; or
(i) a heavy chain comprising a CDR1 of SEQ ID NO: 1, a CDR2 of SEQ ID NO: 2 and a CDR3 of SEQ ID NO: 6; and (ii) a light chain comprising a CDR1 of SEQ ID NO: 14, a CDR2 of SEQ ID NO: 15 and a CDR3 of SEQ ID NO: 16; or
(i) a heavy chain comprising a CDR1 of SEQ ID NO: 1, a CDR2 of SEQ ID NO: 2 and a CDR3 of SEQ ID NO: 7; and (ii) a light chain comprising a CDR1 of SEQ ID NO: 14, a CDR2 of SEQ ID NO: 15 and a CDR3 of SEQ ID NO: 16; or
(i) a heavy chain comprising a CDR1 of SEQ ID NO: 1, a CDR2 of SEQ ID NO: 2 and a CDR3 of SEQ ID NO: 8; and (ii) a light chain comprising a CDR1 of SEQ ID NO: 14, a CDR2 of SEQ ID NO: 15 and a CDR3 of SEQ ID NO: 16; or
(i) a heavy chain comprising a CDR1 of SEQ ID NO: 1, a CDR2 of SEQ ID NO: 2 and a CDR3 of SEQ ID NO: 9; and (ii) a light chain comprising a CDR1 of SEQ ID NO: 14, a CDR2 of SEQ ID NO: 15 and a CDR3 of SEQ ID NO: 16; or
(i) a heavy chain comprising a CDR1 of SEQ ID NO: 1, a CDR2 of SEQ ID NO: 2 and a CDR3 of SEQ ID NO: 10; and (ii) a light chain comprising a CDR1 of SEQ ID NO: 14, a CDR2 of SEQ ID NO: 15 and a CDR3 of SEQ ID NO: 16; or
(i) a heavy chain comprising a CDR1 of SEQ ID NO: 1, a CDR2 of SEQ ID NO: 2 and a CDR3 of SEQ ID NO: 11; and (ii) a light chain comprising a CDR1 of SEQ ID NO: 14, a CDR2 of SEQ ID NO: 15 and a CDR3 of SEQ ID NO: 16.

### Framework

The term "antibody framework" as used herein refers to the part of the variable domain, either VL and/or VH, which serves as a scaffold for the antigen binding loops (CDRs) of this variable domain.

In one aspect, the anti-PD1 antibody according to the invention comprises framework regions, in particular heavy chain variable region framework regions (HFR) HFR1, HFR2, HFR3 and HFR4 and light chain variable region framework regions (LFR) LFR1, LFR2, LFR3 and LFR4.

Preferably, the anti-PD1 antibody according to the invention comprises human or humanized framework regions. A "human acceptor framework" for the purposes herein is a framework comprising the amino acid sequence of a light chain variable domain (VL) framework or a heavy chain variable domain (VH) framework derived from a human immunoglobulin framework or a human consensus framework, as defined below. A human acceptor framework derived from a human immunoglobulin framework or a human consensus framework may comprise the same amino acid sequence thereof, or it may contain amino acid sequence changes. In some aspects, the number of amino acid changes are 10 or less, 9 or less, 8 or less, 7 or less, 6 or less, 5 or less, 4 or less, 3 or less, or 2 or less. In some aspects, the VL acceptor human framework is identical in sequence to the VL human immunoglobulin framework sequence or human consensus framework sequence. A "human consensus framework" is a framework which represents the most commonly occurring amino acid residues in a selection of human immunoglobulin VL or VH framework sequences.

Particularly, the anti-PD1 antibody comprises heavy chain variable region framework regions (HFR) HFR1, HFR2, HFR3 and HFR4 comprising an amino acid sequence of SEQ ID NOs: 41, 42, 43 and 44, respectively, optionally with one, two or three modification(s) selected from substitution(s), addition(s), deletion(s) and any combination thereof at any position but positions 27, 29 and 32 of HFR3, i.e., of SEQ ID NO: 43. Preferably, the anti-PD1 antibody comprises HFR1 of SEQ ID NO: 41, HFR2 of SEQ ID NO: 42, HFR3 of SEQ ID NO: 43 and HFR4 of SEQ ID NO: 44.

Alternatively or additionally, the anti-PD1 antibody comprises light chain variable region framework regions (LFR) LFR1, LFR2, LFR3 and LFR4 comprising an amino acid sequence of SEQ ID NOs: 45, 46, 47 and 48, respectively, optionally with one, two or three modification(s) selected from substitution(s), addition(s), deletion(s) and any combination thereof. Preferably, the humanized anti-PD1 antibody comprises LFR1 of SEQ ID NO: 45, LFR2 of SEQ ID NO: 46, LFR3 of SEQ ID NO: 47 and LFR4 of SEQ ID NO: 48.

### VH-VL

The VL and VH domain of the anti hPD1 antibody comprised in the bifunctional molecule according to the invention may comprise four framework regions interrupted by three complementary determining regions preferably operably linked in the following order: FR1-CDR1-FR2-CDR2-FR3-CDR3-FR4 (from amino terminus to carboxy terminus).

In one embodiment, the anti-human-PD-1 humanized antibody comprised in the bifunctional molecule comprises:
(a) a heavy chain variable region (VH) comprising or consisting of an amino acid sequence of SEQ ID NO: 17, wherein X1 is D or E and X2 is selected from the group consisting of T, H, A, Y, N, E and S preferably in the group consisting of H, A, Y, N, E; optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 17;
(b) a light chain variable region (VL) comprising or consisting of an amino acid sequence of SEQ ID NO: 26, wherein X is G or T, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 26.

In another embodiment, the anti-human-PD-1 humanized antibody comprised in the bifunctional molecule comprises:
(a) a heavy chain variable region (VH) comprising or consisting of an amino acid sequence of SEQ ID NO: 17, wherein either X1 is D and X2 is selected from the group consisting of T, H, A, Y, N, E, preferably in the group consisting of H, A, Y, N, E; or X1 is E and X2 is selected from the group consisting of T, H, A, Y, N, E and S preferably in the group consisting of H, A, Y, N, E and S; optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 17;
(b) a light chain variable region (VL) comprising or consisting of an amino acid sequence of SEQ ID NO: 26, wherein X is G or T, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 26.

In another embodiment, the anti-human-PD-1 humanized antibody comprised in the bifunctional molecule comprises:
(a) a heavy chain variable region (VH) comprising or consisting of an amino acid sequence of SEQ ID NO: 17, wherein X1 is D and X2 is selected from the group consisting of T, H, A, Y, N, E, preferably in the group consisting of H, A, Y, N, E, optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 17;
(b) a light chain variable region (VL) comprising or consisting of an amino acid sequence of SEQ ID NO: 26, wherein X is G or T, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 26.

In another embodiment, the anti-human-PD-1 humanized antibody comprised in the bifunctional molecule comprises:
(a) a heavy chain variable region (VH) comprising or consisting of an amino acid sequence of SEQ ID NO: 17, wherein X1 is E and X2 is selected from the group consisting of T, H, A, Y, N, E and S preferably in the group consisting of H, A, Y, N, E and S; optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 17;
(b) a light chain variable region (VL) comprising or consisting of an amino acid sequence of SEQ ID NO: 26, wherein X is G or T, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 26.

In another embodiment, the anti-human-PD-1 humanized antibody comprised in the bifunctional molecule comprises:
(a) a heavy chain variable region (VH) comprising or consisting of an amino acid sequence of SEQ ID NO: 18, 19, 20, 21, 22, 23, 24 or 25, optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 18, 19, 20, 21, 22, 23, 24 or 25, respectively;
(b) a light chain variable region (VL) comprising or consisting of an amino acid sequence of SEQ ID NO: 27 or SEQ ID NO: 28, optionally with one, two or three modification(s) selected from substitution(s), addition(s), deletion(s) and any combination thereof at any position positions 3, 4, 7, 14, 17, 18, 28, 29, 33, 34, 39, 42, 44, 50, 81, 88, 94, 97, 99 and 105 of SEQ ID NO: 27 or SEQ ID NO: 28.

In another embodiment, the anti-human-PD-1 humanized antibody comprised in the bifunctional molecule comprises:
(a) a heavy chain variable region (VH) comprising or consisting of an amino acid sequence of SEQ ID NO: 18 optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 18 respectively; and (b) a light chain variable region (VL) comprising or consisting of an amino acid sequence of SEQ ID NO: 27, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 27; or
(a) a heavy chain variable region (VH) comprising or consisting of an amino acid sequence of SEQ ID NO: 19 optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 19 respectively; and (b) a light chain variable region (VL) comprising or consisting of an amino acid sequence of SEQ ID NO: 27, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 27; or
(a) a heavy chain variable region (VH) comprising or consisting of an amino acid sequence of SEQ ID NO: 20 optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 20 respectively; and (b) a light chain variable region (VL) comprising or consisting of an amino acid sequence of SEQ ID NO: 27, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 27; or
(a) a heavy chain variable region (VH) comprising or consisting of an amino acid sequence of SEQ ID NO: 21 optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 21 respectively; and (b) a light chain variable region (VL) comprising or consisting of an amino acid sequence of SEQ ID NO: 27, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 27; or
(a) a heavy chain variable region (VH) comprising or consisting of an amino acid sequence of SEQ ID NO: 22 optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 22 respectively; and (b) a light chain variable region (VL) comprising or consisting of an amino acid sequence of SEQ ID NO: 27, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 27; or
(a) a heavy chain variable region (VH) comprising or consisting of an amino acid sequence of SEQ ID NO: 23 optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 23 respectively; and (b) a light chain variable region (VL) comprising or consisting of an amino acid sequence of SEQ ID NO: 27, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 27; or
(a) a heavy chain variable region (VH) comprising or consisting of an amino acid sequence of SEQ ID NO: 24 optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 24 respectively; and (b) a light chain variable region (VL) comprising or consisting of an amino acid sequence of SEQ ID NO: 27, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 27; or
(a) a heavy chain variable region (VH) comprising or consisting of an amino acid sequence of SEQ ID NO: 25 optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 25; and (b) a light chain variable region (VL) comprising or consisting of an amino acid sequence of SEQ ID NO: 27, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 27; or
(a) a heavy chain variable region (VH) comprising or consisting of an amino acid sequence of SEQ ID NO: 18 optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 18 respectively; and (b) a light chain variable region (VL) comprising or consisting of an amino acid sequence of SEQ ID NO: 28, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 28. ; or
(a) a heavy chain variable region (VH) comprising or consisting of an amino acid sequence of SEQ ID NO: 19 optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 19 respectively; and (b) a light chain variable region (VL) comprising or consisting of an amino acid sequence of SEQ ID NO: 28, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 28; or
(a) a heavy chain variable region (VH) comprising or consisting of an amino acid sequence of SEQ ID NO: 20 optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 20 respectively; and (b) a light chain variable region (VL) comprising or consisting of an amino acid sequence of SEQ ID NO: 28, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 28; or
(a) a heavy chain variable region (VH) comprising or consisting of an amino acid sequence of SEQ ID NO: 21 optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 21 respectively; and (b) a light chain variable region (VL) comprising or consisting of an amino acid sequence of SEQ ID NO: 28, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 28; or
(a) a heavy chain variable region (VH) comprising or consisting of an amino acid sequence of SEQ ID NO: 22 optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 22 respectively; and (b) a light chain variable region (VL) comprising or consisting of an amino acid sequence of SEQ ID NO: 28, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 28. ; or
(a) a heavy chain variable region (VH) comprising or consisting of an amino acid sequence of SEQ ID NO: 23 optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 23 respectively; and (b) a light chain variable region (VL) comprising or consisting of an amino acid sequence of SEQ ID NO: 28, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 28; or
(a) a heavy chain variable region (VH) comprising or consisting of an amino acid sequence of SEQ ID NO: 24 optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 24 respectively; and (b) a light chain variable region (VL) comprising or consisting of an amino acid sequence of SEQ ID NO: 28, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 28; or
(a) a heavy chain variable region (VH) comprising or consisting of an amino acid sequence of SEQ ID NO: 25 optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 25; and (b) a light chain variable region (VL) comprising or consisting of an amino acid sequence of SEQ ID NO: 28, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 28.

In a particular aspect, the modifications are substitutions, in particular conservative substitutions.

### CH-CL

In one embodiment, the heavy chain (CH) and the light chain (CL) comprises the VL and VH sequences as described hereabove.

In a particular embodiment, the anti-human-PD-1 antibody comprised in the bifunctional molecule comprises:
(a) a heavy chain comprising or consisting of an amino acid sequence selected from the group consisting of SEQ ID NO: 29, 30, 31, 32, 33, 34, 35 or 36, optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 29, 30, 31, 32, 33, 34, 35 or 36, respectively, and
(b) a light chain comprising or consisting of an amino acid sequence of SEQ ID NO: 37 or SEQ ID NO: 38, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 37 or SEQ ID NO: 38.

In another embodiment, the anti-human-PD-1 humanized antibody comprised in the bifunctional molecule comprises:
(a) a heavy chain comprising or consisting of an amino acid sequence selected from the group consisting of SEQ ID NO: 29, optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 29, and (b) a light chain comprising or consisting of an amino acid sequence of SEQ ID NO: 37, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 37; or
(a) a heavy chain comprising or consisting of an amino acid sequence selected from the group consisting of SEQ ID NO: 30, optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 30, and (b) a light chain comprising or consisting of an amino acid sequence of SEQ ID NO: 37, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 37; or
(a) a heavy chain comprising or consisting of an amino acid sequence selected from the group consisting of SEQ ID NO: 31, optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 31, and (b) a light chain comprising or consisting of an amino acid sequence of SEQ ID NO: 37, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 37; or
(a) a heavy chain comprising or consisting of an amino acid sequence selected from the group consisting of SEQ ID NO: 32, optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 32, and (b) a light chain comprising or consisting of an amino acid sequence of SEQ ID NO: 37, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 37; or
(a) a heavy chain comprising or consisting of an amino acid sequence selected from the group consisting of SEQ ID NO: 33, optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 33, and (b) a light chain comprising or consisting of an amino acid sequence of SEQ ID NO: 37, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 37; or
(a) a heavy chain comprising or consisting of an amino acid sequence selected from the group consisting of SEQ ID NO: 34, optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 34, and (b) a light chain comprising or consisting of an amino acid sequence of SEQ ID NO: 37, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 37; or
(a) a heavy chain comprising or consisting of an amino acid sequence selected from the group consisting of SEQ ID NO: 35, optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 35, and (b) a light chain comprising or consisting of an amino acid sequence of SEQ ID NO: 37, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 37; or
(a) a heavy chain comprising or consisting of an amino acid sequence selected from the group consisting of SEQ ID NO: 36, optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 36, and (b) a light chain comprising or consisting of an amino acid sequence of SEQ ID NO: 37, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 37; or
(a) a heavy chain comprising or consisting of an amino acid sequence selected from the group consisting of SEQ ID NO: 29, optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 29, and (b) a light chain comprising or consisting of an amino acid sequence of SEQ ID NO: 38, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 38; or
(a) a heavy chain comprising or consisting of an amino acid sequence selected from the group consisting of SEQ ID NO: 30, optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 30, and (b) a light chain comprising or consisting of an amino acid sequence of SEQ ID NO: 38, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 38; or
(a) a heavy chain comprising or consisting of an amino acid sequence selected from the group consisting of SEQ ID NO: 31, optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 31, and (b) a light chain comprising or consisting of an amino acid sequence of SEQ ID NO: 38, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 38; or
(a) a heavy chain comprising or consisting of an amino acid sequence selected from the group consisting of SEQ ID NO: 32, optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 32, and (b) a light chain comprising or consisting of an amino acid sequence of SEQ ID NO: 38, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 38; or
(a) a heavy chain comprising or consisting of an amino acid sequence selected from the group consisting of SEQ ID NO: 33, optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 33, and (b) a light chain comprising or consisting of an amino acid sequence of SEQ ID NO: 38, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 38; or
(a) a heavy chain comprising or consisting of an amino acid sequence selected from the group consisting of SEQ ID NO: 34, optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 34, and (b) a light chain comprising or consisting of an amino acid sequence of SEQ ID NO: 38, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 38; or
(a) a heavy chain comprising or consisting of an amino acid sequence selected from the group consisting of SEQ ID NO: 35, optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 35, and (b) a light chain comprising or consisting of an amino acid sequence of SEQ ID NO: 38, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 38; or
(a) a heavy chain comprising or consisting of an amino acid sequence selected from the group consisting of SEQ ID NO: 36, optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 36, and (b) a light chain comprising or consisting of an amino acid sequence of SEQ ID NO: 38, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 38.

Preferably, the modifications are substitutions, in particular conservative substitutions.

### Fc and hinge region

Several researches to develop therapeutic antibodies had led to engineer the Fc regions to optimize antibody properties allowing the generation of molecules that are better suited to the pharmacology activity required of them. The Fc region of an antibody mediates its serum half-life and effector functions, such as complement-dependent cytotoxicity (CDC), antibody-dependent cellular cytotoxicity (ADCC) and antibody-dependent cell phagocytosis (ADCP). Several mutations located at the interface between the CH2 and CH3 domains, such as T250Q/M428L, M252Y/S254T/T256E and H433K/N434F, have been shown to increase the binding affinity to FcRn and the half-life of IgG1 in vivo. However, there is not always a direct relationship between increased FcRn binding and improved half-life. One approach to improve the efficacy of a therapeutic antibody is to increase its serum persistence, thereby allowing higher circulating levels, less frequent administration and reduced doses. Engineering Fc regions may be desired to either reduce or increase the effector function of the antibody. For antibodies that target cell-surface molecules, especially those on immune cells, abrogating effector functions is required. Conversely, for antibodies intended for oncology use, increasing effector functions may improve the therapeutic activity. The four human IgG isotypes bind the activating Fcy receptors (FcyRI, FcyRlla, FcyRllla), the inhibitory FcyRllb receptor, and the first component of complement (C1q) with different affinities, yielding very different effector functions. Binding of IgG to the FcyRs or C1q depends on residues located in the hinge region and the CH2 domain. Two regions of the CH2 domain are critical for FcyRs and C1q binding, and have unique sequences in IgG2 and IgG4.

The humanized antibody according to the invention optionally comprises at least a portion of an immunoglobulin constant region (Fc), typically that of a human or humanized immunoglobulin. Preferably, the Fc region is a part of the humanized anti-hPD-1 antibody described herein. The humanized anti-hPD1 antibody comprised in the bifunctional molecule of the invention can include a constant region of an immunoglobulin or a fragment, analog, variant, mutant, or derivative of the constant region. As well known by one skilled in the art, the choice of IgG isotypes of the heavy chain constant domain centers on whether specific functions are required and the need for a suitable in vivo half-life. For example, antibodies designed for selective eradication of cancer cells typically require an active isotype that permits complement activation and effector-mediated cell killing by antibody-dependent cell-mediated cytotoxicity. Both human IgG1 and IgG3 (shorter half-life) isotypes meet these criteria, particularly human IgG1 isotype (wild type and variants). In particular, depending of the IgG isotype of the heavy chain constant domain (particularly human wild type and variants IgG1 isotype), the humanized anti-hPD1 antibody of the invention can be cytotoxic towards cells expressing PD-1 via a CDC, ADCC and/or ADCP mechanism. In fact, the fragment crystallisable (Fc) region interacts with a variety of accessory molecules to mediate indirect effector functions such as antibody-dependent cellular cytotoxicity (ADCC), antibody-dependent cellular phagocytosis (ADCP) and complement-dependent cytotoxicity (CDC).

In preferred embodiments, the constant region is derived from a human immunoglobulin heavy chain, for example, IgG1, IgG2, IgG3, IgG4, or other classes. In a further aspect, the human constant region is selected from the group consisting of IgG1, IgG2, IgG2, IgG3 and IgG4. Preferably, the humanized anti-PD1 antibody comprises an IgG1 or an IgG4 Fc-region.

In a particular aspect, the humanized anti-PD1 antibody comprises a human IgG1 Fc region, optionally with a substitution or a combination of substitutions selected from the group consisting of T250Q/M428L; M252Y/S254T/T256E + H433K/N434F; E233P/L234V/L235A/G236A + A327G/A330S/P331S; E333A; S239D/A330L/I332E; P257I/Q311; K326W/E333S; S239D/I332E/G236A; N297A; L234A/L235A; N297A + M252Y/S254T/T256E; K444A and K322A, preferably selected from the group consisting of N297A optionally in combination with M252Y/S254T/T256E, and L234A/L235A.

More preferably, the humanized anti-hPD1 antibody comprises an IgG4 Fc-region, optionally with a substitution or a combination of substitutions selected from the group consisting of S228P; L234A/L235A, S228P + M252Y/S254T/T256E and K444A. Even more preferably, the humanized anti-hPD1 antibody comprised in the bifunctional molecule according to the invention comprises an IgG4 Fc-region with a S228P that stabilizes the IgG4.

In one embodiment, the anti-PD1 antibody comprises a truncated Fc region or a fragment of the Fc region. In one embodiment, the constant region includes a CH2 domain. In another embodiment, the constant region includes CH2 and CH3 domains or includes hinge-CH2-CH3. Alternatively, the constant region can include all or a portion of the hinge region, the CH2 domain and/or the CH3 domain. In a preferred embodiment, the constant region contains a CH2 and/or a CH3 domain derived from a human IgG4 heavy chain.

In another embodiment, the constant region includes a CH2 domain and at least a portion of a hinge region. The hinge region can be derived from an immunoglobulin heavy chain, e.g., IgG1, IgG2, IgG3, IgG4, or other classes. Preferably, the hinge region is derived from human IgG1, IgG2, IgG3, IgG4, or other suitable classes, mutated or not. More preferably the hinge region is derived from a human IgG1 heavy chain. In one embodiment, the constant region includes a CH2 domain derived from a first antibody isotype and a hinge region derived from a second antibody isotype. In a specific embodiment, the CH2 domain is derived from a human IgG2 or IgG4 heavy chain, while the hinge region is derived from an altered human IgG1 heavy chain.

In one embodiment, the constant region contains a mutation that reduces affinity for an Fc receptor or reduces Fc effector function. For example, the constant region can contain a mutation that eliminates the glycosylation site within the constant region of an IgG heavy chain.

In another embodiment, the constant region includes a CH2 domain and at least a portion of a hinge region. The hinge region can be derived from an immunoglobulin heavy chain, e.g., IgG1, IgG2, IgG3, IgG4, or other classes. Preferably, the hinge region is derived from human IgG1, IgG2, IgG3, IgG4, or other suitable classes. The IgG1 hinge region has three cysteines, two of which are involved in disulfide bonds between the two heavy chains of the immunoglobulin. These same cysteines permit efficient and consistent disulfide bonding formation between Fc portions. Therefore, a preferred hinge region of the present invention is derived from IgG1, more preferably from human IgG1. In some embodiments, the first cysteine within the human IgG1 hinge region is mutated to another amino acid, preferably serine. The IgG2 isotype hinge region has four disulfide bonds that tend to promote oligomerization and possibly incorrect disulfide bonding during secretion in recombinant systems. A suitable hinge region can be derived from an IgG2 hinge; the first two cysteines are each preferably mutated to another amino acid. The hinge region of IgG4 is known to form interchain disulfide bonds inefficiently. However, a suitable hinge region for the present invention can be derived from the IgG4 hinge region, preferably containing a mutation that enhances correct formation of disulfide bonds between heavy chain-derived moieties (Angal S, et al. (1993) Mol. Immunol., 30:105-8). More preferably the hinge region is derived from a human IgG4 heavy chain.

In one embodiment, the constant region includes a CH2 domain derived from a first antibody isotype and a hinge region derived from a second antibody isotype. In a specific embodiment, the CH2 domain is derived from a human IgG4 heavy chain, while the hinge region is derived from an altered human IgG1 heavy chain.

In accordance with the present invention, the constant region can contain CH2 and/or CH3 domains and a hinge region that are derived from different antibody isotypes, i.e., a hybrid constant region. For example, in one embodiment, the constant region contains CH2 and/or CH3 domains derived from IgG2 or IgG4 and a mutant hinge region derived from IgG1. Alternatively, a mutant hinge region from another IgG subclass is used in a hybrid constant region. For example, a mutant form of the IgG4 hinge that allows efficient disulfide bonding between the two heavy chains can be used. A mutant hinge can also be derived from an IgG2 hinge in which the first two cysteines are each mutated to another amino acid. Assembly of such hybrid constant regions has been described in U.S. Patent Publication No. 20030044423.

In one embodiment, the constant region can contain CH2 and/or CH3 has one of the mutations described in the Table D below, or any combination thereof.

**Table D: Suitable human engineered Fc domain of an antibody. Numbering of residues in the heavy chain constant region is according to EU numbering (Edelman, G.M. et al., Proc. Natl. Acad. USA, 63, 78-85 (1969); www.imgt.org/IMGTScientificChart/Numbering/Hu_IGHGnber.html#refs).**

| **Engineered Fc** | **Isotype** | **Mutations** | **FcR/C1q Binding** | **Effector Function** |
|---|---|---|---|---|
| hIgG1e1-Fc | IgG1 | T250Q/M428L | Increased binding to FcRn | Increased half-life |
| hIgG1e2-Fc | IgG1 | M252Y/S254T/T256E + H433K/N434F | Increased binding to FcRn | Increased half-life |
| hIgG1e3-Fc | IgG1 | E233P/L234V/L235A/G236A + A327G/A330S/P331S | Reduced binding to FcγRI | Reduced ADCC and CDC |
| hIgG1e4-Fc | IgG1 | E333A | Increased binding to FcγRIIIa | Increased ADCC and CDC |
| hIgG1e5-Fc | IgG1 | S239D/A330L/I332E | Increased binding to FcγRIIIa | Increased ADCC |
| hIgG1e6-Fc | IgG1 | P257I/Q311 | Increased binding to FcRn | Unchanged half-life |
| hIgG1e7-Fc | IgG1 | K326W/E333S | Increased binding to C1q | Increased CDC |
| hIgG1e9-Fc | IgG1 | S239D/I332E/G236A | Increased FcγRIIa/FcγRIIb ratio | Increased macrophage phagocytosis |
| hIgG1e9-Fc | IgG1 | N297A | Reduced binding to FcγRI | Reduced ADCC and CDC |
| hIgG1e9-Fc | IgG1 | LALA (L234A/L235A) | Reduced binding to FcγRI | Reduced ADCC and CDC |
| hIgG1e10-Fc | IgG1 | N297A + YTE (N298A + M252Y/S254T/T256E) | Reduced binding to FcγRI Increased binding to FcRn | Reduced ADCC and CDC Increased half-life |
| hIgG1e11-Fc | IgG1 | K322A | Reduced binding to C1q | Reduced CDC |
| hIgG1e3-Fc | IgG1 | K444A | | Abolition of cleavage motif at the Cter of the antibody |
| hIgG2e1-Fc | IgG4 | S228P | - | Reduced Fab-arm exchange |
| hIgG4e1-Fc | IgG4 | LALA (L234A/L235A) | Increased binding to FcRn | Increased half-life |
| hIgG4e2-Fc | IgG4 | S228P+ YTE (S228P + M252Y/S254T/T256E) | - Increased binding to FcRn | Reduced Fab-arm exchange Increased half-life |
| hIgG4e3-Fc | IgG4 | K444A | | Abolition of cleavage motif at the Cter of the antibody |

In certain embodiments, amino acid modifications may be introduced into the Fc region of an antibody provided herein to generate an Fc region variant. In certain embodiments, the Fc region variant possesses some, but not all, effector functions. Such antibodies may be useful, for example, in applications in which the half-life of the antibody *in vivo* is important, yet certain effector functions are unnecessary or deleterious. Examples of effector functions include complement-dependent cytotoxicity (CDC) and antibody-directed complement-mediated cytotoxicity (ADCC). Numerous substitutions or substitutions or deletions with altered effector function are known in the art.

In one embodiment, the constant region contains a mutation that reduces affinity for an Fc receptor or reduces Fc effector function. For example, the constant region can contain a mutation that eliminates the glycosylation site within the constant region of an IgG heavy chain. Preferably, the CH2 domain contains a mutation that eliminates the glycosylation site within the CH2 domain.

In one embodiment, the anti-hPD1 according to the invention has a heavy chain constant domain of SEQ ID NO. 39 and/or a light chain constant domain of SEQ ID. 40, particularly a heavy chain constant domain of SEQ ID NO. 39 and a light chain constant domain of SEQ ID. 40.

In another embodiment, the anti-hPD1 according to the invention has a heavy chain constant domain of SEQ ID NO: 57 and/or a light chain constant domain of SEQ ID. 40, particularly a heavy chain constant domain of SEQ ID NO:57 and a light chain constant domain of SEQ ID. 40.

**Table E. Example of a heavy chain constant domain and a light chain constant domain suitable for the humanized antibodies according to the invention.**

| | |
|---|---|
| Heavy chain constant domain (IgG4m-S228P) SEQ ID NO: 39 | ASTKGPSVFPLAPCSRSTSESTAALGCLVKDYFPEPVTVSWNSGALTSGVHTFPAVL QSSGLYSLSSVVTVPSSSLGTKTYTCNVDHKPSNTKVDKRVESKYGPPCPPCPAPEFL GGPSVFLFPPKPKDTLMISRTPEVTCVVVDVSQEDPEVQFNWYVDGVEVHNAKTK PREEQFNSTYRVVSVLTVLHQDWLNGKEYKCKVSNKGLPSSIEKTISKAKGQPREPQ VYTLPPSQEEMTKNQVSLTCLVKGFYPSDIAVEWESNGQPENNYKTTPPVLDSDGS FFLYSRLTVDKSRWQEGNVFSCSVMHEALHNHYTQKSLSLSPGK |
| Light chain constant domain (CLkappa) SEQ ID NO: 40 | |
| Heavy chain constant domain (IgG1m-N298A) SEQ ID NO:57 | ASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCDKTHTCPPCPAP ELLGGPSVFLFPPKPKDTLMISRTPEVTCVVVDVSHEDPEVKFNWYVDGVEVHNAK TKPREEQYASTYRVVSVLTVLHQDWLNGKEYKCKVSNKALPAPIEKTISKAKGQPRE PQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPGK |

The alteration of amino acids near the junction of the Fc portion and the non-Fc portion can dramatically increase the serum half-life of the Fc molecule (PCT publication WO 01/58957). Accordingly, the junction region of a protein or polypeptide of the present invention can contain alterations that, relative to the naturally-occurring sequences of an immunoglobulin heavy chain and erythropoietin, preferably lie within about 10 amino acids of the junction point. These amino acid changes can cause an increase in hydrophobicity. In one embodiment, the constant region is derived from an IgG sequence in which the C-terminal lysine residue is replaced. Preferably, the C-terminal lysine of an IgG sequence is replaced with a non-lysine amino acid, such as alanine or leucine, to further increase serum half-life. In particular, K444 amino acid in the IgG1 or IgG4 domain may be substituted by an alanine to reduce proteolytic cleavage. Then, in one embodiment, the anti-PD1 antibody comprises at least one further amino acid substitution consisting of K444A.

In one embodiment, the anti-PD1 antibody comprises an additional cysteine residue at the C-terminal domain of the IgG to create an additional disulfide bond and potentially restrict the flexibility of the bifunctional molecule.

In certain embodiments, an antibody may be altered to increase, decrease or eliminate the extent to which it is glycosylated.

### Humanness

For purposes of this disclosure, "humanness" is measured using the T20 score analyzer to quantify the humanness of the variable region of monoclonal antibodies as described in Gao S H, Huang K, Tu H, Adler A S. BMC Biotechnology. 2013: 13:55.

A web-based tool is provided to calculate the T20 score of antibody sequences using the T20 Cutoff Human Databases: http://abAnalyzer.lakepharma.com. In computing a T20 score, an input VH, VK, or VL variable region protein sequence is first assigned Kabat numbering, and CDR residues are identified. The full-length sequence or the framework only sequence (with CDR residues removed) is compared to every sequence in a respective antibody database using the blastp protein-protein BLAST algorithm. The sequence identity between each pairwise comparison is isolated, and after every sequence in the database has been analyzed, the sequences are sorted from high to low based on the sequence identity to the input sequence. The percent identity of the Top 20 matched sequences is averaged to obtain the T20 score.

T20 humanness score is a parameter commonly used in the field of antibody humanization first disclosed by Gao et al (BMC Biotechnol, 2013, 13, 55). T20 humanness score is usually used in patent application for defining a humanized antibody (e.g., WO15161311, WO17127664, WO18136626, WO18190719, WO19060750, or WO19170677).

For each chain type (VH, VK, VL) and sequence length (full-length or framework only) in the "All Human Databases," each antibody sequence was scored with its respective database using the T20 score analyzer. The T20 score was obtained for the top 20 matched sequences after the input sequence itself was excluded (the percent identity of sequences 2 through 21 were averaged since sequence 1 was always the input antibody itself). The T20 scores for each group were sorted from high to low. The decrease in score was roughly linear for most of the sequences; however, the T20 scores for the bottom ^{~}15% of antibodies started decreasing sharply. Therefore, the bottom 15 percent of sequences were removed and the remaining sequences formed the T20 Cutoff Human Databases, where the T20 score cutoff indicates the lowest T20 score of a sequence in the new database.

As used herein, a "humanized antibody" is one that has a T20 humanness score of at least 80% or at least 85%, more preferably at least 88%, even more preferably at least 90 %, most preferably a T20 humanness score comprised between 85% and 95%, preferably between 88% and 92%.

Accordingly, the humanized anti-PD1 antibody according to the disclosure comprised in the bifunctional molecule has a T20 humanness score of at least 80% or at least 85%, more preferably at least 88%, even more preferably at least 90 %, most preferably a T20 humanness score comprised between 85% and 95%, preferably between 88% and 92%.

### Peptide Linker

This invention includes a bifunctional molecule which may comprise a peptide linker between the humanized anti-PD-1 antibody and the immunotherapeutic agent. The peptide linker usually has a length and flexibility enough to ensure that the two protein elements connected with the linker in between have enough freedom in space to exert their functions and avoid influences of the formation of a-helix and β-fold on the stability of the recombinant bifunctional molecule.

In an aspect of the disclosure, the humanized anti-hPD1 antibody is preferably linked to an immunotherapeutic agent by a peptide linker. In other words, the invention relates to bifunctional molecule comprising an anti-PD1 antibody as detailed herein, with a chain, e.g., the light or heavy chain, preferably the heavy chain, is linked to an immunotherapeutic agent through a peptide linker. As used herein, the term "linker" refers to a sequence of at least one amino acid that links the immunotherapeutic agent and the anti-PD-1 immunoglobulin sequence portion. Such a linker may be useful to prevent steric hindrances. The linker is usually 3-44 amino acid residues in length. Preferably, the linker has 3-30 amino acid residues. In some embodiments, the linker has 3, 4, 5, 6, 7, 8, 9, 10, 11, 12, 13, 14, 15, 16, 17, 18, 19, 20, 21, 22, 23, 24, 25, 26, 27, 28, 29 or 30 amino acid residues.

In an embodiment, the invention relates to a bifunctional molecule comprising a humanized anti-PD-1 antibody as defined above and an immunotherapeutic agent, wherein a chain of the antibody, e.g., the light or heavy chain, preferably the heavy chain, even more preferably the C-terminus of the heavy or light chain is linked to an immunotherapeutic agent, preferably to the N-terminus of the immunotherapeutic agent, by a peptide linker.

In a particular aspect, the invention relates to a bifunctional molecule comprising a humanized anti-hPD-1 antibody as defined above, wherein the immunotherapeutic agent is linked to the C-terminal end of the heavy chain of said antibody (e.g., the C-terminal end of the heavy chain constant domain), preferably by a peptide linker.

In an embodiment, the invention relates to bifunctional molecule comprising a humanized anti-PD-1 antibody as defined above, wherein the immunotherapeutic agent is linked to the C-terminal end of the light chain of said antibody (e.g., the C-terminal end of the light chain constant domain), preferably by a peptide linker.

The linker sequence may be a naturally occurring sequence or a non-naturally occurring sequence. If used for therapeutic purposes, the linker is preferably non-immunogenic in the subject to which the bifunctional molecule is administered. One useful group of linker sequences are linkers derived from the hinge region of heavy chain antibodies as described in WO 96/34103 and WO 94/04678. Other examples are poly-alanine linker sequences. Further preferred examples of linker sequences are Gly/Ser linkers of different length including (Gly4Ser)₄, (Gly4Ser)₃, (Gly4Ser)₂, Gly4Ser, Gly3Ser, Gly3, Gly2ser and (Gly3Ser2)₃, in particular (Gly4Ser)₃.

In one embodiment, the linker comprised in the bifunctional molecule is selected in the group consisting of (Gly4Ser)₄, (Gly4Ser)₃, (Gly4Ser)₂, Gly4Ser, Gly3Ser, Gly3, Gly2ser and (Gly3Ser2)₃, preferably is (Gly4Ser)₃.

In an embodiment, the invention relates to a bifunctional molecule that comprises a humanized anti-PD-1 antibody as defined above wherein the antibody is linked to an immunotherapeutic agent by a linker sequence, preferably selected from the group consisting of (GGGGS)₃, (GGGGS)₄, (GGGGS)₂, GGGGS, GGGS, GGG, GGS and (GGGS)₃, even more preferably by (GGGGS)₃.

Preferably, the heavy chain, preferably the C terminus of the heavy chain of the anti-PD-1 antibody is genetically fused via a flexible (Gly₄Ser)₃ linker to the N-terminus of the immunotherapeutic agent. At the fusion junction, the C-terminal lysine residue of the antibody heavy chain can be mutated to alanine to reduce proteolytic cleavage.

Preferably, the heavy chain, preferably the C terminus of the light chain of the anti-PD-1 antibody is genetically fused via a flexible (Gly₄Ser)₃ linker to the N-terminus of the immunotherapeutic agent. At the fusion junction, the C-terminal lysine residue of the antibody light chain can be mutated to alanine to reduce proteolytic cleavage.

### Immunotherapeutic agent

In the invention, the bifunctional molecule comprises the humanized anti-hPD-1 antibodies or fragments thereof as described above linked, preferably via a peptide linker, to an immunotherapeutic agent. Such humanized antibodies may also be referred to as "bifunctional antibodies" because they have two therapeutic effects: a first effect resulting from the interaction of the humanized anti-hPD-1 antibody with hPD-1 and a second effect resulting from the interaction of the immunotherapeutic agent with its ligand or receptor.

As used herein, the term "immunotherapeutic agents" refers to agents that have an effect on the immune system, in particular an inhibitory effect or a stimulatory effect, preferably a stimulatory effect. The agents can be for instance a molecule having an effect on the activation or inhibition on cells of the immune system. For instance, the immunotherapeutic agents can be selected from: T-cell growth factors, in particular growth factors to increase number and repertoire of naive T cells, growth factors to increase the number of dendritic cells (DCs), agonists to activate DCs and other antigen-presenting cells (APCs), adjuvants to allow and augment cancer vaccines, agonists to activate and stimulate T cells, inhibitors of T-cell checkpoint blockade, T-cell growth factors to increase the growth and survival of immune T cells, agents to inhibit, block, or neutralize cancer cell and immune cell-derived immunosuppressive cytokine. Preferably, the immunotherapeutic agent is a peptide, a polypeptide or a protein. In one embodiment, the immunotherapeutic agent is a non-antibody entity or portion. The immunotherapeutic agent can also consist in one or more other binding molecules, an antibody binding mimetic, a receptor or an extracellular domain thereof, a ligand of a receptor or a fragment thereof having the same functional activity. As used herein an "antibody mimetic" is related to an organic compound that, like antibodies, can specifically bind antigens, but that are not structurally related to antibodies. They are usually peptides or proteins with a molar mass of about 3 to 20 kDa. Examples of antibody mimetic comprises affilins, affimers, affitins, anticalins and avimers.

The immunotherapeutic agent may be mutated or altered so that the biological activity of the immunotherapeutic agent is altered, e.g. the biological activity is increased, decreased or totally inhibited.

In one embodiment, the immunotherapeutic agent consists in a fragment thereof. Preferably, such fragment retains the biological activity of the immunotherapeutic agent.

In one aspect, the immunotherapeutic agent or fragment thereof has a size of at least 10 kDa, at least, 15 kDa, at least 20 kDa, at least 25 kDa, at least 30 kDa, at least 35 kDa, at least 40 kDa, at least 45 kDa or at least 50kDa. Preferably, the immunotherapeutic agent or fragment thereof has a size comprised between 10 kDa and 50 kDa, between 10 kDa and 40 kDa, between 10 kDa and 30 kDa, between 10 kDa and 20 kDa, between 20 kDa and 50 kDa, between 20 kDa and 40 kDa or between 20 kDa and 30 kDa.

Particularly, immunotherapeutic agents of the disclosure are selected from the group consisting of immune checkpoint blockers or activators, in particular of adaptive immune cells (T or B lymphocytes), therapeutic vaccines (DNA, RNA or peptide vaccines) or immunoconjugates such as antibody-drug conjugates. In one approach, the immunotherapeutic agent is a T cell inhibition checkpoint receptor protein on the T cell, (for example CTLA-4, BTLA, LAG-3, TIM-3, and LAIR1). In another approach, the immunotherapeutic agent is a counter-receptor or ligand on antigen presenting cells and tumor cells (which co-opt some of these counter-receptors for their own immune evasion), for example B7-DC, HVEM, TIM-4, B7-H3, or B7-H4.

Other immunotherapeutic agent of the disclosure can bind, without limitation, hormone receptors (e.g., estrogen, progesterone), cytokine receptors (i.e., type I, such as growth hormone receptor, prolactin, erythropoietin; type II; members of the immunoglobulin superfamily, such as interleukin-1; tumor necrosis factor receptor family, such as CD27, CD30, CD40; chemokine receptors, such as interleukin-8, CCR1, CXCR4; transforming growth factor (TGF) beta receptors); cell adhesion molecules (e.g., integrin); and vascular endothelial growth factor (VEGF) receptors (e.g., neurophilin (NRP) receptors, such as NRP1, NRP2).

Preferably, the immunotherapeutic agent is from human or derived from human.

In an aspect that is not claimed, the immunotherapeutic agent is selected from the group consisting of tumor targeting peptides, cytokines, cytokines receptors costimulatory molecules, inhibitory or coinhibitory molecules, preferably human transmembrane immune protein of type I or II, even more preferably the extracellular domain thereof, molecular chaperone inhibitors (e.g., a HSP90 inhibitor), or tubulin inhibitors (e.g., taxane) and/or stabilizers or DNA replication inhibitors or any anti-cancer agent or any derivatives and/or analogues thereof to provide an additional therapeutic benefit.

In a particular aspect of the disclosure that is not claimed, the immunotherapeutic agent is a molecule that targets an Fc receptor, e.g., human FcyRI (CD64) or a human Fcα receptor (CD89). Therefore, the disclosure includes bispecific molecules capable of binding both to FcyR or FcαR expressing effector cells (e.g., monocytes, macrophages or polymorphonuclear cells (PMNs)), and to target cells expressing PD-1. These bifunctional molecules target PD-1 expressing cells to effector cell and trigger Fc receptor-mediated effector cell activities, such as phagocytosis of PD-1 expressing cells, antibody dependent cell-mediated cytotoxicity (ADCC), cytokine release, or generation of superoxide anion.

For instance, the immunotherapeutic agent could be selected in the non-exhaustive list of Table F below comprising ICOSL, CD86, B7H4, B7H3, CD28H, PDL2, PDL1, DNAM, CTLA-4, Lag-3, TIGIT, 2B4, BTLA, HVEM, CD101, nectin-1, nectin-2, nectin-3, NELC-5, TLT-2, LFA-3, TIM3, TIM4, LAIR1, SIRPG, IL10R, IL6RA, IL-1R1, IL-1RAcP, IL6RB, TGFBRII, CSF1R, IL22R, VEGFR1, VEGFR2, VEGFR3, CD111, CD112, CD155, CD113, VISTA, CD244, OX40, , SIRPalpha, CD80, CD24, Siglec-10, Fas, IL15RA, SIRB1, SIRB2, LTBR, IL21R, GITR, CD40L, OX40L, FasL, TRAIL, TNF, LIGHT, APRIL, GITRL, CD30, CD70, CD40, CD27, CD30, CD153, RANK, CLEC3A, CLEC4A, CLEC4E, CLEC4L, CLEC51, CLEC6, CLEC7A, NKG2D, BTL-II, TGFRII, DECTIN-1, DC-SIGN, , LT-alpha, LT-beta, 4-1BBL, MINCLE, TGFβ, IL-1, IL-2, IL-4, IL-6, IL-7, IL-10, IL-12A, IL12B, IL-15, IL-21 and IL-18.

In a very specific embodiment, the immunotherapeutic agent is Interleukin-2 (IL-2), preferably a human IL-2, for example as disclosed under the UniProt accession number P60568 or a mutant or variant thereof. IL-2 can be mutated in various ways to reduce its toxicity and/or increase its efficacy. Hu et al. (Blood 101, 4853-4861 (2003), US Pat. Publ. No. 2003/0124678) have substituted the arginine residue in position 38 of IL-2 by tryptophan to eliminate IL-2's vasopermeability activity. Shanafelt et al. (Nature Biotechnol 18, 1 197-1202 (2000)) have mutated asparagine 88 to arginine to enhance selectivity for T cells over NK cells. Heaton et al. (Cancer Res 53, 2597-602 (1993); US Pat. No. 5,229, 109) have introduced two mutations, Arg38Ala and Phe42Lys, to reduce the secretion of proinflammatory cytokines from NK cells. Gillies et al. (US Pat. Publ. No. 2007/0036752) have substituted three residues of IL-2 (Asp20Thr, Asn88Arg, and Glnl26Asp) that contribute to affinity for the intermediate-affinity IL-2 receptor to reduce VLS. Gillies et al. (WO 2008/0034473) have also mutated the interface of IL-2 with CD25 by amino acid substitution Arg38Trp and Phe42Lys to reduce interaction with CD25 and activation of Treg cells for enhancing efficacy. In one aspect, the immunotherapeutic agent is an IL-2 mutant for example as described in WO 2012/107417 or WO 2018/184964. Mutants of human IL-2 (hIL-2) with decreased affinity to CD25 may for example be generated by amino acid substitution at amino acid position 3, 35, 38, 42, 43, 45 or 72 or combinations thereof, corresponding to residues position of human IL-2. Preferably, the mutant IL-2 is a human IL-2 molecule comprising the amino acid substitutions T3A, F42A, Y45A, L72G and/or C125A, preferably F42A, Y45A and L72G, more preferably T3A, F42A, Y45A, L72G and C125A, for example as disclosed in WO 2018/184964. Even more preferably, the immunotherapeutic agent is an IL-2 mutant having the amino-acid sequence set forth is SEQ ID NO:58 with the substitutions F42A, Y45A and L72G, preferably T3A, F42A, Y45A, L72G and C125A.

The Inventors have observed that when the immunotherapeutic agent is a Type I or Type II transmembrane protein, the immunotherapeutic agent remain functional when it is grafted on the heavy chain and when it is grafted on the light chain of the humanized anti-hPD-1 according to the invention.

Preferably, the bifunctional molecule comprises a humanized anti-hPD1 antibody linked to a type I or a type II transmembrane protein, in particular the extracellular domain thereof. Even more preferably, the bifunctional molecule comprises a type I or a type II transmembrane protein, in particular the extracellular domain thereof, linked to the carboxy-terminus of a humanized anti-hPD1 antibody as described herein, preferably by a peptide linker.

Type I transmembrane proteins are characterized by an N-terminus outside of cell membrane and a C-terminus inside of cell membrane. Type I transmembrane proteins usually include members of immunoglobulin super family (CD4, CD8, CD28, CTLA4, CD86, etc.), receptor kinases (TGF-β receptor, EGFR, VEGFR, PDGFR, HGF receptor, etc.), and cytokine receptors (TNF receptor, RANK, IL-6 receptor, CSF1 receptor, c-kit, etc.). Type I transmembrane proteins could induce various biological reactions after their binding to corresponding ligands. There is no special restriction on the type I transmembrane protein used in this invention. Thus, all the type I transmembrane proteins with biological activity can be used in this invention. For instance, the type I transmembrane proteins could be selected in the non-exhaustive list comprising ICOSL, CD86, B7H4, B7H3, CD28H, PDL2, PDL1, DNAM, CTLA-4, Lag-3, TIGIT, 2B4, BTLA, HVEM, CD101, nectin-1, nectin-2, nectin-3, NELC-5, TLT-2, LFA-3, TIM3, TIM4, LAIR1, SIRPG, IL10R, IL6RA, IL-1R1, IL-1RAcP, IL6RB, TGFBRII, CSF1R, IL22R, VEGFR1, VEGFR2, VEGFR3, CD111, CD112, CD155, CD113, VISTA, CD244, OX40, SIRPalpha, CD80, CD24, Siglec-10, Fas, IL15RA, SIRB1, SIRB2, LTBR, IL21R and GITR.

In a preferred embodiment, the bifunctional molecule according to the invention comprises a type I transmembrane protein, preferably selected from the group consisting of CD86, ICOSL, PD-L1, PD-L2, ICOSL, RANK, VEGFR1, VEGFR2, CTLA4, TGF-pRII, B7-H3, B7-H4, HVEM, BTLA, LAG3, TIM3, VISTA, CD111, CD113, TIGIT, SIRPalpha, CD80 and or a combination thereof.

Type II transmembrane proteins are characterized by a C-terminus outside of cell membrane and an N-terminus inside of cell membrane. Usually type II transmembrane proteins are C-Type lectins or C-Type lectin-like receptors. The extracellular domain of the receptor includes a carbohydrate -binding domain (or referred to as C-Type Lectin Domain, CTLD). Proteins with carbohydrate -binding domain are involved with various functions such as cell adhesion, platelet activation, immunity of pathogens, inducement of apoptosis, etc. There is no special restriction on the type II transmembrane proteins used in this invention. Thus, all the type II transmembrane proteins with biological activity can be used in this invention. For instance, the type I transmembrane proteins could be selected in the non-exhaustive list comprising CD40L, OX40L, FasL, TRAIL, TNF, LIGHT, APRIL, GITRL, CD30, CD70, CD40, CD27, CD30, CD153, RANK, CLEC3A, CLEC4A, CLEC4E, CLEC4L, CLEC51, CLEC6, CLEC7A, NKG2D, BTL-II, TGFRII, DECTIN-1, DC-SIGN, LT-alpha, LT-beta, 4-1BBL and MINCLE, preferably a member of the TNF family.

In a preferred embodiment, the bifunctional molecule according to the invention comprises a type II transmembrane protein, preferably selected from the group consisting of OX40L, DECTIN-1, NKG2D, DC-SIGN, 4-1BBL, MINCLE or a combination thereof.

In a particular aspect, the immunotherapeutic agent comprised in the bifunctional molecule is an immune checkpoint blocker or activator. It will be understood that such immunotherapeutic agent can be different to h-PD1, PD-L1, PD-L2 or to a molecule targeting and/or binding h-PD1, PD-L1 and/or PDL-2.

Numerous immune checkpoint blockers or activators are known in the art. In the context of the invention, examples of immune checkpoint blockers or activators of adaptive immune cells (B or T lymphocytes) that could be useful are CTLA-4, CD86, CD28, CD40, CD40L, ICOS, ICOS-L, OX40L, GITR, HVEM, BTLA, CD160, LIGHT, TNFRSF25, 2B4, CD48, Tim1, Tim3, Tim4, Gal9, LAG-3, CD40, CD40L, CD70, CD27, VISTA, B7H3, B7H4 (B7x), TIGIT, CD112, HHLA2 (B7-H7), TMIGD2 (CD28H), Butyrophilin-like2 (BTNL2), variants and fragments thereof, in particular CD86, 0X40L, ICOSL, variants and fragments thereof.

Preferably, the bifunctional molecule according to the invention comprises a humanized anti-PD-1 antibody or antigen-binding fragment thereof as defined above linked, preferably by a peptide linker, to an immunotherapeutic agent selected from the group consisting of CD86, ICOSL, PD-L1, PD-L2, ICOSL, RANK, VEGFR1, VEGFR2, CTLA4, TGF-pRII, B7-H3, B7-H4, HVEM, BTLA, LAG3, TIM3, VISTA, CD111, CD113, TIGIT, OX40L, DECTIN-1, NKG2D, DC-SIGN, and MINCLE.

In a preferred embodiment, the bifunctional molecule according to the invention comprises a cytokine as the immunotherapeutic agent, preferably selected from the group consisting of TGFβ, IL-1, IL-2, IL-6, IL-10, IL-12A, IL12B, IL-15, IL-21 and IL-18.

In one embodiment, the type I transmembrane protein, the type II protein or the cytokine or fragment thereof has a size of at least 10 kDa, at least, 15 kDa, at least 20 kDa, at least 25 kDa, at least 30 kDa, at least 35 kDa, at least 40 kDa, at least 45 kDa or at least 50kDa. Preferably, the immunotherapeutic agent has a size comprised between 10 kDa and 50 kDa, between 10 kDa and 40 kDa, between 10 kDa and 30 kDa, between 10 kDa and 20 kDa, between 20 kDa and 50 kDa, between 20 kDa and 40 kDa or between 20 kDa and 30 kDa.

In one embodiment, the immunotherapeutic agent comprised in the bifunctional molecule according to the invention is selected in the Table F below.

**Table F. Immunotherapeutic agent of interest.**

| **Categor y** | **Name** | **Official name** | **Uniprot reference** |
|---|---|---|---|
| | **ICOSL** | ICOS ligand (B7 homolog 2, B7-H2) (B7-like protein GI50) (B7-related protein 1, B7RP-1) (CD antigen CD275) | 075144 |
| | **CD86** | T-lymphocyte activation antigen CD86 (Activation B7-2 antigen) (B70) (BU63) (CTLA-4 counter-receptor B7.2) (FUN-1) (CD antigen CD86) | P42081 |
| | **B7H4** | V-set domain-containing T-cell activation inhibitor 1 (B7 homolog 4, B7-H4) (B7h.5) (Immune costimulatory protein B7-H4) (Protein B7S1) (T-cell costimulatory molecule B7x) | Q7Z7D3 |
| | **B7H3** | CD276 antigen (4Ig-B7-H3) (B7 homolog 3, B7-H3) (Costimulatory molecule) (CD antigen CD276) | Q5ZPR |
| | **CD28H** | Transmembrane and immunoglobulin domain-containing protein 2 (CD28 homolog) (Immunoglobulin and proline-rich receptor 1, IGPR-1) | Q96BF3 |
| | **PDL2** | Programmed cell death 1 ligand 2, PD-1 ligand 2, PD-L2, PDCD1 ligand 2, Programmed death ligand 2 (Butyrophilin B7-DC, B7-DC) (CD antigen CD273) | Q9BQ51 |
| | **PDL1** | Programmed cell death 1 ligand 1, PD-L1, PDCD1 ligand 1, Programmed death ligand 1 (B7 homolog 1, B7-H1) (CD antigen CD274) | Q9NZQ7 |
| | **DNAM** | D226 antigen (DNAX accessory molecule 1, DNAM-1) (CD antigen CD226) | Q15762 |
| **Type I** | **CTLA-4** | Cytotoxic T-lymphocyte protein 4 (Cytotoxic T-lymphocyte-associated antigen 4, CTLA-4) (CD antigen CD152) | P16410 |
| | **Lag-3** | Lymphocyte activation gene 3 protein, LAG-3 (Protein FDC) (CD antigen CD223) | P18627 |
| | **Tim3** | Hepatitis A virus cellular receptor 2, HAVcr-2 (T-cell immunoglobulin and mucin domain-containing protein 3, TIMD-3) (T-cell immunoglobulin mucin receptor 3, TIM-3) (T-cell membrane protein 3) | Q8TDQ0 |
| | **TIGIT** | T-cell immunoreceptor with Ig and ITIM domains (V-set and immunoglobulin domain-containing protein 9) (V-set and transmembrane domain-containing protein 3) | Q495A1 |
| | **2B4** | Natural killer cell receptor 2B4 (NK cell type I receptor protein 2B4, NKR2B4) (Non-MHC restricted killing associated) (SLAM family member 4, SLAMF4) (Signaling lymphocytic activation molecule 4) (CD antigen CD244) | Q07763 |
| | **BTLA** | B- and T-lymphocyte attenuator (B- and T-lymphocyte-associated protein) (CD antigen CD272) | Q7Z6A9 |
| | **HVEM** | Tumor necrosis factor receptor superfamily member 14 (Herpes virus entry mediator A, Herpesvirus entry mediator A, HveA) (Tumor necrosis factor receptor-like 2, TR2) (CD antigen CD270) | Q92956 |
| | **CD101** | Immunoglobulin superfamily member 2, IgSF2 (Cell surface glycoprotein V7) (Glu-Trp-Ile EWI motif-containing protein 101, EWI-101) (CD antigen CD101) | Q93033 |
| | **nectin-1** | Nectin-1 (Herpes virus entry mediator C, Herpesvirus entry mediator C, HveC) (Herpesvirus Ig-like receptor, HIgR) (Nectin cell adhesion molecule 1) (Poliovirus receptor-related protein 1) (CD antigen CD111) | Q15223 |
| | **nectin-2** | Nectin-2 (Herpes virus entry mediator B, Herpesvirus entry mediator B, HveB) (Nectin cell adhesion molecule 2) (Poliovirus receptor-related protein 2) (CD antigen CD112) | Q92692 |
| | **nectin-3** | Nectin-3 (CDw113) (Nectin cell adhesion molecule 3) (Poliovirus receptor-related protein 3) (CD antigen CD113) | Q9NQS3 |
| | **NELC-5** | Poliovirus receptor (Nectin-like protein 5, NECL-5) (CD antigen CD155) | P15151 |
| | **TLT-2** | Transmembrane and immunoglobulin domain-containing protein 2 (CD28 homolog) (Immunoglobulin and proline-rich receptor 1, IGPR-1) | Q5T2D2 |
| | **LFA-3** | Lymphocyte function-associated antigen 3, Ag3 (Surface glycoprotein LFA-3) (CD antigen CD58) | P19256 |
| | **TIM4** | T-cell immunoglobulin and mucin domain-containing protein 4, TIMD-4 (T-cell immunoglobulin mucin receptor 4, TIM-4) (T-cell membrane protein 4) | Q96H15 |
| | **LAIR1** | Leukocyte-associated immunoglobulin-like receptor 1, LAIR-1, hLAIR1(CD antigen CD305) | Q6GTX8 |
| | **SIRPG** | Signal-regulatory protein gamma, SIRP-gamma (CD172 antigen-like family member B) (Signal-regulatory protein beta-2, SIRP-b2, SIRP-beta-2) (CD antigen CD172g) | Q9P1W8 |
| | **IL10R** | Interleukin-10 receptor subunit beta, IL-10 receptor subunit beta, IL-10R subunit beta, IL-10RB (Cytokine receptor class-II member 4) (Cytokine receptor family 2 member 4, CRF2-4) (Interleukin-10 receptor subunit 2, IL-10R subunit 2, IL-10R2) (CD antigen CDw210b) | Q08334 |
| | **IL6RA** | Interleukin-6 receptor subunit alpha, IL-6 receptor subunit alpha, IL-6R subunit alpha, IL-6R-alpha, IL-6RA (IL-6R 1) (Membrane glycoprotein 80, gp80) (CD antigen CD126) | P08887 |
| | **IL-1R1** | Interleukin-1 receptor type 1, IL-1R-1, IL-1RT-1, IL-1RT1 (CD121 antigen-like family member A) (Interleukin-1 receptor alpha, IL-1R-alpha) (Interleukin-1 receptor type I) (p80) (CD antigen CD121a) [Cleaved into: Interleukin-1 receptor type 1, membrane form, mIL-1R1, mIL-1RI; Interleukin-1 receptor type 1, soluble form, sIL-1R1, sIL-1RI] | P14778 |
| | **IL-1RAcP** | Interleukin-1 receptor accessory protein, IL-1 receptor accessory protein, IL-1RAcP (Interleukin-1 receptor 3, IL-1R-3, IL-1R3) | Q9NPH3 |
| | **IL6RB** | Interleukin-6 receptor subunit beta, IL-6 receptor subunit beta, IL-6R subunit beta, IL-6R-beta, IL-6RB (CDw130) (Interleukin-6 signal transducer) (Membrane glycoprotein 130, gp130) (Oncostatin-M receptor subunit alpha) (CD antigen CD130) | P40189 |
| | **TGFBRII** | TGF-beta receptor type-2, TGFR-2, EC 2.7.11.30 (TGF-beta type II receptor) (Transforming growth factor-beta receptor type II, TGF-beta receptor type II, TbetaR-II) | P37173 |
| | **CSF1R** | Macrophage colony-stimulating factor 1 receptor (CSF-1 receptor, CSF-1-R, CSF-1R, M-CSF-R, EC 2.7.10.1) (Proto-oncogene c-Fms) (CD antigen CD115) | P07333 |
| | **IL22R** | Interleukin-22 receptor subunit alpha-2, IL-22 receptor subunit alpha-2, IL-22R-alpha-2, IL-22RA2 (Cytokine receptor class-II member 10) (Cytokine receptor family 2 member 10, CRF2-10) (Cytokine receptor family type 2, soluble 1, CRF2-S1) (Interleukin-22-binding protein, IL-22BP, IL22BP) (ZcytoR16) | Q969J5 |
| | **VEGFR1** | Vascular endothelial growth factor receptor 1, VEGFR-1, EC 2.7.10.1 (Fms-like tyrosine kinase 1, FLT-1) (Tyrosine-protein kinase FRT) (Tyrosine-protein kinase receptor FLT, FLT) (Vascular permeability factor receptor) | P17948 |
| | **VEGFR2** | Vascular endothelial growth factor receptor 2, VEGFR-2, EC 2.7.10.1 (Fetal liver kinase 1, FLK-1) (Kinase insert domain receptor, KDR) (Protein-tyrosine kinase receptor flk-1) (CD antigen CD309) | P35968 |
| | **VEGFR3** | Vascular endothelial growth factor receptor 3, VEGFR-3, EC 2.7.10.1 (Fms-like tyrosine kinase 4, FLT-4) (Tyrosine-protein kinase receptor FLT4) | P35916 |
| | **CD40L** | CD40 ligand, CD40-L (T-cell antigen Gp39) (TNF-related activation protein, TRAP) (Tumor necrosis factor ligand superfamily member 5) (CD antigen CD154) | P29965 |
| | **CD96** | T cell surface protein tactile, cell surface antigen CD96, T-cell activated increased late expression protein | P40200 |
| | **CD80** | T-lymphocyte activation antigen CD80 (Activation B7-1 antigen, BB1, CTLA-4 counter-receptor B7.1, B7) | P33681 |
| | **SIRPa** | Tyrosine-protein phosphatase non-receptor type substrate 1 (SHP substrate 1, SHPS-1, Brain Ig-like molecule with tyrosine-based activation motifs, Bit, CD172 antigen-like family member A, Inhibitory receptor SHPS-1, Macrophage fusion receptor, MyD-1 antigen, Signal-regulatory protein alpha-1, Sirp-alpha-1, Signal-regulatory protein alpha-2, Sirp-alpha-2, Signal-regulatory protein alpha-3, Sirp-alpha-3, p84, CD172a) | P78324 |
| | **CD24** | Signal transducer CD24, Small cell lung carcinoma cluster 4 antigen | P25063 |
| | **Siglec-10** | Sialic acid-binding Ig-like lectin 10, Siglec-10 (Siglec-like protein 2) | Q96LC7 |
| | **Fas** | Tumor necrosis factor receptor superfamily member 6 (Apo-1 antigen) (Apoptosis-mediating surface antigen FAS) (FASLG receptor) (CD antigen CD95) | P25445 |
| | **IL15RA_** | Interleukin-15 receptor subunit alpha, IL-15 receptor subunit alpha, IL-15R-alpha, IL-15RA (CD antigen CD215) [Cleaved into: Soluble interleukin-15 receptor subunit alpha, sIL-15 receptor subunit alpha, sIL-15R-alpha, sIL-15RA] | Q13261 |
| | SIRB1_ | Signal-regulatory protein beta-1, SIRP-beta-1 (CD172 antigen-like family member B) (CD antigen CD172b) | 000241 |
| | SIRB2_ | Signal-regulatory protein beta-2, SIRP-beta-2 (Protein tyrosine phosphatase non-receptor type substrate 1-like 3) (Protein tyrosine phosphatase non-receptor type substrate protein) | Q5JXA9 |
| | **LTBR** | Tumor necrosis factor receptor superfamily member 3 (Lymphotoxin-beta receptor) (Tumor necrosis factor C receptor) (Tumor necrosis factor receptor 2-related protein) (Tumor necrosis factor receptor type III, TNF-RIII, TNFR-III) | P36941 |
| | **TGFBR1** | TGF-beta receptor type-1 (TGFR-1) Activin A receptor type ll-like protein kinase, Activin receptor-like kinase 5, ALK-5, Serine/threonine-protein kinase receptor R4, SKR4, Transforming growth factor-beta receptor type I (TbetaR-I) | F8VVC4 |
| | **IL21R** | Interleukin-21 receptor, IL-21 receptor, IL-21R (Novel interleukin receptor) (CD antigen CD360) | Q9HBE5 |
| | **OX40L** | Tumor necrosis factor receptor superfamily member 4 (ACT35 antigen) (OX40L receptor) (TAX transcriptionally-activated glycoprotein 1 receptor) (CD antigen CD134) | P43489 |
| | **FasL** | Tumor necrosis factor ligand superfamily member 6 (Apoptosis antigen ligand, APTL) (CD95 ligand, CD95-L) (Fas antigen ligand, Fas ligand, FasL) (CD antigen CD178) [Cleaved into: Tumor necrosis factor ligand superfamily member 6, membrane form; Tumor necrosis factor ligand superfamily member 6, soluble form (Receptor-binding FasL ectodomain) (Soluble Fas ligand, sFasL) ; ADAM10-processed FasL form, APL; FasL intracellular domain, FasL ICD (SPPL2A-processed FasL form, SPA) ] | P48023 |
| **Type II** | **TRAIL** | Tumor necrosis factor receptor superfamily member 10D (Decoy receptor 2, DcR2) (TNF-related apoptosis-inducing ligand receptor 4, TRAIL receptor 4, TRAIL-R4) (TRAIL receptor with a truncated death domain) (CD antigen CD264) | Q9UBN6 |
| | **TNF** | Tumor necrosis factor (Cachectin) (TNF-alpha) (Tumor necrosis factor ligand superfamily member 2, TNF-a) [Cleaved into: Tumor necrosis factor, membrane form (N-terminal fragment, NTF) ; Intracellular domain 1, ICD1; Intracellular domain 2, ICD2; C-domain 1; C-domain 2; Tumor necrosis factor, soluble form] | P01375 |
| | **LIGHT** | Tumor necrosis factor ligand superfamily member 14(Herpes virus entry mediator ligand, HVEM-L, Herpesvirus entry mediator ligand) (CD antigen CD258) [Cleaved into: Tumor necrosis factor ligand superfamily member 14, membrane form; Tumor necrosis factor ligand superfamily member 14, soluble form] | 043557 |
| | **APRIL** | Tumor necrosis factor ligand superfamily member 13 (A proliferation-inducing ligand, APRIL) (CD antigen CD256) | Q9D777 |
| | **GITRL** | Tumor necrosis factor ligand superfamily member 18 (GITR ligand, GITRL) (Glucocorticoid-induced TNF-related ligand) | Q7TS55 |
| | **CD30** | Tumor necrosis factor ligand superfamily member 8 (CD30 ligand, CD30-L) (CD antigen CD153) | P32971 |
| | **CD70** | CD70 antigen (CD27 ligand, CD27-L) (Tumor necrosis factor ligand superfamily member 7) (CD antigen CD70) | P32970 |
| | **CD40** | Tumor necrosis factor receptor superfamily member 5 (B-cell surface antigen CD40) (Bp50) (CD40L receptor) (CDw40) (CD antigen CD40) | P25942 |
| | **CD27** | CD27 antigen (CD27L receptor) (T-cell activation antigen CD27) (T14) (Tumor necrosis factor receptor superfamily member 7) (CD antigen CD27) | P26842 |
| | **CD30** | Tumor necrosis factor receptor superfamily member 8 (CD30L receptor) (Ki-1 antigen) (Lymphocyte activation antigen CD30) (CD antigen CD30) | P28908 |
| | **RANK** | Tumor necrosis factor receptor superfamily member 11A (Osteoclast differentiation factor receptor, ODFR) (Receptor activator of NF-KB) (CD antigen CD265) | Q9Y6Q6 |
| | **CLEC1** | C-type lectin domain family 1 member A (C-type lectin-like receptor 1, CLEC-1) | Q8NC01 |
| | **CLEC2 /CLE1B** | C-type lectin domain family 1 member B (C-type lectin-like receptor 2, CLEC-2) | Q9P126 |
| | **CLEC3A** | C-type lectin domain family 3 member A (C-type lectin superfamily member 1) (Cartilage-derived C-type lectin) | Q9EPW4 |
| | **CLEC4A** | C-type lectin domain family 4 member A (C-type lectin DDB27) (C-type lectin superfamily member 6) (Dendritic cell immunoreceptor) (Lectin-like immunoreceptor) | Q9UMR7 |
| | **CLEC4E** | C-type lectin domain family 4 member E (C-type lectin superfamily member 9) (Macrophage-inducible C-type lectin, MINCLE) | Q9ULY5 |
| | **CLEC4L** | CD209 antigen (C-type lectin domain family 4 member L) (Dendritic cell-specific ICAM-3-grabbing non-integrin 1, DC-SIGN, DC-SIGN1) (CD antigen CD209) | Q9NNX6 |
| | **CLEC51** | C-type lectin domain family 5 member A (C-type lectin superfamily member 5) (Myeloid DAP12-associating lectin 1, MDL-1) | Q9NY25 |
| | **CLEC6** | C-type lectin domain family 6 member A (C-type lectin superfamily member 10) (Dendritic cell-associated C-type lectin 2, DC-associated C-type lectin 2, Dectin-2) | Q6EIG7 |
| | **CLEC7A** | C-type lectin domain family 7 member A (Beta-glucan receptor) (C-type lectin superfamily member 12) (Dendritic cell-associated C-type lectin 1, DC-associated C-type lectin 1, Dectin-1) | Q9BXN2 |
| | **NKG2D** | NKG2-D type II integral membrane protein (Killer cell lectin-like receptor subfamily K member 1) (NK cell receptor D) (NKG2-D-activating NK receptor) (CD antigen CD314) | P26718 |
| | **BTL-II** | Butyrophilin-like protein 2, BTL-II | Q9UIR0 |
| | **LT-alpha** | TNFB_HUMAN Lymphotoxin-alpha, LT-alpha (TNF-beta) (Tumor necrosis factor ligand superfamily member 1) | P01374 |
| | **LT-beta** | Lymphotoxin-beta (LT-beta) Tumor necrosis factor C | Q06643 |
| | **4-1BBL** | Tumor necrosis factor ligand superfamily member 9 (4-1BB ligand) | P41273 |
| | **IL-1 B** | Interleukin-1 beta, IL-1 beta, Catabolin | P01584 |
| | **IL-1A** | Interleukin-1 alpha, IL-1 alpha, Hematopoietin-1 | P01583 |
| | **IL-4** | Interleukin-4 (B-cell stimulatory factor 1, BSF-1, Binetrakin, Lymphocyte stimulatory factor 1, Pitrakinra) | P05112 |
| | **IL-6** | Interleukin-6, IL-6 (B-cell stimulatory factor 2, BSF-2) (CTL differentiation factor, CDF) (Hybridoma growth factor) (Interferon beta-2, IFN-beta-2) | P05231 |
| | **IL-7** | Interleukin-7 | P13232 |
| | **IL-10** | Interleukin-10, IL-10 (Cytokine synthesis inhibitory factor, CSIF) | P22301 |
| **Cytokin e** | **IL-12 A** | Interleukin-12 subunit alpha, IL-12A (Cytotoxic lymphocyte maturation factor 35 kDa subunit, CLMF p35) (IL-12 subunit p35) (NK cell stimulatory factor chain 1, NKSF1) | P29459 |
| | **IL12 B** | Interleukin-12 subunit beta, IL-12B (Cytotoxic lymphocyte maturation factor 40 kDa subunit, CLMF p40) (IL-12 subunit p40) (NK cell stimulatory factor chain 2, NKSF2) | P29460 |
| | **IL15** | Interleukin-15, IL-15 | P40933 |
| | **IL2** | Interleukin-2, IL-2 (T-cell growth factor, TCGF) | P60568 |
| | **IL18** | Interleukin-18 (Iboctadekin, Interferon gamma-inducing factor, IFN-gamma-inducing factor, Interleukin-1 gamma, IL-1 gamma) | Q14116 |
| | **IL21** | Interleukin-21, IL-21 (Za11) | Q9HBE4 |

In particular, the bifunctional molecule of the invention can be obtained by linking functional variants or functional fragments of immunotherapeutic agents, in particular immune checkpoint blockers or activators, to the humanized anti-hPD-1 antibodies described herein. Preferably, the immunotherapeutic agent corresponds to the extracellular domains (ECD) of an immune checkpoint blocker or activator.

In an embodiment, the invention relates to a humanized anti-PD-1 antibody as defined above, wherein the immunotherapeutic agent comprises or consists of the extracellular domain (ECD) of a protein, selected from the group consisting of CD86, PD-L1, PD-L2, ICOSL, RANK (Tumor necrosis factor receptor superfamily member 11A), VEGF-R1, VEGF-R2, CTLA4, B7-H3, B7-H4, HVEM, CD40, CD111, CD112, CD155, CD113, IL1-R1, IL1-RAcP, LFA-3, CD28, ICOS, BTLA, LAG3, TIGIT, VISTA, CD244, OX40, SIRPalpha, CD80, CD24, Siglec-10, Fas, IL15RA, SIRB1, SIRB2, LTBR, IL21R, CD27, TIM3, TIM4, GITR, LAIR1, CD30, OX40L, TGFRII, DECTIN-1, NKG2D, DC-SIGN, LT-alpha, LT-beta, 4-1BBL, MINCLE, CD70, CD40L, CD153, GITRL, BTL-II, variants and fragments thereof. In particular, the fragments of the immunotherapeutic agents according to the invention have a size inferior or equal to 500, 400, 300, 200, 100 or 50 amino acids.

A reference sequence of the extracellular domain of human OX40L, used in the examples of the present application, corresponds to the sequence associated to SEQ ID NO: 51.

A reference sequence of the extracellular domain of human ICOSL, used in the examples of the present application, corresponds to the sequence associated to SEQ ID NO: 52.

A reference sequence of the extracellular domain of human CD86, used in the examples of the present application, corresponds to the sequence associated to SEQ ID NO: 53.

**Table G. Reference sequences of the ECDs of OX40L, ICOSL and CD86.**

| | |
|---|---|
| **ECD of OX40L** | QVSHRYPRIQSIKVQFTEYKKEKGFILTSQKEDEIMKVQNNSVIINCDGFYLI SLKGYFSQEVNISLHYQKDEEPLFQLKKVRSVNSLMVASLTYKDKVYLNVT TDNTSLDDFHVNGGELILIHQNPGEFCVL |
| SEQ ID NO: 51 | |
| **ECD of ICOSL** | |
| SEQ ID NO: 52 | |
| **ECD of CD86** | APLKIQAYFNETADLPCQFANSQNQSLSELVVFWQDQENLVLNEVYLGKE KFDSVHSKYMGRTSFDSDSWTLRLHNLQIKDKGLYQCIIHHKKPTGMIRIH QMNSELSVLANFSQPEIVPISNITENVYINLTCSSIHGYPEPKKMSVLLRTK NSTIEYDGVMQKSQDNVTELYDVSISLSVSFPDVTSNMTIFCILETDKTRLL SSPFSIELEDPQPPPDH |
| SEQ ID NO: 53 | |
| **ECD of CD80** | |
| SEQ ID NO:54 | |
| **ECD 4-1BBL** | REGPELSPDDPAGLLDLRQGMFAQLVAQNVLLIDGPLSWYSDPGLAGVSL TGGLSYKEDTKELVVAKAGVYYVFFQLELRRVVAGEGSGSVSLALHLQPLR SAAGAAALALTVDLPPASSEARNSAFGFQGRLLHLSAGQRLGVHLHTEAR ARHAWQLTQGATVLGLFRVTPEIPAGLPSPRSE |
| SEQ ID NO:55 | |
| **ECD of IL-7** | DCDIEGKDGKQYESVLMVSIDQLLDSMKEIGSNCLNNEFNFFKRHICDAN KEGMFLFRAARKLRQFLKMNSTGDFDLHLLKVSEGTTILLNCTGQVKGRK PAALGEAQPTKSLEENKSLKEQKKLNDLCFLKRLLQEIKTCWNKILMGTKE H |
| **SEQ ID NO:56** | |

The bifunctional molecule according to the invention comprises antibodies but the disclosure may also comprise macromolecules such as artificial proteins, peptides and any chemical compounds with the capacity to bind antigens mimicking that of antibodies, also termed herein "antigen-binding antibody mimetics", which are not claimed. Such proteins comprise affitins and anticalins. Affitins are artificial proteins with the ability to selectively bind antigens. They are structurally derived from the DNA binding protein Sac7d, found in Sulfolobus acidocaldarius, a microorganism belonging to the archaeal domain. By randomizing the amino acids on the binding surface of Sac7d, e.g. by generating variants corresponding to random substitutions of 11 residues of the binding interface of Sac7d, an affitin library may be generated and subjecting the resulting protein library to rounds of ribosome display, the affinity can be directed towards various targets, such as peptides, proteins, viruses and bacteria. Affitins are antibody mimetics and are being developed as tools in biotechnology. They have also been used as specific inhibitors for various enzymes (Krehenbrink et al., J. mol. Biol., 383:5, 2008). The skilled person may readily develop anticalins with the required binding properties using methods know in the art, in particular as disclosed in patent application WO2008068637 and the above-cited publication, in particular the generation of phage display and/or ribosome display libraries and their screening using an antigen as disclosed herein. Anticalins are artificial proteins that are able to bind to antigens, either to proteins or to small molecules. They are antibody mimetic derived from human lipocalins which are a family of naturally binding proteins. Anticalins are about eight times smaller with a size of about 180 amino acids and a mass of about 20 kDa (Skerra, Febs J., 275:11, 2008). Anticalin phage display libraries have been generated which allow for the screening and selection, in particular of anticalins with specific binding properties. The skilled person may readily develop affitins with the required binding properties using methods know in the art, in particular as disclosed in EP patent EP1270725 B1, US patent US8536307 B2, (Schlehuber and Skerra, Biophys. Chem., 96:2-3, 2002) and the above-cited publication, in particular the generation of phage display and/or ribosome display libraries and their screening using an antigen as dislosed herein. Anticalins and affitins may both be produced in a number of expression system comprising bacterial expression systems. Thus, the disclosure provides affitins, anticalins and other similar antibody mimetics with the features of the humanized antibodies described herein, in particular with regard to the binding to PD-1, the inhibition of the interaction between PD-1 and PD-L1 and/or PD-L2, the non-inhibition of the proliferation of T cells and the increase of the proliferation of T cells all of which are contemplated as macromolecules of the disclosure. All the aspects disclosed herein for antibodies are transposed mutatis mutandis to the macromolecules of the disclosure, in particular to antigen-binding antibody mimetics. The known protein drugs so far include growth factor, hormonal protein, zymoprotein, cytokine, interferon, erythropoietin, molecule and the like. Except for molecules, other protein pharmaceuticals are all homogeneous proteins comprising only one kind of protein component. Although the existing molecule pharmaceuticals (such as Etanercept and ilonacept), produced by the confusion of the extracellular domain of a receptor protein with the Fc fragment of human IgG, are composed of two protein components, they still play only one function of blocking the binding of the endogenous receptor and its corresponding ligand.

### Bifunctional molecule

The invention particularly provides a bifunctional molecule that comprises or consists in a humanized anti-hPD1 antibody and an immunotherapeutic agent, the humanized anti-hPD1 antibody being covalently linked to the immunotherapeutic agent as a fusion protein. Particularly, the bifunctional molecule according to the invention comprises two entities: a first entity comprising or consisting essentially of a humanized anti-hPD1 antibody; a second entity comprising or consisting essentially of an immunotherapeutic agent, these two entities being optionally linked by a peptide linker.

Particularly, the bifunctional molecule according to the invention comprises one, two, three or four molecules of the immunotherapeutic agent. Particularly, the bifunctional molecule may comprise only one molecule of the immunotherapeutic agent, linked to only one light chain or heavy chain of the anti-PD-1 antibody. The bifunctional molecule may also comprise two molecules of the immunotherapeutic agent, linked to either the light or heavy chains of the anti-PD-1 antibody. The bifunctional molecule may also comprise two molecules of the immunotherapeutic agent, a first one linked to the light chain of the anti-PD-1 antibody and a second one linked to the heavy chain of the anti-PD-1 antibody. The bifunctional molecule may also comprise three molecules of the immunotherapeutic agent, two of them being linked to either the light or heavy chain of the anti-PD-1 antibody and the last one linked to the other chain of the anti-PD-1 antibody. Finally, the bifunctional molecule may also comprise four molecules of the immunotherapeutic agent, two molecules linked to the light chain of the anti-PD-1 antibody and two others linked to the heavy chains of the anti-PD-1 antibody. Accordingly, the bifunctional molecule comprises between one to four molecules of an immunotherapeutic agent as disclosed herein.

In one embodiment, only one of the light chains comprises one molecule of immunotherapeutic agent (e.g. the bifunctional molecule comprises one molecule of immunotherapeutic agent), only one of the heavy chains comprises one molecule of immunotherapeutic agent (e.g. the bifunctional molecule comprises one molecule of immunotherapeutic agent), each light chain comprises one molecule of immunotherapeutic agent (e.g. the bifunctional molecule comprises two molecules of immunotherapeutic agent), each heavy chain comprises one molecule of immunotherapeutic agent (e.g. the bifunctional molecule comprises two molecules of immunotherapeutic agent), only one of the light chain and only one heavy chain comprises one molecule of immunotherapeutic agent (e.g. the bifunctional molecule comprises two molecules of immunotherapeutic agent), each light chain comprises one molecule of immunotherapeutic agent and only one of the heavy chains comprises one molecule of immunotherapeutic agent (e.g. the bifunctional molecule comprises three molecule of immunotherapeutic agent), each heavy chain comprises one molecule of immunotherapeutic agent and only one of the light chains comprises one molecule of immunotherapeutic agent (e.g. the bifunctional molecule comprises three molecule of immunotherapeutic agent), or both light chains and heavy chains comprises one molecule of immunotherapeutic agent (e.g. the bifunctional molecule comprises four molecules of immunotherapeutic agent).

In one aspect, the bifunctional molecule according to the disclosure comprises or consists of:
(a) a humanized anti-human PD-1 antibody, which comprises:
   (i) a heavy chain variable domain comprising HCDR1, HCDR2 and HCDR3, and
   (ii) a light chain variable domain comprising LCDR1, LCDR2 and LCDR3,
   wherein:
   - the heavy chain CDR1 (HCDR1) comprises or consists of an amino acid sequence of SEQ ID NO: 1, optionally with one, two or three modification(s) selected from substitution(s), addition(s), deletion(s) and any combination thereof at any position but position 3 of SEQ ID NO: 1;
      - the heavy chain CDR2 (HCDR2) comprises or consists of an amino acid sequence of SEQ ID NO: 2, optionally with one, two or three modification(s) selected from substitution(s), addition(s), deletion(s) and any combination thereof at any position but positions 13, 14 and 16 of SEQ ID NO: 2;
   - the heavy chain CDR3 (HCDR3) comprises or consists of an amino acid sequence of SEQ ID NO: 3 wherein either X1 is D or E and X2 is selected from the group consisting of T, H, A, Y, N, E and S, preferably in the group consisting of H, A, Y, N and E; optionally with one, two or three modification(s) selected from substitution(s), addition(s), deletion(s) and any combination thereof at any position but positions 2, 3, 7 and 8 of SEQ ID NO: 3;
   - the light chain CDR1 (LCDR1) comprises or consists of an amino acid sequence of SEQ ID NO: 12 wherein X is G or T, optionally with one, two or three modification(s) selected from substitution(s), addition(s), deletion(s) and any combination thereof at any position but positions 5, 6, 10, 11 and 16 of SEQ ID NO: 12;
   - the light chain CDR2 (LCDR2) comprises or consists of an amino acid sequence of SEQ ID NO: 15, optionally with one, two or three modification(s) selected from substitution(s), addition(s), deletion(s) and any combination thereof; and
   - the light chain CDR3 (LCDR3) comprises or consists of an amino acid sequence of SEQ ID NO:16, optionally with one, two or three modification(s) selected from substitution(s), addition(s), deletion(s) and any combination thereof at any position but positions 1, 4 and 6 of SEQ ID NO: 16; and
(b) an immunotherapeutic agent, preferably between one to four molecules of an immunotherapeutic agent or a fragment thereof,
wherein an antibody heavy chain or light chain is covalently linked to the immunotherapeutic agent as a fusion protein, preferably by a peptide linker.

More specifically, the immunotherapeutic agent can be any immunotherapeutic agent or an extracellular part thereof as disclosed before in section "Immunotherapeutic agent" and the humanized anti-human PD-1 antibody can be any the humanized anti-human PD-1 antibody as disclosed before in section "anti-PD-1 antibody".

Preferably, the N-terminal end of the immunotherapeutic agent is connected to the C-terminal end of a heavy chain or of a light chain of the humanized anti-human PD-1 antibody, optionally though a peptide linker. Optionally, such bifunctional molecule comprises at least one peptide linker connecting the N-terminus of the immunotherapeutic agent to the C-terminus of a heavy chain or of a light chain of the humanized anti-human PD-1 antibody, the peptide linker being preferably selected from the group consisting of (GGGGS)₃, (GGGGS)₄, (GGGGS)₂, GGGGS, GGGS, GGG, GGS and (GGGS)₃, even more preferably is (GGGGS)₃.

In another embodiment, the invention relates to a bifunctional molecule that comprises a humanized anti-hPD1 antibody, which comprises or consists of:
(i) a heavy chain variable region (VH) comprising or consisting of an amino acid sequence of SEQ ID NO: 17, wherein X1 is D or E and X2 is selected from the group consisting of T, H, A, Y, N, E and S preferably in the group consisting of H, A, Y, N, E; optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 17; and
(ii) a light chain variable region (VL) comprising or consisting of an amino acid sequence of SEQ ID NO: 26, wherein X is G or T, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 26,
wherein the heavy chain and/or light chain is linked, preferably via a peptide linker, at its C-terminal extremity to an immunotherapeutic agent, preferably the extracellular domain (or ECD) of an immunotherapeutic agent, such immunotherapeutic agent being selected from the group consisting of cytokines, cytokines receptors, stimulatory or costimulatory molecules, inhibitory or coinhibitory molecules, preferably an immunotherapeutic agent of human transmembrane immune protein of type I or II, especially of type I.

In another aspect, the disclosure relates to a bifunctional molecule that comprises a humanized anti-hPD1 antibody, which comprises or consists of:
(i) a heavy chain variable region (VH) comprising or consisting of an amino acid sequence of SEQ ID NO: 17, wherein either X1 is D or E and X2 is selected from the group consisting of T, H, A, Y, N, E and S, preferably in the group consisting of H, A, Y, N, E; optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 17;
(ii) a light chain variable region (VL) comprising or consisting of an amino acid sequence of SEQ ID NO: 26, wherein X is G or T, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 26,
(iii) optionally a peptide linker selected from the group consisting of (GGGGS)₃, (GGGGS)₄, (GGGGS)₂, GGGGS, GGGS, GGG, GGS and (GGGS)₃
wherein the heavy chain and/or light chain is linked at its C-terminal extremity, optionally trough the peptide linker, to an immunotherapeutic agent, preferably the extracellular domain (or ECD) of an immunotherapeutic agent, selected from the group consisting of consisting of ICOSL, CD86, B7H4, B7H3, CD28H, PDL2, PDL1, DNAM, CTLA-4, Lag-3, TIGIT, 2B4, BTLA, HVEM, CD101, nectin-1, nectin-2, nectin-3, NELC-5, TLT-2, LFA-3, TIM3, TIM4, LAIR1, SIRPG, IL10R, IL6RA, IL-1R1, IL-1RAcP, IL6RB, TGFBRII, CSF1R, IL22R, VEGFR1, VEGFR2, VEGFR3, CD111, CD112, CD155, CD113, VISTA, CD244, OX40, SIRPalpha, CD80, CD24, Siglec-10, Fas, IL15RA, SIRB1, SIRB2, LTBR, IL21R, GITR, CD40L, OX40L, FasL, TRAIL, TNF, LIGHT, APRIL, GITRL, CD30, CD70, CD40, CD27, CD30, CD153, RANK, CLEC1, CLEC2 /CLE1B, CLEC3A, CLEC4A, CLEC4E, CLEC4L, CLEC51, CLEC6, CLEC7A, NKG2D, BTL-II, TGFRII, DECTIN-1, DC-SIGN, LT-alpha, LT-beta, 4-1BBL, MINCLE, TGFβ, IL-1, IL-2, IL-4, IL-6, IL-7, IL-10, IL-12A, IL12B, IL-15, IL-18 and IL-21.

In a very specific embodiment, the invention relates to a bifunctional molecule that comprises a humanized anti-hPD1 antibody, which comprises or consists of:
(i) a heavy chain variable region (VH) comprising or consisting of an amino acid sequence of SEQ ID NO: 17, wherein either X1 is D or E and X2 is selected from the group consisting of T, H, A, Y, N, E and S, preferably in the group consisting of H, A, Y, N, E; optionally with one, two or three modification(s) selected from substitution(s), addition(s), deletion(s) and any combination thereof at any position but positions 7, 16, 17, 20, 33, 38, 43, 46, 62, 63, 65, 69, 73, 76, 78, 80, 84, 85, 88, 93, 95, 96, 97, 98, 100, 101, 105, 106 and 112 of SEQ ID NO: 17;
(ii) a light chain variable region (VL) comprising or consisting of an amino acid sequence of SEQ ID NO: 26, wherein X is G or T, optionally with one, two or three modification(s) selected from substitution(s), addition(s), deletion(s) and any combination thereof at any position but positions 3, 4, 7, 14, 17, 18, 28, 29, 33, 34, 39, 42, 44, 50, 81, 88, 94, 97, 99 and 105 of SEQ ID NO: 26,
(iii) optionally a peptide linker selected from the group consisting of (GGGGS)₃, (GGGGS)₄, (GGGGS)₂, GGGGS, GGGS, GGG, GGS and (GGGS)₃
wherein the heavy chain and/or light chain is linked at its C-terminal extremity, optionally trough the peptide linker, to an IL-2 or mutant thereof, preferably as disclosed above, more preferably having the amino acid sequence set forth is SEQ ID NO:58.

Binding of the bifunctional molecules to their specific targets can be confirmed by, for example, enzyme-linked immunosorbent assay (ELISA), radioimmunoassay (RIA), FACS analysis, bioassay (e.g., growth inhibition), or Western Blot assay. Each of these assays generally detects the presence of protein-antibody complexes of particular interest by employing a labeled reagent (e.g., an antibody) specific for the complex of interest. For example, the humanized anti-hPD-1 antibody/immunotherapeutic agent complexes can be detected using e.g., an enzyme-linked antibody or antibody fragment which recognizes and specifically binds to the immunotherapeutic agent itself or to the ligand of the immunotherapeutic agent, depending on the nature of the immunotherapeutic agent.

In some examples, the bifunctional molecule described herein suppresses the PD-1 signaling pathway by at least 20%, at least 40%, at least 50%, at least 75%, at least 90%, at least 100%, or by at least 2-fold, at least 5-fold, at least 10-fold, at least 20-fold, at least 50-fold, at least 100-fold, or at least 1000-fold.

Preferably, such bifunctional molecule has the ability to block or inhibit the interaction between PD-1 and its ligand (e.g. PD-L1 and/or PD-L2). In certain embodiments, the bifunctional molecule inhibits the binding interaction between PD-1 and its ligands (e.g. PD-L1 and/or PD-L2) by at least 50%. In certain embodiments, this inhibition may be greater than 60%, greater than 70%, greater than 80%, or greater than 90%.

In some examples, the bifunctional molecule described herein suppresses or decreases the PD-1 signaling pathway by at least 20%, at least 40%, at least 50%, at least 75%, at least 90%, at least 100%, or by at least 2-fold, at least 5-fold, at least 10-fold, at least 20-fold, at least 50-fold, at least 100-fold, or at least 1000-fold.

In some examples, the bifunctional molecule described herein stimulates IFN gamma secretion.

In another example, the bifunctional molecule described herein potentiate the activation of T cells, stimulate the secretion of IFNg by T cells and/or stimulate proliferation of immune cells such as T cells.

### Preparation of bifunctional molecule - Nucleic acid molecules encoding the bifunctional molecule, Recombinant Expression Vectors and Host Cells comprising such

To create a bifunctional molecule of the invention, a humanized anti-hPD1 antibody of the invention is functionally linked to an immunotherapeutic agent. Both entities of the bifunctional molecule are encoded in the same vector and produced as a fusion protein. Accordingly, also disclosed herein are nucleic acids encoding any of the bifunctional molecule described herein, vectors such as expression vectors or recombinant viruses comprising these nucleic acids, and host cells comprising the nucleic acids and/or vectors.

To produce a bifunctional fusion protein which is secreted in stable form by mammalian cells, according to the present invention, nucleic acid sequences coding for the bifunctional molecule are subcloned into an expression vector which is generally used to transfect mammalian cells. General techniques for producing molecules comprising antibody sequences are described in Coligan et al. (eds.), Current protocols in immunology, at pp. 10.19.1-10.19.11 (Wiley Interscience 1992), and in "Antibody engineering: a practical guide" from W. H. Freeman and Company (1992), in which commentary relevant to production of molecules is dispersed throughout the respective texts.

Generally, such method comprises the following steps of:
(1) transfecting or transforming appropriate host cells with the polynucleotide(s) or its variants encoding the recombinant bifunctional molecule of the invention or the vector containing the polynucleotide(s);
(2) culturing the host cells in an appropriate medium; and
(3) optionally isolating or purifying the protein from the medium or host cells.

The invention further relates to a nucleic acid encoding a bifunctional molecule as disclosed above, a vector, preferably an expression vector, comprising the nucleic acid of the invention, a genetically engineered host cell transformed with the vector of the invention or directly with the sequence encoding the recombinant bifunctional molecule, and a method for producing the protein of the invention by recombinant techniques.

The nucleic acid, the vector and the host cells are more particularly described hereafter.

### Nucleic acid sequence

The invention also relates to a nucleic acid molecule encoding the bifunctional molecule as defined above or to a group of nucleic acid molecules encoding the bifunctional molecule as defined above.

Antibody DNA sequences can for example be amplified from RNA of cells that synthesize an immunoglobulin, synthesized using PCR with cloned immunoglobulins, or synthesized via oligonucleotides that encode known signal peptide amino acid sequences. Preferably, the peptide signal comprises or consists of the amino acid sequence of SEQ ID NO: 49 for the VH and/or CH; and/or of the amino acid sequence of SEQ ID NO: 50 for the VL and/or CL. Particularly, the peptide signal is in the N-terminal of the CH, VH, CL and/or VL.

Such nucleic acid may encode an amino acid sequence comprising the VL and/or an amino acid sequence comprising the VH of the antibody (e.g., the light and/or heavy chains of the antibody). Such nucleic acid may be readily isolated and sequenced using conventional procedures.

Particularly, the nucleic acid molecules encoding the bifunctional molecule as defined above comprises:
- a first nucleic acid molecule encoding a variable heavy chain domain of an anti-hPD-1 antibody as disclosed herein, optionally with a peptide signal of SEQ ID NO. 49, and
- a second nucleic acid molecule encoding a variable light chain domain of an anti-hPD-1 antibody as disclosed herein, optionally with a peptide signal of SEQ ID NO. 50, and
- a nucleic acid encoding the immunotherapeutic agent operably linked to either the first nucleic acid or to the second nucleic acid or both, optionally through a nucleic acid encoding a linker.

In one embodiment, the nucleic acid molecules encoding the bifunctional molecule as defined above comprises:
- a first nucleic acid molecule encoding a variable heavy chain domain of SEQ ID NO: 17, wherein X1 is D or E and X2 is selected from the group consisting of T, H, A, Y, N, E and S preferably in the group consisting of H, A, Y, N, and E; optionally with a peptide signal of SEQ ID NO. 49, and
- a second nucleic acid molecule encoding a variable light chain domain of SEQ ID NO: 26, wherein X is G or T; optionally with a peptide signal of SEQ ID NO: 50, and
- a nucleic acid encoding the immunotherapeutic agent operably linked to either the first nucleic acid or to the second nucleic acid or both, optionally through a nucleic acid encoding a peptide linker.

In another embodiment, the nucleic acid molecules encoding the bifunctional molecule as defined above comprises:
- a first nucleic acid molecule encoding a variable heavy chain domain of SEQ ID NO: 17, wherein X1 is D and X2 is selected from the group consisting of T, H, A, Y, N, E, preferably in the group consisting of H, A, Y, N, E, or wherein X1 is E and X2 is selected from the group consisting of T, H, A, Y, N, E and S preferably in the group consisting of H, A, Y, N, E and S; optionally with a peptide signal of SEQ ID NO. 49, and
- a second nucleic acid molecule encoding a variable light chain domain of SEQ ID NO: 26, wherein X is G or T; optionally with a peptide signal of SEQ ID NO. 50, and
- a nucleic acid encoding the immunotherapeutic agent operably linked to either the first nucleic acid or to the second nucleic acid or both, optionally through a nucleic acid encoding a peptide linker.

Preferably, the nucleic acid molecules encoding the bifunctional molecule as defined above comprises:
- a first nucleic acid molecule encoding a variable heavy chain domain of the amino acid sequence set forth in SEQ ID NO: 18, 19, 20, 21, 22, 23, 24 or 25; optionally with a peptide signal of SEQ ID NO. 49, and
- a second nucleic acid molecule encoding a variable light chain domain of the amino acid sequence set forth in SEQ ID NO: 27 or SEQ ID NO: 28; optionally with a peptide signal of SEQ ID NO. 50, and
- a nucleic acid encoding the immunotherapeutic agent operably linked to either the first nucleic acid or to the second nucleic acid or both, optionally through a nucleic acid encoding a peptide linker.

In a very particular embodiment, the nucleic acid molecule encoding a variable heavy chain domain has the sequence set forth in SEQ ID NO: 61 and/or the nucleic acid molecule encoding a variable light chain domain has the sequence set forth in SEQ ID NO: 62.

By operably linked is intended that the nucleic acid encodes a protein fusion including the variable heavy or light chain domain, optionally the linker peptide, and the immunotherapeutic agent. Preferably, the linker is selected from the group consisting of (GGGGS)₃, (GGGGS)₄, (GGGGS)₂, GGGGS, GGGS, GGG, GGS and (GGGS)₃, even more preferably is (GGGGS)₃.

In one embodiment, the nucleic acid molecule is an isolated, particularly non-natural, nucleic acid molecule.

The nucleic acid molecule or group of nucleic acid molecules encoding a bifunctional molecule according to the invention is(are) preferably comprised in a vector or a group of vectors.

### Vectors

In another aspect, the invention relates to a vector comprising the nucleic acid molecule or the group of nucleic acid molecules as defined above.

As used herein, a "vector" is a nucleic acid molecule used as a vehicle to transfer genetic material into a cell. The term "vector" encompasses plasmids, viruses, cosmids and artificial chromosomes. In general, engineered vectors comprise an origin of replication, a multicloning site and a selectable marker. The vector itself is generally a nucleotide sequence, commonly a DNA sequence, that comprises an insert (transgene) and a larger sequence that serves as the "backbone" of the vector. Modern vectors may encompass additional features besides the transgene insert and a backbone: promoter, genetic marker, antibiotic resistance, reporter gene, targeting sequence, protein purification tag. Vectors called expression vectors (expression constructs) specifically are for the expression of the transgene in the target cell, and generally have control sequences.

In one embodiment, both the heavy and light chain coding sequences and/or the constant region of the anti-PD1 antibody are included in one expression vector. Each of the heavy chain coding sequence and the light chain coding sequence may be in operable linkage to a suitable promoter, the heavy chain and/or the light chain being in operable linkage to an immunotherapeutic agent according to the invention. Alternatively, expression of both the heavy chain and the light chain may be driven by the same promoter. In another embodiment, each of the heavy and light chains of the antibody is cloned in to an individual vector, one or both of the heavy and light chains, the heavy chain and/or the light chain being in operable linkage to an immunotherapeutic agent according to the invention. In the latter case, the expression vectors encoding the heavy and light chains can be co-transfected into one host cell for expression of both chains, which can be assembled to form intact antibodies either *in vivo* or *in vitro.* Alternatively, the expression vector encoding the heavy chain and that encoding the light chain can be introduced into different host cells for expression each of the heavy and light chains, which can then be purified and assembled to form intact antibodies *in vitro.*

The nucleic acid molecule encoding the humanized anti-PD-1 antibody can be cloned into a vector by those skilled in the art, and then transformed into host cells. Accordingly, the present invention also provides a recombinant vector, which comprises a nucleic acid molecule encoding the anti-PD-1 antibody of the present invention. In one preferred embodiment, the expression vector further comprises a promoter and a nucleic acid sequence encoding a secretion signal peptide, and optionally at least one drug-resistance gene for screening.

Suitable expression vectors typically contain (1) prokaryotic DNA elements coding for a bacterial replication origin and an antibiotic resistance marker to provide for the growth and selection of the expression vector in a bacterial host; (2) eukaryotic DNA elements that control initiation of transcription, such as a promoter; and (3) DNA elements that control the processing of transcripts, such as a transcription termination/polyadenylation sequence.

The methods known to the artisans in the art can be used to construct an expression vector containing the nucleic acid sequence of the bifunctional molecule described herein and appropriate regulatory components for transcription/translation. These methods include in vitro recombinant DNA techniques, DNA synthesis techniques, *in vivo* recombinant techniques, etc. The DNA sequence is efficiently linked to a proper promoter in the expression vector to direct the synthesis of mRNA. The expression vector may further comprise a ribosome -binding site for initiating the translation, transcription terminator and the like.

An expression vector can be introduced into host cells using a variety of techniques including calcium phosphate transfection, liposome-mediated transfection, electroporation, and the like. Preferably, transfected cells are selected and propagated wherein the expression vector is stably integrated in the host cell genome to produce stable transformants. Techniques for introducing vectors into eukaryotic cells and techniques for selecting stable transformants using a dominant selectable marker are described by Sambrook, by Ausubel, by Bebbington, "Expression of Antibody Genes in Nonlymphoid Mammalian Cells," in 2 METHODS: A companion to methods in enzymology 136 (1991), and by Murray (ed.), Gene transfer and expression protocols (Humana Press 1991). Suitable cloning vectors are described by Sambrook et al. (eds.), MOLECULAR CLONING: A LABORATORY MANUAL, Second Edition (Cold Spring Harbor Press 1989) (hereafter "Sambrook"); by Ausubel et al. (eds.), CURRENT PROTOCOLS IN MOLECULAR BIOLOGY (Wiley Interscience 1987) (hereafter "Ausubel"); and by Brown (ed.), MOLECULAR BIOLOGY LABFAX (Academic Press 1991).

### Host cells

In another aspect, the invention relates to a host cell comprising a vector or a nucleic acid molecule or group of nucleic acid molecules as defined above, for example for bifunctional molecule production purposes.

As used herein, the term "host cell" is intended to include any individual cell or cell culture that can be or has been recipient of vectors, exogenous nucleic acid molecules, and polynucleotides encoding the antibody construct of the present invention; and/or recipients of the antibody construct or bifunctional molecule itself. The introduction of the respective material into the cell can be carried out by way of transformation, transfection and the like. The term "host cell" is also intended to include progeny or potential progeny of a single cell. Suitable host cells include prokaryotic or eukaryotic cells, and also include but are not limited to bacteria, yeast cells, fungi cells, plant cells, and animal cells such as insect cells and mammalian cells, *e.g*., murine, rat, rabbit, macaque or human.

In one embodiment, a host cell comprises (e.g., has been transformed with): (1) a vector comprising a nucleic acid that encodes an amino acid sequence comprising the VL of the antibody and/or an amino acid sequence comprising the VH of the antibody and/or the constant region of the antibody, or (2) a first vector comprising a nucleic acid that encodes an amino acid sequence comprising the VL of the antibody and a second vector comprising a nucleic acid that encodes an amino acid sequence comprising the VH of the antibody.

In another embodiment, a host cell comprises (e.g., has been transformed with) a vector comprising both of the entities of the bifunctional molecule. Preferably, a host cell comprises (e.g., has been transformed with) a vector comprising a first nucleic acid molecule encoding a variable heavy chain domain of an anti-hPD-1 antibody as disclosed herein, and a second nucleic acid molecule encoding a variable light chain domain of an anti-hPD-1 antibody as disclosed herein, operably linked to a third nucleic acid encoding an immunotherapeutic agent as disclosed herein.

A method of humanized anti-PD1 antibody production is also provided herein. The method comprises culturing a host cell comprising a nucleic acid encoding the antibody, as provided above, under conditions suitable for expression of the antibody, and optionally recovering the antibody from the host cell (or host cell culture medium). Particularly, for recombinant production of a humanized anti-PD1 antibody, nucleic acid encoding an antibody, e.g., as described above, is isolated and inserted into one or more vectors for further cloning and/or expression in a host cell.

A bifunctional molecule of the present invention is preferably expressed in eukaryotic cells such as mammalian cells, plant cells, insect cells or yeast cells. Mammalian cells are especially preferred eukaryotic hosts because mammalian cells provide suitable post-translational modifications such as glycosylation. Preferably, such suitable eukaryotic host cell may be fungi such as *Pichia pastoris, Saccharomyces cerevisiae, Schizosaccharomyces pombe;* insect cell such as *Mythimna separate;* plant cell such as tobacco, and mammalian cells such as BHK cells, 293 cells, CHO cells, NSO cells and COS cells. Other examples of useful mammalian host cell lines are CV-1 in Origin with SV40 genes cell (COS cell), monkey kidney CV1 line transformed by SV40 (COS-7); human embryonic kidney line (293 or 293 cells as described, e.g., in Graham, F.L. et al, J. Gen Virol. 36 (1977) 59-74); baby hamster kidney cells (BHK); mouse Sertoli cells (TM4 cells as described, e.g., in Mather, J.P., Biol. Reprod. 23 (1980) 243-252); Human Epithelial Kidney cell (HEK cell); monkey kidney cells (CV1); African green monkey kidney cells (VERO-76); human cervical carcinoma cells (HELA); canine kidney cells (MDCK; buffalo rat liver cells (BRL 3 A); human lung cells (W138); human liver cells (Hep G2); mouse mammary tumor (MMT 060562); TRI cells, as described, e.g., in Mather, J.P. et al, Annals N.Y. Acad. Sci. 383 (1982) 44-68; MRC 5 cells; and FS4 cells. Other useful mammalian host cell lines include Chinese hamster ovary (CHO) cells, including DHFR" CHO cells (Urlaub, G. et al, Proc. Natl. Acad. Sci. USA 77 (1980) 4216-4220); and myeloma cell lines such as Y0, NSO and Sp2/0. For a review of certain mammalian host cell lines suitable for antibody production, see, e.g., Yazaki, P. and Wu, A.M., Methods in Molecular Biology, Vol. 248, Lo, B.K.C. (ed.), Humana Press, Totowa, NJ (2004), pp. 255-268. For example, mammalian cell lines that are adapted to grow in suspension may be useful.

Particularly, the host cell of the present invention is selected from the group consisting of CHO cell, COS cell, NSO cell, and HEK cell.

For a mammalian host, the transcriptional and translational regulatory signals of the expression vector may be derived from viral sources, such as adenovirus, bovine papilloma virus, simian virus, or the like, in which the regulatory signals are associated with a particular gene which has a high level of expression. Suitable transcriptional and translational regulatory sequences also can be obtained from mammalian genes, such as actin, collagen, myosin, and metallothionein genes.

Stable transformants that produce a bifunctional molecule according to the invention can be identified using a variety of methods. After molecule-producing cells have been identified, the host cells are cultured under conditions (e.g. temperature, medium) suitable for their growth and for bifunctional molecule expression. The bifunctional molecules are then isolated and/or purified by any methods known in the art. These methods include, but are not limited to, conventional renaturation treatment, treatment by protein precipitant (such as salt precipitation), centrifugation, cell lysis by osmosis, sonication, supercentrifugation, molecular sieve chromatography or gel chromatography, adsorption chromatography, ion exchange chromatography, HPLC, any other liquid chromatography, and the combination thereof. As described, for example, by Coligan, bifunctional molecule isolation techniques may particularly include affinity chromatography with Protein-A Sepharose, size-exclusion chromatography and ion exchange chromatography. Protein A preferably is used to isolate the bifunctional molecules of the invention.

### Method of selecting a suitable bifunctional molecule

In an aspect, the disclosure relates to a method of selecting a bifunctional molecule of the disclosure, comprising or consisting of at least one of the following steps:
a. testing (e.g. according to a method describing in the Examples X) the ability of the bifunctional molecule to bind to PD-1,
b. testing (e.g. according to a method describing in the Examples X) the ability of the bifunctional molecule to inhibit the binding of human PD-L1 and/or PD-L2 to human PD-1;
c. testing (e.g. according to a method describing in the Example X) the ability of a bifunctional molecule not to inhibit the T cells proliferation, preferably the ability to increase the proliferation of T cells, particularly regulatory T cells;
d. testing (e.g. according to a method describing in the Example X) the ability of a bifunctional molecule not to inhibit the T cells activation, preferably the ability to increase the activation of T cells;
e. testing (e.g. according to a method describing in the Example X) the ability of a bifunctional molecule to increase the secretion of IFNγ by human PBMC;
f. testing (e.g. according to a method describing in the Example X) the ability of a bifunctional molecule to bind to the ligand or receptor of the immunotherapeutic agent;
and optionally comprising the following step:
g. selecting a bifunctional molecule which specifically binds to PD-1, and/or which significantly inhibits the binding of PD-L1 and/or PD-L2 to PD-1, and/or which does not significantly inhibit, preferably increases, the proliferation and/or activation of human T-cells, and/or which increases the secretion of IFNγ by human PBMC, and/or which is able to bind the ligand or receptor of the immunotherapeutic agent.

The method of selecting a modified antibody of the disclosure can advantageously being performed further to the method of manufacturing a bifunctional molecule according to the disclosure as described hereabove.

### Pharmaceutical Composition and Method of Administration Thereof

The present invention also relates to a pharmaceutical composition comprising any of the bifunctional molecule described herein, the nucleic acid molecule, the group of nucleic acid molecules, the vector and/or the host cells as described hereabove, preferably as the active ingredient or compound. The formulations can be sterilized and, if desired, mixed with auxiliary agents such as pharmaceutically acceptable carriers and excipients which do not deleteriously interact with the bifunctional molecule of the invention, nucleic acid, vector and/or host cell of the invention. Optionally, the pharmaceutical composition may further comprise an additional therapeutic agent as detailed below.

Preferably, the pharmaceutical compositions of the present invention may comprise a bifunctional molecule as described herein, the nucleic acid molecule, the group of nucleic acid molecules, the vector and/or the host cells as described hereabove in combination with one or more pharmaceutically or physiologically acceptable carriers, diluents, excipients, salt, and anti-oxidant as described hereafter. Desirably, a pharmaceutically acceptable form is employed which does not adversely affect the desired immune potentiating effects of the bifunctional molecule according to the invention. To facilitate administration, the bifunctional molecule as described herein can be made into a pharmaceutical composition for *in vivo* administration. The means of making such a composition have been described in the art (see, for instance, Remington: The Science and Practice of Pharmacy, Lippincott Williams & Wilkins, 21st edition (2005).

Particularly, the pharmaceutical composition according to the invention can be formulated for any conventional route of administration including a topical, enteral, oral, parenteral, intranasal, intravenous, intramuscular, subcutaneous or intraocular administration and the like. Preferably, the pharmaceutical composition according to the invention is formulated for enteral or parenteral route of administration. Compositions and formulations for parenteral administration may include sterile aqueous solutions that may also contain buffers, diluents and other suitable additives such as, but not limited to, penetration enhancers, carder compounds and other pharmaceutically acceptable carriers or excipients.

The pharmaceutical composition may be prepared by mixing an agent having the desired degree of purity with optional pharmaceutically acceptable carriers, excipients or stabilizers (Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN TM, PLURONICS TM or polyethylene glycol (PEG).

A solid pharmaceutically acceptable vehicle may include one or more substances which may also act as flavoring agents, lubricants, solubilizers, suspending agents, dyes, fillers, glidants, compression aids, inert binders, sweeteners, preservatives, dyes, coatings, or tablet- disintegrating agents. Suitable solid vehicles include, for example calcium phosphate, magnesium stearate, talc, sugars, lactose, dextrin, starch, gelatin, cellulose, polyvinylpyrrolidine, low melting waxes and ion exchange resins. Pharmaceutically acceptable carriers include sterile aqueous solutions or dispersions and sterile powders for the extemporaneous preparation of sterile injectable solutions or dispersion. Except insofar as any conventional media or agent is incompatible with the active compound, use thereof in the pharmaceutical compositions of the invention is contemplated.

The bifunctional molecule according to the invention may be dissolved or suspended in a pharmaceutically acceptable liquid vehicle such as water, an organic solvent, ethanol, polyol (for example, glycerol, propylene glycol, and liquid polyethylene glycol, and the like a mixture of both or pharmaceutically acceptable oils or fats and suitable mixtures thereof. The liquid vehicle can contain other suitable pharmaceutical additives such as solubilizers, emulsifiers, buffers, preservatives, sweeteners, flavoring agents, suspending agents, wetting agents, thickening agents, colors, viscosity regulators, stabilizers or osmo-regulators. Suitable examples of liquid vehicles for oral and enteral administration include water (partially containing additives as above, e.g. cellulose derivatives, preferably sodium carboxymethyl cellulose solution), alcohols (including monohydric alcohols and polyhydric alcohols, e.g. glycols) and their derivatives, and oils (e.g. fractionated coconut oil and peanut oil). For parenteral administration, the vehicle can also be an oily ester such as ethyl oleate and isopropyl myristate. Sterile liquid vehicles are useful in sterile liquid form compositions for enteral administration. The liquid vehicle for pressurized compositions can be a halogenated hydrocarbon or other pharmaceutically acceptable propellant.

The pharmaceutical composition of the invention may further comprise one or more pharmaceutically acceptable salts. A "pharmaceutically acceptable salt" refers to a salt that retains the desired biological activity of the parent compound and does not impart any undesired toxicological effects. Examples of such salts include acid addition salts and base addition salts. Acid addition salts include those derived from nontoxic inorganic acids, such as hydrochloric, nitric, phosphoric, sulfuric, hydrobromic, hydroiodic, phosphorous and the like, as well as from nontoxic organic acids such as aliphatic mono- and dicarboxylic acids, phenyl-substituted alkanoic acids, hydroxy alkanoic acids, aromatic acids, aliphatic and aromatic sulfonic acids and the like. Base addition salts include those derived from alkaline metals or alkaline earth metals, such as sodium, potassium, magnesium, calcium and the like, as well as from nontoxic organic amines, such as N,N'-dibenzylethylenediamine, N-methylglucamine, chloroprocaine, choline, diethanolamine, ethylenediamine, procaine and the like.

A pharmaceutical composition of the invention also may include a pharmaceutically acceptable anti-oxidant. Examples of pharmaceutically acceptable antioxidants include: water soluble antioxidants, such as ascorbic acid, cysteine hydrochloride, sodium bisulfate, sodium metabisulfite, sodium sulfite and the like; oil-soluble antioxidants, such as ascorbyl palmitate, butylated hydroxyanisole (BHA), butylated hydroxytoluene (BHT), lecithin, propyl gallate, alpha-tocopherol, and the like; and metal chelating agents, such as citric acid, ethylenediamine tetra-acetic acid (EDTA), sorbitol, tartaric acid, phosphoric acid, and the like.

To facilitate delivery, any of the bifunctional molecule or its encoding nucleic acids can be conjugated with a chaperon agent. The chaperon agent can be a naturally occurring substance, such as a protein (e.g., human serum albumin, low-density lipoprotein, or globulin), carbohydrate (e.g., a dextran, pullulan, chitin, chitosan, inulin, cyclodextrin or hyaluronic acid), or lipid. It can also be a recombinant or synthetic molecule, such as a synthetic polymer, e.g., a synthetic polyamino acid. Examples of polyamino acids include polylysine (PLL), poly L aspartic acid, poly L-glutamic acid, styrene-maleic acid anhydride copolymer, poly(L-lactide-co-glycolied) copolymer, divinyl ether-maleic anhydride copolymer, N-(2-hydroxypropyl) methacrylamide copolymer (HMPA), polyethylene glycol (PEG), polyvinyl alcohol (PVA), polyurethane, poly(2-ethylacryllic acid), N-isopropylacrylamide polymers, and polyphosphazine. In one example, the chaperon agent is a micelle, liposome, nanoparticle, or microsphere. Methods for preparing such a micelle, liposome, nanoparticle, or microsphere are well known in the art. See, e.g., US Patents 5,108,921; 5,354,844; 5,416,016; and 5,527,5285.

Pharmaceutical composition typically must be sterile and stable under the conditions of manufacture and storage. The pharmaceutical composition can be formulated as a solution, micro-emulsion, liposome, or other ordered structure suitable to high drug concentration and/or in suitable for injection. The proper fluidity can be maintained, for example, by the use of a coating such as lecithin, by the maintenance of the required particle size in the case of dispersion and by the use of surfactants. In one embodiment, the pharmaceutical composition is an injectable composition that may contain various carriers such as vegetable oils, dimethylactamide, dimethyformamide, ethyl lactate, ethyl carbonate, isopropyl myristate, ethanol, and polyols (glycerol, propylene glycol, liquid polyethylene glycol, and the like). For intravenous injection, water soluble antibodies can be administered by the drip method, whereby a pharmaceutical formulation containing the antibody and physiologically acceptable excipients is infused. Physiologically acceptable excipients may include, for example, 5% dextrose, 0.9% saline, Ringer's solution or other suitable excipients. Intramuscular preparations, e.g., a sterile formulation of a suitable soluble salt form of the antibody, can be dissolved and administered in a pharmaceutical excipient such as Water-for-Injection, 0.9% saline, or 5% glucose solution.

Sterile injectable solutions can be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof. Prolonged absorption of the injectable compositions can be brought about by including in the composition an agent that delays absorption, for example, monostearate salts and gelatin.

Prevention of presence of microorganisms may be ensured both by sterilization procedures, and by the inclusion of various antibacterial and antifungal agents, for example, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

It will be understood by one skilled in the art that the formulations of the invention may be isotonic with human blood that is the formulations of the invention have essentially the same osmotic pressure as human blood. Such isotonic formulations generally have an osmotic pressure from about 250 mOSm to about 350 mOSm. Isotonicity can be measured by, for example, a vapor pressure or ice-freezing type osmometer. Tonicity of a formulation is adjusted by the use of tonicity modifiers. "Tonicity modifiers" are those pharmaceutically acceptable inert substances that can be added to the formulation to provide an isotonicity of the formulation. Tonicity modifiers suitable for this invention include, but are not limited to, saccharides, salts and amino acids.

Pharmaceutical compositions according to the invention may be formulated to release the active ingredients (e.g. the bifunctional molecule of the invention) substantially immediately upon administration or at any predetermined time or time period after administration. The pharmaceutical composition in some aspects can employ time-released, delayed release, and sustained release delivery systems such that the delivery of the composition occurs prior to, and with sufficient time to cause, sensitization of the site to be treated. Means known in the art can be used to prevent or minimize release and absorption of the composition until it reaches the target tissue or organ, or to ensure timed-release of the composition. Such systems can avoid repeated administrations of the composition, thereby increasing convenience to the subject and the physician.

The amount of active ingredient which can be combined with a carrier material to produce a single dosage form will vary depending upon the subject being treated, and the particular mode of administration. The amount of active ingredient which can be combined with a carrier material to produce a single dosage form will generally be that amount of the composition which produces a therapeutic effect.

### Subject, regimen and administration

The present invention relates to a bifunctional molecule as disclosed herein; a nucleic acid or a vector encoding such, a host cell or a pharmaceutical composition, a nucleic acid, a vector or a host cell, for use as a medicament or for use in the treatment of a disease or for administration in a subject or for use as a medicament. Examples of treatments are more particularly described hereafter under the section "Methods and Uses".

The subject to treat may be a human, particularly a human at the prenatal stage, a new-born, a child, an infant, an adolescent or an adult, in particular an adult of at least 30 years old, 40 years old, preferably an adult of at least 50 years old, still more preferably an adult of at least 60 years old, even more preferably an adult of at least 70 years old.

Particularly, the subject is affected with a disease that may involve the PD-1/PDL-1 pathway, particularly wherein, at least one of the ligands of PD-1 (e.g. PDL-1 and/or PDL-2) or PD-1 is/are expressed, especially overexpressed. Preferably, the subject is suffering from cancer, even more preferably from a PD1, PD-L1 and/or PD-L2 positive cancer or a PD-1 positive cancer. Examples of diseases and cancers are more particularly described hereafter under the section "Methods and Uses". In a particular embodiment, the subject has already received at least one line of treatment, preferably several lines of treatment, prior to the administration of the bifunctional molecule according to the invention or of a pharmaceutical composition according to the invention.

Conventional methods, known to those of ordinary skill in the art of medicine, can be used to administer bifunctional molecule or the pharmaceutical composition disclosed herein to the subject, depending upon the type of diseases to be treated or the site of the disease. This composition can be administered via conventional routes, e.g., administered orally, parenterally, enterally, by inhalation spray, topically, rectally, nasally, buccally, vaginally or via an implanted reservoir. The term "parenterally" as used herein includes subcutaneous, intra-cutaneous, intravenous, intramuscular, intra-articular, intra-arterial, intra-synovial, intra-tumoral, intra-sternal, intra-thecal, intra-lesion, and intracranial injection or infusion techniques. When administered parenterally, the pharmaceutical composition according to the disclosure is preferably administered by intravenous route of administration. When administered enterally, the pharmaceutical composition according to the disclosure is preferably administered by oral route of administration. This composition can also be administered locally.

The form of the pharmaceutical compositions, the route of administration and the dose of administration of the pharmaceutical composition or the bifunctional molecule according to the disclosure can be adjusted by the man skilled in the art according to the type and severity of the infection, and to the patient, in particular its age, weight, sex, and general physical condition. The compositions of the present disclosure may be administered in a number of ways depending upon whether local or systemic treatment is desired.

Preferably, the treatment with the bifunctional molecule or with a pharmaceutical composition according to the disclosure is administered regularly, preferably between every day, every week or every month, more preferably between every day and every one, two, three or four weeks. In a particular aspect, the treatment is administered several times a day, preferably 2 or 3 times a day.

The duration of treatment with the bifunctional molecule or with a pharmaceutical composition according to the disclosure according to the disclosure is preferably comprised between 1 day and 20 weeks, more preferably between 1 day and 10 weeks, still more preferably between 1 day and 4 weeks, even more preferably between 1 day and 2 weeks. Alternatively, the treatment may last as long as the disease persists.

The bifunctional molecule disclosed herein may be provided at an effective dose range from about 1 ng/kg body weight to about 30 mg/kg body weight, 1 µg/kg to about 20 mg/kg, 10 µg/kg to about 10 mg/kg, or from 100 µg/kg to 5 mg/kg, optionally every one, two, three or four weeks, preferably by parenteral or oral administration, in particular by intravenous or subcutaneous administration.

Particularly, the bifunctional molecule according to the disclosure can be administered at a subtherapeutic dose. The term "subtherapeutic dose" as used herein refers to a dose that is below the effective monotherapy dosage levels commonly used to treat a disease, or a dose that currently is not typically used for effective monotherapy with anti-hPD1 antibodies.

### Methods and Uses

### Use in the treatment of a disease

The bifunctional molecules, nucleic acids, vectors, host cells, compositions and methods of the present invention have numerous *in vitro* and *in vivo* utilities and applications. For example, the bifunctional molecule, the nucleic acids, the vectors, the host cells and/or the pharmaceutical compositions described herein can be used as therapeutic agents, diagnostic agents and medical researches. Particularly, any of the bifunctional molecule, nucleic acid, group of nucleic acid, vector, host cells or pharmaceutical composition provided herein may be used in therapeutic methods and/or for therapeutic purposes. Particularly, the bifunctional molecule, nucleic acid, vector or pharmaceutical composition provided herein may be useful for the treatment of any disease or condition, preferably involving PD-1, such as cancer, autoimmune disease, and infection or other diseases associated with immune deficiency, such as T cell dysfunction.

Preferably, the invention relates to a method of treatment of a pathology, disease and/or disorder that could be prevented or treated by the inhibition of the binding of PD-L1 and/or PD-L2 to PD-1.

Even more preferably, the invention relates to a method of treatment of a disease and/or disorder selected from the group consisting of a cancer and an infectious disease, preferably a chronic infection, in a subject in need thereof comprising administering to said subject an effective amount of the bifunctional molecule or pharmaceutical composition as defined above. Examples of such diseases are more particularly described hereafter.

Particularly, the bifunctional molecule according to the invention are called "bifunctional checkpoint inhibitors" as they target both PD-1/PD-L1/PD-L2 and another signaling pathways.

The invention particularly concerns a bifunctional molecule, a nucleic acid, a group of nucleic acids or a vector encoding such, or a pharmaceutical composition comprising such for use in the treatment of a pathology, disease and/or disorder that could be prevented or treated by the inhibition of the binding of PD-L1 and/or PD-L2 to PD-1.

Accordingly, disclosed herein are methods for treating a disease, in particular associated with the PD-1 and/or PD-1/PD-L1 and/or PD-1/PD-L2 signaling pathway, comprising administering to a subject in need of a treatment an effective amount of any of the bifunctional molecule or pharmaceutical composition described herein. Physiological data of the patient (e.g. age, size, and weight) and the routes of administration have also to be taken into account to determine the appropriate dosage, so as a therapeutically effective amount will be administered to the patient.

In another aspect the bifunctional molecules disclosed herein can be administered to a subject, e.g., *in vivo,* to enhance immunity, preferably in order to treat a disorder and/or disease. Accordingly, in one aspect, the invention provides a method of modifying an immune response in a subject comprising administering to the subject a bifunctional molecule, nucleic acid, vector or pharmaceutical composition of the invention such that the immune response in the subject is modified. Preferably, the immune response is enhanced, increased, stimulated or up-regulated. The bifunctional molecule or pharmaceutical composition can be used to enhance immune responses such as T cell activation in a subject in need of a treatment. The immune response enhancement can result in the inhibition of the binding of PD-L1 and/or PD-L2 to PD-1 thereby reducing the immunosuppressive environment, stimulating the proliferation and/or the activation of human T-cells and/or the IFNγ secretion by human PBMC.

The invention particularly provides a method of enhancing an immune response in a subject, comprising administering to the subject a therapeutic effective amount of any of the bifunctional molecule, nucleic acid, vector or pharmaceutical composition comprising such described herein, such that an immune response in the subject is enhanced.

In some embodiments, the amount of the bifunctional molecule described herein is effective in suppressing the PD-1 signaling (e.g., reducing the PD-1 signaling by at least 20%, 30%, 50%, 80%, 100%, 200%, 400%, or 500% as compared to a control). In other embodiments, the amount of the anti-PD-1 antibody described herein is effective in activating immune responses (e.g., by at least 20%, 30%, 50%, 80%, 100%, 200%, 400%, or 500% as compared to a control).

In some embodiments, the amount of the humanized anti-hPD-1 antibody described herein is effective in the inhibition of the binding of human PD-L1 and/or PD-L2 to human PD-1 e.g., inhibiting the binding by at least 20%, 30%, 50%, 80%, 100%, 200%, 400%, or 500% as compared to a control).

In some embodiments, the amount of the humanized anti-hPD-1 antibody described herein is sufficient to have an antagonist activity of the binding of human PD-L1 and/or PD-L2 to human PD-1 e.g., inhibiting the binding by at least 20%, 30%, 50%, 80%, 100%, 200%, 400%, or 500% as compared to a control).

The present invention also relates to a bifunctional molecule as described herein; a nucleic acid or a vector encoding such, or a pharmaceutical composition comprising such for use in the treatment of a disorder and/or disease in a subject and/or for use as a medicament or vaccine.

A subject in need of a treatment may be a human having, at risk for, or suspected of having a disease associated with the signaling pathway mediated by PD-1. Such a patient can be identified by routine medical examination. For example, a subject suitable for the treatment can be identified by examining whether such subject carries PD-1, PD-L1 and/or PD-L2 positive cells. In one embodiment, a subject who needs a treatment is a patient having, suspected of having, or at risk for a disease, preferably a PD-1, PDL1 and/or PDL2 positive disease, even more preferably a disease where PD-1 and/or at least one ligand of PD-1 is overexpressed. In such subject, the disruption of PD-1/PD-L1 and/or PD-1/PD-L2 interaction thanks to the administration of the bifunctional molecule or pharmaceutical composition according to the invention may enhance immune response of the subject. In some embodiments, any of the humanized anti-PD-1 antibodies or pharmaceutical composition described herein can be used for treating PD-1 positive cells.

### Cancer

It is known in the art that blockade of PD-1 by antibodies can enhance the immune response to cancerous cells in a patient. Thus, in one aspect, the invention provides a bifunctional molecule or a pharmaceutical composition for use in the treatment of a subject having a cancer, comprising administering to the individual an effective amount of the bifunctional molecule or pharmaceutical composition, preferably to disrupt or inhibit the PD1/PD-L1 and/or PD1/PD-L2 interaction.

In one embodiment, a subject who needs a treatment is a patient having, suspected of having, or at risk for a disease, preferably a PD-1 or PD-L1 positive cancer, even more preferably a cancer where PD-1 or PD-L1 is expressed or overexpressed. For example, a patient suitable for the treatment can be identified by examining whether such a patient carries PD-L1 positive tumor cells. Additionally or alternatively, the subject suitable for the treatment is a subject having tumor infiltrating T cells that express or overexpress PD-1.

In another embodiment, a subject is a patient having, suspected of having, or at risk for a cancer development, preferably a PD-L1 and/or PD-L2 positive cancer. In some embodiments, any of bifunctional molecule or pharmaceutical composition described herein can be used for treating PD-L1 and/or PD-L2 positive tumors. For example, a human patient suitable for the treatment can be identified by examining whether such a patient carries PD-L1 and/or PD-L2 positive cancer cells.

In further aspects, a bifunctional molecule or pharmaceutical composition for use in treating cancer, preferably a PD-1, PD-L1 and/or PD-L2 positive cancer, even more preferably a cancer wherein PD-1, PD-L1 and/or PD-L2 is/are overexpressed is provided.

In a particular aspect, the bifunctional molecule or pharmaceutical composition according to the present invention is for use in the treatment of cancer by activating exhausted T cells.

In an aspect of the disclosure, the cancer to be treated is associated with exhausted T cells.

Preferably, by "PD-L1 positive tumor cells" or "PD-L2 positive tumor cells" is intended to refer to a population of tumor cells in which PD-L1 or PD-L2, respectively, are expressed in at least 10% of tumor cells, preferable at least 20, 30, 40 or 50 % of tumor cells.

Any suitable cancer may be treated with the bifunctional molecule provided herein can be hematopoietic cancer or solid cancer. Such cancers include carcinoma, cervical cancer, colorectal cancer, esophageal cancer, gastric cancer, gastrointestinal cancer, head and neck cancer, kidney cancer, liver cancer, lung cancer, lymphoma, glioma, mesothelioma, melanoma, stomach cancer, urethral cancer environmentally induced cancers and any combinations of said cancers. The present invention is also useful for treatment of metastatic cancers, especially metastatic cancers that express PD-L1 (Iwai et al. (2005) Int. Immunol. 17: 133-144). Additionally, the invention includes refractory or recurrent malignancies.

In a particular aspect, the cancer is a hematologic malignancy or a solid tumor with high expression of PD-1 and/or PD-L1. Such a cancer can be selected from the group consisting of hematolymphoid neoplasms, angioimmunoblastic T cell lymphoma, myelodysplastic syndrome, acute myeloid leukemia. In a particular aspect, the cancer is a cancer induced by virus or associated with immunodeficiency. Such a cancer can be selected from the group consisting of Kaposi sarcoma (e.g., associated with Kaposi sarcoma herpes virus); cervical, anal, penile and vulvar squamous cell cancer and oropharyngeal cancers (e.g., associated with human papilloma virus); B cell non-Hodgkin lymphomas (NHL) including diffuse large B-cell lymphoma, Burkitt lymphoma, plasmablastic lymphoma, primary central nervous system lymphoma, HHV-8 primary effusion lymphoma, classic Hodgkin lymphoma, and lymphoproliferative disorders (e.g., associated with Epstein-Barr virus (EBV) and/or Kaposi sarcoma herpes virus); hepatocellular carcinoma (e.g., associated with hepatitis B and/or C viruses); Merkel cell carcinoma (e.g., associated with Merkel cell polyoma virus (MPV)); and cancer associated with human immunodeficiency virus infection (HIV) infection.

Preferably, the cancer to be treated or prevented is selected from the group consisting of metastatic or not metastatic, Melanoma , malignant mesothelioma, Non-Small Cell Lung Cancer, Renal Cell Carcinoma, Hodgkin's Lymphoma, Head and Neck Cancer, Urothelial Carcinoma, Colorectal Cancer, Hepatocellular Carcinoma, Small Cell Lung Cancer Metastatic Merkel Cell Carcinoma, Gastric or Gastroesophageal cancers and Cervical Cancer.

Preferred cancers for treatment include cancers typically responsive to immunotherapy. Alternatively, preferred cancers for treatment are cancers non-responsive to immunotherapy.

By way of example and not wishing to be bound by theory, treatment with an anti-cancer antibody or an anti-cancer immunoconjugate or other current anti-cancer therapy that lead to cancer cell death would potentiate an immune response mediated by PD- 1. Accordingly, a treatment of a hyper proliferative disease (e.g., a cancer tumor) may include a bifunctional molecule as disclosed herein combined with an anti-cancer treatment, concurrently or sequentially or any combination thereof, which may potentiate an anti-tumor immune response by the host. Preferably, the bifunctional molecule may be used in combination with other immunogenic agents, standard cancer treatments, or other antibodies as described hereafter.

### Infectious disease

The bifunctional molecule, nucleic acid, group of nucleic acid, vector, host cells or pharmaceutical composition of the invention may be used to treat patients that have been exposed to particular toxins or pathogens.

Any suitable infection may be treated with the bifunctional molecule, nucleic acid, group of nucleic acid, vector, host cells or pharmaceutical composition provided herein.

Some examples of pathogenic viruses causing infections treatable by methods of the invention include HIV, hepatitis (A, B, or C), herpes virus (e.g., VZV, HSV-1, HAV-6, HSV-II, and CMV, Epstein Barr virus), adenovirus, influenza virus, flaviviruses, echovirus, rhinovirus, coxsackie virus, coronavirus, respiratory syncytial virus, mumps virus, rotavirus, measles virus, rubella virus, parvovirus, vaccinia virus, HTLV virus, dengue virus, papillomavirus, molluscum virus, poliovirus, rabies virus, JC virus and arboviral encephalitis virus.

Particularly, the bifunctional molecule or pharmaceutical compositions of the invention are used to treat patients that have chronic viral infection, such infection being caused by viruses selected from the group consisting of Retroviruses, Anellovirus, Circovirus, Herpesvirus, Varicella zoster virus (VZV), Cytomegalovirus (CMV), Epstein-Barr virus (EBV), Polyomavirus BK, Polyomavirus, Adeno-associated virus (AAV), Herpes simplex type 1 (HSV-1), Adenovirus, Herpes simplex type 2 (HSV-2), Kaposi's sarcoma herpesvirus (KSHV), Hepatitis B virus (HBV), GB virus C, Papilloma virus, Hepatitis C virus (HCV), Human immunodeficiency virus (HIV), Hepatitis D virus (HDV), Human T cell leukemia virus type 1 (HTLV1), Xenotropic murine leukemia virus-related virus (XMLV), Rubella virus, German measles, Parvovirus B19, Measles virus, Coxsackie virus.

Some examples of pathogenic bacteria causing infections treatable by methods of the invention include chlamydia, rickettsial bacteria, mycobacteria, staphylococci, streptococci, pneumonococci, meningococci and conococci, klebsiella, proteus, serratia, pseudomonas, legionella, diphtheria, salmonella, bacilli, cholera, tetanus, botulism, anthrax, plague, leptospirosis, and Lymes disease bacteria.

Some examples of pathogenic fungi causing infections treatable by methods of the invention include Candida (albicans, krusei, glabrata, tropicalis, etc.), Cryptococcus neoformans, Aspergillus (fumigatus, niger, etc.), Genus Mucorales (mucor, absidia, rhizophus), Sporothrix schenkii, Blastomyces dermatitidis, Paracoccidioides brasiliensis, Coccidioides immitis and Histoplasma capsulatum.

Some examples of pathogenic parasites causing infections treatable by methods of the invention include Entamoeba histolytica, Balantidium coli, Naegleriafowleri, Acanthamoeba sp., Giardia lambia, Cryptosporidium sp., Pneumocystis carinii, Plasmodium vivax, Babesia microti, Trypanosoma brucei, Trypanosoma cruzi, Leishmania donovani, Toxoplasma gondi, and Nippostrongylus brasiliensis.

In all of the above methods, the bifunctional molecule can be combined with other forms of immunotherapy such as cytokine treatment (e.g., interferons, GM-CSF, G-CSF, IL-2), or any therapy, which provides for enhanced presentation of tumor antigens.

### Combined therapy

In particular, bifunctional molecule of the present invention can be combined with some other potential strategies for overcoming immune evasion mechanisms with agents in clinical development or already on the market (Antonia et al. Immuno-oncology combinations: a review of clinical experience and future prospects. Clin. Cancer Res. Off. J. Am. Assoc. Cancer Res. 20, 6258-6268, 2014). Such combination with the bifunctional molecule according to the invention may be useful notably for:
1- Reversing the inhibition of adaptive immunity (blocking T-cell checkpoint pathways);
2- Switching on adaptive immunity (promoting T-cell costimulatory receptor signaling using agonist molecules, in particular antibodies),
3- Improving the function of innate immune cells;
4- Activating the immune system (potentiating immune-cell effector function), for example through vaccine-based strategies.

Accordingly, also provided herein are combined therapies for any of the diseases associated with the PD-1 signaling as described herein with any of the bifunctional molecule or pharmaceutical composition comprising such, as described herein and a suitable second agent. In an aspect, the bifunctional molecule and the second agent can be present in a pharmaceutical composition as described above. Alternatively, the terms "combination therapy" or "combined therapy", as used herein, embrace administration of these two agents (e.g., a bifunctional molecule as described herein and an additional or second suitable therapeutic agent) in a sequential manner, that is, wherein each therapeutic agent is administered at a different time, as well as administration of these therapeutic agents, or at least two of the agents, in a substantially simultaneous manner. Sequential or substantially simultaneous administration of each agent can be affected by any appropriate route. The agents can be administered by the same route or by different routes. For example, a first agent (e.g., a bifunctional molecule) can be administered orally, and an additional therapeutic agent (e.g., an anti-cancer agent, an anti-infection agent; or an immune modulator) can be administered intravenously. Alternatively, an agent of the combination selected may be administered by intravenous injection while the other agents of the combination may be administered orally.

In another aspect, the invention relates to a therapeutic mean, in particular a combination product mean, which comprises as active ingredients: a bifunctional molecule as defined above and an additional therapeutic agent, wherein said active ingredients are formulated for separate, sequential or combined therapy, in particular for combined or sequential use.

As used herein, the term "sequential" means, unless otherwise specified, characterized by a regular sequence or order, e.g., if a dosage regimen includes the administration of a bifunctional molecule and the second agent, a sequential dosage regimen could include administration of the bifunctional molecule of the invention before, simultaneously, substantially simultaneously, or after administration of the second agent, but both agents will be administered in a regular sequence or order. The term "separate" means, unless otherwise specified, to keep apart one from the other. The term "simultaneously" means, unless otherwise specified, happening or done at the same time, i.e., the agents of the invention are administered at the same time. The term "substantially simultaneously" means that the agents are administered within minutes of each other (e.g., within 15 minutes of each other) and intends to embrace joint administration as well as consecutive administration, but if the administration is consecutive it is separated in time for only a short period (e.g., the time it would take a medical practitioner to administer two compounds separately).

It should be appreciated that any combination as described herein may be used in any sequence for treating the disorder or disease described herein. The combinations described herein may be selected on the basis of a number of factors, which include but are not limited to the effectiveness of inhibiting or preventing the target disease progression, the effectiveness for mitigating the side effects of another agent of the combination, or the effectiveness of mitigating symptoms related to the target disease. For example, a combined therapy described herein may reduce any of the side effects associated with each individual members of the combination.

When the bifunctional molecule or the pharmaceutical composition described here is co-used with an additional therapeutic agent, a sub-therapeutic dosage of either the bifunctional molecule, the pharmaceutical composition or of the additional or second agent, or a sub-therapeutic dosage of both, can be used in the treatment of a subject, preferably a subject having, or at risk of developing a disease or disorder associated with the cell signaling mediated by PD-1.

In an aspect, the additional or second therapeutic agent can be selected in the non-exhaustive list comprising alkylating agents, angiogenesis inhibitors, antibodies, antimetabolites, antimitotics, antiproliferatives, antivirals, aurora kinase inhibitors, apoptosis promoters (for example, Bcl-2 family inhibitors), activators of death receptor pathway, Bcr-Abl kinase inhibitors, BiTE (Bi-Specific T cell Engager) antibodies, antibody drug conjugates, biologic response modifiers, Bruton's tyrosine kinase (BTK) inhibitors, cyclin-dependent kinase inhibitors, cell cycle inhibitors, cyclooxygenase-2 inhibitors, DVDs, leukemia viral oncogene homolog (ErbB2) receptor inhibitors, growth factor inhibitors, heat shock protein (HSP)-90 inhibitors, histone deacetylase (HDAC) inhibitors, hormonal therapies, immunologicals, inhibitors of inhibitors of apoptosis proteins (IAPs), intercalating antibiotics, kinase inhibitors, kinesin inhibitors, Jak2 inhibitors, mammalian target of rapamycin inhibitors, microRNAs, mitogen-activated extracellular signal-regulated kinase inhibitors, multivalent binding proteins, non-steroidal anti-inflammatory drugs (NSAIDs), poly ADP (adenosine diphosphate)-ribose polymerase (PARP) inhibitors, platinum chemotherapeutics, polo-like kinase (Plk) inhibitors, phosphoinositide-3 kinase (PI3K) inhibitors, proteasome inhibitors, purine analogs, pyrimidine analogs, receptor tyrosine kinase inhibitors, retinoids/deltoids plant alkaloids, small inhibitory ribonucleic acids (siRNAs), topoisomerase inhibitors, ubiquitin ligase inhibitors, hypomethylating agents, checkpoints inhibitors, peptide vaccine and the like, epitopes or neoepitopes from tumor antigens, as well as combinations of one or more of these agents.

For instance, the additional therapeutic agent can be selected in the group consisting of chemotherapy, radiotherapy, targeted therapy, antiangiogenic agents, hypomethylating agents, cancer vaccines, epitopes or neoepitopes from tumor antigens, myeloid checkpoints inhibitors, other immunotherapies, and HDAC inhibitors.

In a preferred embodiment, the second therapeutic agent is selected from the group consisting of chemotherapeutic agents, radiotherapy agents, immunotherapeutic agents, cell therapy agents (such as CAR-T cells), antibiotics and probiotics. Said immunotherapeutic agent can also be an antibody targeting tumoral antigen, particularly selected from the group consisting of anti-Her2, anti-EGFR, anti-CD20, anti-CD19, anti-CD52.

In an embodiment, the invention relates to a combined therapy as defined above, wherein the second therapeutic agent is particularly selected from the group consisting of therapeutic vaccines, immune checkpoint blockers or activators, in particular of adaptive immune cells (T and B lymphocytes) and antibody-drug conjugates. Preferably, suitable agents for co-use with any of the humanized anti-hPD-1 antibodies or with the pharmaceutical composition according to the invention include an antibody binding to a co-stimulatory receptor (e.g., OX40, CD40, ICOS, CD27, HVEM or GITR), an agent that induces immunogenic cell death (e.g., a chemotherapeutic agent, a radio-therapeutic agent, an antiangiogenic agent, or an agent for targeted therapies), an agent that inhibits a checkpoint molecule (e.g., CTLA4, LAG3, TIM3, B7H3, B7H4, BTLA, or TIGIT), a cancer vaccine, an agent that modifies an immunosuppressive enzyme (e.g., IDO1 or iNOS), an agent that targets T_{reg} cells, an agent for adoptive cell therapy, or an agent that modulates myeloid cells.

In an embodiment, the invention relates to a combined therapy as defined above, wherein the second therapeutic agent is an immune checkpoint blocker or activator of adaptive immune cells (T and B lymphocytes) selected from the group consisting of anti-CTLA4, anti-CD2, anti-CD28, anti-CD40, anti-HVEM, anti-BTLA, anti-CD160, anti-TIGIT, anti-TIM-1/3, anti-LAG-3, anti-2B4, and anti-OX40, anti-CD40 agonist, CD40-L, TLR agonists, anti-ICOS, ICOS-L and B-cell receptor agonists.

In one embodiment, the additional or second therapeutic agent is an antibody targeting tumoral antigen, particularly selected from the group consisting of anti-Her2, anti-EGFR, anti-CD20, anti-CD19 and anti-CD52.

Specific examples of second therapeutic agents are provided in WO 2018/053106, pages 36-43.

Combination therapy could also rely on the combination of the administration of bifunctional molecule with surgery, chemotherapy (e.g. such as docetaxel or decarbazine), radiotherapy, immunotherapy (e.g. such as antibodies targeting CD40, CTLA-4), gene targeting and modulation and/or other agents such as immune-modulators, angiogenesis inhibitors and any combinations thereof.

### Kits

Any of the bifunctional molecules or compositions described herein may be included in a kit provided by the present disclosure, which is not claimed. The present disclosure particularly provides kits for use in enhancing immune responses and/or treating diseases (e.g. cancer and/or infection) associated with the PD-1 signaling.

In the context of the present disclosure, the term "kit" means two or more components (one of which corresponding to the bifunctional molecule, the nucleic acid molecule, the vector or the cell of the disclosure) packaged in a container, recipient or otherwise. A kit can hence be described as a set of products and/or utensils that are sufficient to achieve a certain goal, which can be marketed as a single unit.

Particularly, a kit according to the disclosure may comprise:
- a bifunctional molecule as defined above,
- a humanized anti-hPD1 antibody linked to an immunotherapeutic agent
- a nucleic acid molecule or a group of nucleic acid molecules encoding said bifunctional molecule,
- a vector comprising said nucleic acid molecule or group of nucleic acid molecules, and/or
- a cell comprising said vector or nucleic acid molecule or group of nucleic acid molecules.

The kit may thus include, in suitable container means, the pharmaceutical composition, and/or the bifunctional molecules, and/or host cells of the present disclosure, and/or vectors encoding the nucleic acid molecules of the present disclosure, and/or nucleic acid molecules or related reagents of the present disclosure. In some aspects, means of taking a sample from an individual and/or of assaying the sample may be provided. In certain aspects the kit includes cells, buffers, cell media, vectors, primers, restriction enzymes, salts, and so forth. The kits may also comprise means for containing a sterile, pharmaceutically acceptable buffer and/or other diluent.

The containers may be unit doses, bulk packages (e.g., multi-dose packages) or sub-unit doses. In an aspect, the disclosure relates to a kit as defined above for a single-dose administration unit. The kit of the disclosure may also contain a first recipient comprising a dried/lyophilized bifunctional molecule and a second recipient comprising an aqueous formulation. In certain aspects of this disclosure, kits containing single-chambered and multi-chambered pre-filled syringes (e.g., liquid syringes and lyosyringes) are provided.

The kits of this disclosure are in suitable packaging. Suitable packaging includes, but is not limited to, vials, bottles, jars, flexible packaging (e.g., sealed Mylar or plastic bags), and the like. Also contemplated are packages for use in combination with a specific device, such as an inhaler, nasal administration device (e.g., an atomizer) or an infusion device such as a minipump. A kit may have a sterile access port (for example the container may be an intravenous solution bag or a vial having a stopper penetrable by a hypodermic injection needle). The container may also have a sterile access port (for example the container may be an intravenous solution bag or a vial having a stopper penetrable by a hypodermic injection needle). At least one active agent in the composition is a bifunctional molecule as described herein comprising a humanized anti-hPD1 antibody linked to an immunotherapeutic agent.

The compositions comprised in the kit according to the disclosure may also be formulated into a syringe compatible composition. In this case, the container means may itself be a syringe, pipette, and/or other such like apparatus, from which the formulation may be applied to an infected area of the body, and/or even applied to and/or mixed with the other components of the kit. The components of the kit may alternatively be provided as dried powder(s). When reagents and/or components are provided as a dry powder, a soluble composition can be reconstituted by the addition of a suitable solvent. It is envisioned that the solvent may also be provided in another container means and be suitable for administration.

In some aspects, the kit further includes an additional agent for treating cancer or an infectious disease, and the additional agent may be combined with the bifunctional molecule, or other components of the kit of the present disclosure or may be provided separately in the kit. Particularly, the kit described herein may include one or more additional therapeutic agents such as those described in the "Combined Therapy" described hereabove. The kit(s) may be tailored to a particular cancer for an individual and comprise respective second cancer therapies for the individual as described hereabove.

The instructions related to the use of the bifunctional molecule or pharmaceutical composition described herein generally include information as to dosage, dosing schedule, route of administration for the intended treatment, means for reconstituting the bifunctional molecule and/or means for diluting the bifunctional molecule of the disclosure. Instructions supplied in the kits of the disclosure are typically written instructions on a label or package insert (e.g., a paper sheet included in the kit in the form of a leaflet or instruction manual). In some aspects, the kit can comprise instructions for use in accordance with any of the methods described herein. The included instructions can comprise a description of administration of the pharmaceutical composition comprising the bifunctional molecule to enhance immune responses and/or to treat a disease as described herein. The kit may further comprise a description of selecting an individual suitable for a treatment based on identifying whether that individual has a disease associated with the PD-1 signaling, e.g., those described herein.

### EXAMPLES

The following Figures and Examples are put forth so as to provide those of ordinary skill in the art with a complete disclosure and description of how to make and use the present invention.

### RESULTS

### Example 1: Productive and binding characteristics of the bifunctional anti-PD1 molecule and its interaction with PDL1 and against the ligand of the

Figure 1 shows that the bifunctional molecule of the invention with humanized anti-PD1 antibodies fused on both heavy and light chains present an improvement of the productive yield compared to the bifunctional molecules with the chimeric anti-PD1 antibodies. As shown figure 1, bifunctional molecules fused to type1 (A (i.e., CD86), B (i.e., CD80) and C (i.e., SIRPa)) or type 2 proteins (A (i.e., OX40L) and B (i.e., 4-1BBL) and cytokines (i.e., IL-7) on any heavy (A) or light chain (B) are better produced than the bifunctional molecule with chimeric version of anti-PD1 antibodies. Figure 1 C shows an improvement of the productive yield as well when cytokine (i.e., IL-7) is fused to heavy and light chains. This molecule includes 4 cytokines fused to the dimer antibody.

The productive yield of the bifunctional molecules with humanized anti PD-1 backbone was compared to two other anti PD-1 backbone Keytruda (pembrolizumab) and Opdivo (Nivolumab). The Figure 2A and B demonstrate that the humanized anti PD-1 antibody of the invention presents a better productivity yield of the bifunctional molecules fused with a cytokine (i.e., IL-7) or type I protein B (i.e., CD80) in comparison to the other anti PD-1 backbones. This improvement was observed using 2 different producer cell lines (CHO and HEK freestyle) and 2 different methods of production (shaking flask versus 12 well plate). To confirm this increase with other fusion type I protein, the inventors generated bifunctional molecules fused to SIRPa with pembrolizumab backbone. As shown in the figure 2C, a higher production yield in cells was also obtained with humanized anti PD-1 backbone of the invention compared to Keytruda backbone. In manufacturing process in fed-batch culture (unoptimized fed-batch CHO cell production in bioreactor), a good productivity (1g/L) of the bifunctional molecules with humanized anti PD-1 fused to CD80 type I protein.

In parallel, the inventors constructed bifunctional molecules with various non anti PD-1 backbone antibodies fused to cytokine or type I or II protein on the heavy or light chain and compared their productivity with the bifunctional molecules with humanized anti PD-1 of the invention. Figure 3 demonstrates that all bifunctional molecules with anti PD-1 humanized backbone of the invention significantly have a higher production yield in contrast to any other backbones confirming the high manufacturability of humanized anti PD-1 described in this invention.

In parallel, inventors compared the PD1 binding of the bifunctional molecules with chimeric or humanized anti-PD1 antibodies. Figure 4 and Table 1 below, show that the immunotherapeutic agents from any type when fused on the heavy or light chain of the antibody keep a good binding to the PD1 antigen. The bifunctional molecules lose a slight efficacy of the binding compared to the control anti-PD1 antibody alone but it remains a strong binder to PD-1 (EC50 < 10 ng/ml).

However, using surface plasmon resonance experiment (Biacore assay), the bifunctional molecules have a similar range affinity to PD-1 compared to the control anti PD-1 alone. The Biacore is the standard experiment used to measure the affinity of the protein and is more sensitive and accurate than ELISA assay. For this assay, anti-human Fc antibody on the sensor chip to capture anti PD-1 alone or the bifunctional molecule were. Then, different concentrations of PD-1 recombinant protein (6,25 to 100nM) were added to measure the affinity. A KD of 3,46nM was obtained for the anti PD-1 alone, a KD of 2,61nM was obtained for the anti PD-1 IL-7 and a KD of 3,83nM for the anti PD-1 SIRPa.

Figure 5 and 6 compare the binding of bifunctional molecules with chimeric and humanized anti-PD1 antibodies fused to different immunotherapeutic agents (type I or II or cytokine proteins) on the heavy chain (Figure 5) or light chain (Figure 6). The results and Tables 2 and 3, indicate that the binding is comparable between chimeric and humanized version of the bifunctional molecules and the fusion of the immunotherapeutic agent on heavy or light chain of the antibody. Inventors also tested a bifunctional molecule with an anti-PD1 antibody fused to heavy and light chains leading to 4 proteins of the same type on one antibody. When comparing data from tables 1, 2, 3, and 4, the EC50 values are comparable. However, the productive yield as presented Figure 1C is still better for the bifunctional molecule with the humanized anti-PD1 antibody than the bifunctional molecule with the chimeric anti-PD1 antibody.

**Table 1: EC50 from Figure 4 of PD-1 binding ELISA assay**

| Molecule | EC50 ng/mL | |
|---|---|---|
| | VH fusion | VL fusion |
| **Type I-protein A (CD86)** | 5.72 | 9.24 |
| **Type I-protein B (CD80)** | 4.18 | 7.79 |
| **Type I-protein C (SIRPa)** | 6.34 | 9.96 |
| **Type II-protein A (OX40L)** | 6.64 | 8 |
| **Type II-protein B (4-1BBL)** | 7.2 | 5.39 |
| **Cytokine-protein A (IL-7)** | 5 | 5.11 |
| **Anti-PD1 Ab alone, no fusion** | 1.67 | |

**Table 2: EC50 from Figure 5 of bifunctional molecules with chimeric and humanized anti-PD1 antibodies with a heavy chain fused to the immunotherapeutic agent.**

| | EC50 (ng/ml) | |
|---|---|---|
| Anti-PD1 fused on its heavy chain to | Chimeric | Humanized |
| **Type I-protein A (CD86)** | 4.92 | 7.13 |
| **Type I-protein B (CD80)** | 3.84 | 5.99 |
| **Type I-protein C (SIRPa)** | 5.99 | 6.84 |
| **Type II-protein A (OX40L)** | 3.89 | 4.86 |
| **Type II-protein B (4-1BBL)** | 2.40 | 6.67 |
| **Cytokine-protein A (IL-7)** | 5.18 | 7.71 |

**Table 3: EC50 from Figure 6 of bifunctional molecules with chimeric and humanized anti-PD1 antibodies with a light chain fused to the immunotherapeutic agent**

| | EC50 (ng/ml) | |
|---|---|---|
| Anti-PD1 fused on its light chain to | Chimeric | Humanized |
| **Type I-protein A (CD86)** | 3.53 | 8.89 |
| **Type I-protein B (CD80)** | 3.99 | 8.91 |
| **Type I-protein C (SIRPa)** | 6.12 | 10.04 |
| **Type II-protein A (OX40L)** | 4.22 | 5.41 |
| **Type II-protein B (4-1BBL)** | 4.68 | 8.00 |
| **Cytokine-protein A (IL-7)** | 5.28 | 6.17 |

**Table 4: EC50 from Figure 7 of bifunctional molecules with chimeric and humanized anti-PD1 antibodies with a light and heavy chains fused to the immunotherapeutic agent. The cytokine fused to anti PD-1 antibody was an IL-7.**

| Anti-PD1 fused to IL-7 on heavy and light chains | EC50 (ng/mL) |
|---|---|
| chimeric | 2.11 |
| humanized | 3.9 |

The antagonist property on PD1-PDL1 interaction was then analyzed for each bifunctional molecule with anti-PD1 antibodies fused to different immunotherapeutic agents compared to the anti-PD1 antibody alone. Figure 8A and Table 5 present results of the ELISA competitive assay. As previously suggested the antagonist property of the bifunctional molecules with anti-PD1 antibodies is not modified when antibody is fused to an immunotherapeutic agent while their binding to PD1 is slightly decreased as shown Figure 4. Antagonist properties on PD1-PDL2 of the bifunctional molecule anti PD-1 VH IL7 or anti PD-1 VH CD80 was also assessed by ELISA (Figure 8B). Both molecules efficiently block PD-L2 binding on PD-1.

**Table 5: IC50 (ng/ml) from Figure 8: Competition PD-1/PD-L1 ELISA assay.**

| Molecule Anti-PD1 fused to | IC50 (ng/ml) | |
|---|---|---|
| | VH fusion | VL fusion |
| Type I-protein A (CD86) | 169.6 | 351.3 |
| Type I-protein C (SIRPa) | 512.2 | 611.9 |
| Type II-protein A (OX40L) | 231.1 | 306.9 |
| Type II-protein B (4-1BBL) | 265.1 | 354.8 |
| Cytokine-protein A (IL-7) | 394.5 | 516.6 |
| Anti-PD1 Ab alone, no fusion | 184.3 | |

In order to test the binding of the immunotherapeutic agents to their ligands, the inventors performed a bridging ELISA assay. Figure 9 presents the results for type I and II proteins where the histogram represents the positive control of the binding of the immunotherapeutic agent alone to its ligand. The ligand binding of the immunotherapeutic agent is conserved when it's fused on the heavy or the light chain of the anti-PD1 antibody.

### Example 2: Ex vivo characterization of the bifunctional molecules with anti-PD1 antibody fused to immunotherapeutic agents on T cell proliferation and activation

In order to measure the efficiency of the bifunctional molecules anti-PD1 on T cell activation and proliferation, the inventors tested the T cell proliferation after treatment with anti-PD1 antibodies alone (pembrolizumab was used as an anti-PD1 antibody control) or with isotype control or with bifunctional molecules with anti-PD1 antibodies fused on VH or VL chains. Results presented Figure 10 show that any bifunctional molecules with anti-PD1 antibodies induce a T cell proliferation at least as good as the anti-PD1 alone or even better than the anti-PD1 alone. The assay measuring the T cell activation shows a very good efficiency on T cell activation as represented Figure 11 by the IFNg secretion. Indeed, bifunctional molecules with anti-PD1 antibodies induce T cell activation at least as good as the control anti-PD1 antibody or even more indicating that bifunctional molecules' format could potentiate the efficiency of the anti-PD1 antibody on T cell proliferation and activation in vivo and could be a better format to target T cells into the tumor.

The bifunctional molecules with anti-PD1 antibodies fused to one, two, three or four immunotherapeutic agents of the invention present a good binding to PD1 and a good inhibitory property against PD1-PDL1 interaction. They are better produced than the bifunctional molecules with chimeric version of the antibodies. Ex vivo they induce a better proliferation and activation on human T cells indicating that they will be more efficient in vivo compare to anti-PD1 antibody alone, particularly on intratumoral T cells.

### Example 3: Pharmacokinetics of the Bicki anti-PD1 antibody in vivo

To analyze the pharmacokinetics of the bifunctional molecules with humanized anti PD-1, mice were intravenously injected with 5mg/kg of bifunctional molecules with humanized anti PD-1 fused to SIRPa on the heavy chain. Two different isotypes, namely IgG1 N298A or IgG4S228P isotypes, were compared. Following injection, plasma concentration was assessed by ELISA at multiple time points. As shown in the Figure 12, the bifunctional molecules with humanized anti PD-1 constructed with a IgG1 N298A demonstrate a better pharmacokinetics profile compared to the bifunctional molecules with humanized anti PD-1 constructed with an IgG4 S228P isotype.

### MATERIAL AND METHODS

### ELISA binding PDI and bridging ELISA assay

For the PD-1 binding ELISA assay, recombinant hPD1 (Sino Biologicals, Beijing, China; reference 10377-H08H) was immobilized on plastic at 0.5µg/ml in carbonate buffer (pH 9.2) and purified antibody were added to measure binding. After incubation and washing, peroxidase-labeled donkey anti-human IgG (Jackson Immunoresearch; USA; reference 709-035-149) was added revealed by colorimetry at 450/650nm using TMB substrate (3,3',5,5' Tetramethylenzidine, BD Bioscience, San Jose, USA).

For bridging ELISA assay, a similar method was used. Recombinant hPD1 was immobilized and purified bifunctional antibodies were added at serial dilution. After incubation and washing, recombinant receptor of Protein C, A or B were then added at 1 µg/mL. Detection was performed using an anti-receptor specific mouse antibody and a peroxidase-labeled donkey anti mouse IgG antibody (Reference 715-036-151). Revelation was performed using conventional method.

### ELISA antagonist: competition between PDL1 or PD-L2 and humanised anti-PD1

Competitive ELISA assay was performed by PD-1:PD-L1 Inhibitor Screening ELISA Assay Pair (AcroBiosystems; USA; reference EP-101). In this assay, recombinant hPDL1 was immobilized on plastic at 2 µg/ml in PBS pH 7.4 buffer. Purified antibody (at different concentrations) were mixed with 0.66 µg/ml final (fix concentration) of biotinylated Human PD1 (AcroBiosystems; USA; reference EP-101) to measure competitive binding for 2h at 37°C. After incubation and washing, peroxidase-labeled streptavidin (Vector laboratoring; USA; reference SA-5004) was added to detect Biotin-CD47Fc binding and revealed by conventional methods. Same ELISA protocol was performed to assess PDL2/PD1 antagonist activity by coating PD-L2 Fc recombinant protein (Sinobiological,# 10292-H02H).

### IFN gamma secretion and T cell proliferation assay

Human T cells were purified from peripheral blood mononuclear cells using an untouched pan T cell isolation kit (reference 130-096-535, MACS Miltenyi Biotech; USA) and an Automacs pro separator (Miltenyi). T cells were incubated on CD3/CD28 coated plate (anti-CD3 clone OKT3 and anti-CD28 clone CD28.2, concentration 3ug/mL each) to stimulate the cells and induce PD-1 expression. Twenty-four hours following stimulation, T cells were harvested, counted and restimulated on anti-CD3 (clone OKT3, 2ug/mL) + recombinant human PD-L1 (Sinobiological, reference 10084-H02H, 5ug/mL) in the presence of an isotype control, un fused anti-PD-1 or anti- PD-1 bifunctional antibodies. At Day 6, supernatant was harvested to quantify IFNg secretion (human IFNg ELISA set, BD Bioscience, USA, reference 555142) and T cell proliferation was assessed by H3 thymidine incorporation.

### Pharmacokinetics and pharmacodynamics of the humanized anti-PD1 antibody in mice

BalbcRJ (female 6-9 weeks) were intra-orbitally with a single dose (34,4nM/kg) of the bifunctional humanized anti PD-1 antibody. Plasma drug concentration was determined by ELISA using an immobilized anti-human light chain antibody (clone NaM76-5F3) diluted serum containing anti-PD-1 antibody was added. Detection was performed with a peroxidase-labeled donkey anti-human IgG (Jackson Immunoresearch; USA; reference 709-035-149) was added and revealed by conventional methods.

### Antibodies and bifunctional molecules

The following antibodies and bifunctional molecules have been used in the different experiments disclosed herein: Pembrolizumab (Keytrudra, Merck) Nivolumab (Opdivo, Bristol-Myers Squibb) , and the bifunctional molecules as disclosed herein comprising an anti-PD1 humanized antibody comprising a heavy chain as defined in SEQ ID : 19, 22 or 24 and a light chain as defined in SEQ ID NO: 28 or an anti-PD1 chimeric antibody comprising an heavy chain as defined is SEQ ID NO: 59 and a light chain as defined in SEQ ID NO: 60.

## Claims

1. A bifunctional molecule comprising or consisting of:
(a) a humanized anti-human PD-1 antibody, which comprises:
(i) a heavy chain variable domain (VH) comprising or consisting of an amino acid sequence selected from the group consisting of SEQ ID NO: 19, 22 or 24, and (ii) a light chain variable domain (VL) comprising or consisting of an amino acid sequence of SEQ ID NO: 28,
and
(b) an immunotherapeutic agent or a fragment thereof, wherein the immunotherapeutic agent or a fragment thereof is a peptide, a polypeptide or a protein and has a size comprised between 10 kDa and 50 kDa; wherein the immunotherapeutic agent or fragment thereof is selected from the list consisting of cytokines, cytokines receptors, chemokines, chemokines receptors, and human transmembrane immune protein of type I or II, preferably the extracellular domain thereof,
wherein the C-terminal end of the heavy and/or light chain(s) of the antibody is covalently linked to the N-terminal end of the immunotherapeutic agent as a fusion protein, preferably by a peptide linker.

2. The bifunctional molecule of claim 1, wherein the humanized anti-human PD-1 comprises (a) a VH comprising or consisting of an amino acid sequence of SEQ ID NO: 24,; and (b) a VL comprising or consisting of an amino acid sequence of SEQ ID NO: 28.

3. The bifunctional molecule of any one of claims 1-2, wherein the antibody is an antagonist of the binding of human PD-L1 and/or PD-L2 to human PD-1.

4. The bifunctional molecule of any one of claims 1-3, wherein the immunotherapeutic agent is :
- a human transmembrane immune protein of type I or a fragment thereof selected from the group consisting of ICOSL, CD86, B7H4, B7H3, CD28H, PDL2, PDL1, DNAM, CTLA-4, Lag-3, TIGIT, 2B4, BTLA, HVEM, CD101, nectin-1, nectin-2, nectin-3, NELC-5, TLT-2, LFA-3, TIM3, TIM4, LAIR1, SIRPG, IL10R, IL6RA, IL-1R1, IL-1RAcP, IL6RB, TGFBRII, CSF1R, IL22R, VEGFR1, VEGFR2, VEGFR3, CD111, CD112, CD155, CD113, VISTA, CD244, OX40, SIRPalpha, CD80, CD24, Siglec-10, Fas, IL15RA, SIRB1, SIRB2, LTBR, IL21R and GITR; or
- a human transmembrane immune protein of type II or a fragment thereof selected from the group consisting of CD40L, OX40L, FasL, TRAIL, TNF, LIGHT, APRIL, GITRL, CD30, CD70, CD40, CD27, CD30, CD153, RANK, CD96, CLEC1, CLEC2 /CLE1B, CLEC3A, CLEC4A, CLEC4E, CLEC4L, CLEC51, CLEC6, CLEC7A, NKG2D, BTL-II, TGFRII, DECTIN-1, DC-SIGN, LT-alpha, LT-beta, 4-1BBL and MINCLE, preferably a member of the TNF family; or
- a cytokine or a fragment thereof selected from the group consisting of TGFβ, IL-1, IL-2, IL-4, IL-6, IL-7, IL-10, IL-12A, IL12B, IL-15, IL-21 and IL-18; or
- a human IL-2 or a mutant thereof, preferably as set forth in SEQ ID NO: 58 with the substitutions F42A, Y45A and L72G, preferably T3A, F42A, Y45A, L72G and C125A.

5. The bifunctional molecule of any one of claims 1 to 4, wherein the antibody comprises:
- a light chain constant domain derived from a human kappa light chain constant domain and a heavy chain constant domain derived from a human IgG1, IgG2, IgG3 or IgG4 heavy chain constant domain; or
- a light chain constant domain derived from a human kappa light chain constant domain and a heavy chain constant domain derived from a human IgG1 heavy chain constant domain, optionally with a substitution or a combination of substitutions selected from the group consisting of T250Q/M428L; M252Y/S254T/T256E + H433K/N434F; E233P/L234V/L235A/G236A + A327G/A330S/P331S; E333A; S239D/A330L/I332E; P257I/Q311; K326W/E333S; S239D/I332E/G236A; N297A; L234A/L235A; N297A + M252Y/S254T/T256E; K322A and K444A, preferably selected from the group consisting of N297A optionally in combination with M252Y/S254T/T256E, L234A/L235A, wherein the numbering of the residues is according to EU numbering; or
- a light chain constant domain derived from a human kappa light chain constant domain and a heavy chain constant domain derived from a human IgG4 heavy chain constant domain, optionally with a substitution or a combination of substitutions selected from the group consisting of S228P; L234A/L235A, S228P + M252Y/S254T/T256E and K444A, wherein the numbering of the residues is according to EU numbering.

6. An isolated nucleic acid sequence or a group of isolated nucleic acid molecules encoding the bifunctional molecule according to any one of claims 1-5.

7. A vector, comprising the nucleic acid or group of nucleic acid molecules according to claim 6.

8. A host cell, comprising the vector according to claim 7 or the nucleic acid or group of nucleic acid molecules of claim 6.

9. A method for producing the bifunctional molecule according to any one of claims 1-5, comprising a step of culturing a host cell according to claim 8 and optionally a step of isolating the bifunctional molecule.

10. A pharmaceutical composition comprising the bifunctional molecule according to any one of claims 1-5, the nucleic acid or group of nucleic acid molecules according to claim 6, the vector of claim 7 or the host cell of claim 8 and a pharmaceutically acceptable carrier.

11. The pharmaceutical composition according to claim 10, wherein it further comprises an additional therapeutic agent, preferably selected in the group consisting of alkylating agents, angiogenesis inhibitors, antibodies, antimetabolites, antimitotics, antiproliferatives, antivirals, aurora kinase inhibitors, apoptosis promoters (for example, Bcl-2 family inhibitors), activators of death receptor pathway, Bcr-Abl kinase inhibitors, BiTE (Bi-Specific T cell Engager) antibodies, antibody drug conjugates, biologic response modifiers, Bruton's tyrosine kinase (BTK) inhibitors, cyclin-dependent kinase inhibitors, cell cycle inhibitors, cyclooxygenase-2 inhibitors, DVDs, leukemia viral oncogene homolog (ErbB2) receptor inhibitors, growth factor inhibitors, heat shock protein (HSP)-90 inhibitors, histone deacetylase (HDAC) inhibitors, hormonal therapies, immunologicals, inhibitors of inhibitors of apoptosis proteins (IAPs), intercalating antibiotics, kinase inhibitors, kinesin inhibitors, Jak2 inhibitors, mammalian target of rapamycin inhibitors, microRNAs, mitogen-activated extracellular signal-regulated kinase inhibitors, multivalent binding proteins, non-steroidal anti-inflammatory drugs (NSAIDs), poly ADP (adenosine diphosphate)-ribose polymerase (PARP) inhibitors, platinum chemotherapeutics, polo-like kinase (Plk) inhibitors, phosphoinositide-3 kinase (PI3K) inhibitors, proteasome inhibitors, purine analogs, pyrimidine analogs, receptor tyrosine kinase inhibitors, retinoids/deltoids plant alkaloids, small inhibitory ribonucleic acids (siRNAs), topoisomerase inhibitors, ubiquitin ligase inhibitors, hypomethylating agents, checkpoints inhibitors, peptide vaccine and the like, epitopes or neoepitopes from tumor antigens, as well as combinations of one or more of these agents.

12. A pharmaceutical composition of claim 10 or 11 or a bifunctional molecule according to any one of claims 1-5, or a nucleic acid or group of nucleic acid molecules according to claim 6, or a vector of claim 7, or a host cell of claim 8 for use as a medicament.

13. The pharmaceutical composition, bifunctional molecule, nucleic acid or group of nucleic acid molecules, vector, or host cell for use according to claim 12, for use in the treatment of cancer, preferably a cancer selected from the group consisting of a hematologic malignancy or a solid tumor with expression of PD-1 and/or PD-L1 such as a cancer selected from the group consisting of hematolymphoid neoplasms, angioimmunoblastic T cell lymphoma, myelodysplastic syndrome, and acute myeloid leukemia, a cancer induced by virus or associated with immunodeficiency such as a cancer selected from the group consisting of Kaposi sarcoma (e.g., associated with Kaposi sarcoma herpes virus); cervical, anal, penile and vulvar squamous cell cancer and oropharyngeal cancers (e.g., associated with human papilloma virus); B cell non-Hodgkin lymphomas (NHL) including diffuse large B-cell lymphoma, Burkitt lymphoma, plasmablastic lymphoma, primary central nervous system lymphoma, HHV-8 primary effusion lymphoma, classic Hodgkin lymphoma, and lymphoproliferative disorders (e.g., associated with Epstein-Barr virus (EBV) and/or Kaposi sarcoma herpes virus); hepatocellular carcinoma (e.g., associated with hepatitis B and/or C viruses); Merkel cell carcinoma (e.g., associated with Merkel cell polyoma virus (MPV)); and cancer associated with human immunodeficiency virus infection (HIV) infection, and a cancer selected from the group consisting of metastatic or not metastatic, Melanoma, malignant mesothelioma, Non-Small Cell Lung Cancer, Renal Cell Carcinoma, Hodgkin's Lymphoma, Head and Neck Cancer, Urothelial Carcinoma, Colorectal Cancer, Hepatocellular Carcinoma, Small Cell Lung Cancer, Metastatic Merkel Cell Carcinoma, Gastric or Gastroesophageal cancers and Cervical Cancer.

14. The pharmaceutical composition, bifunctional molecule, nucleic acid or group of nucleic acid molecules, vector, or host cell for use according to any one of claims 12 or 13, for use in combination with radiotherapy or an additional therapeutic agent, preferably selected in the group consisting of alkylating agents, angiogenesis inhibitors, antibodies, antimetabolites, antimitotics, antiproliferatives, antivirals, aurora kinase inhibitors, apoptosis promoters (for example, Bcl-2 family inhibitors), activators of death receptor pathway, Bcr-Abl kinase inhibitors, BiTE (Bi-Specific T cell Engager) antibodies, antibody drug conjugates, biologic response modifiers, Bruton's tyrosine kinase (BTK) inhibitors, cyclin-dependent kinase inhibitors, cell cycle inhibitors, cyclooxygenase-2 inhibitors, DVDs, leukemia viral oncogene homolog (ErbB2) receptor inhibitors, growth factor inhibitors, heat shock protein (HSP)-90 inhibitors, histone deacetylase (HDAC) inhibitors, hormonal therapies, immunologicals, inhibitors of inhibitors of apoptosis proteins (IAPs), intercalating antibiotics, kinase inhibitors, kinesin inhibitors, Jak2 inhibitors, mammalian target of rapamycin inhibitors, microRNAs, mitogen-activated extracellular signal-regulated kinase inhibitors, multivalent binding proteins, non-steroidal anti-inflammatory drugs (NSAIDs), poly ADP (adenosine diphosphate)-ribose polymerase (PARP) inhibitors, platinum chemotherapeutics, polo-like kinase (Plk) inhibitors, phosphoinositide-3 kinase (PI3K) inhibitors, proteasome inhibitors, purine analogs, pyrimidine analogs, receptor tyrosine kinase inhibitors, retinoids/deltoids plant alkaloids, small inhibitory ribonucleic acids (siRNAs), topoisomerase inhibitors, ubiquitin ligase inhibitors, hypomethylating agents, checkpoints inhibitors, peptide vaccine and the like, epitopes or neoepitopes from tumor antigens, as well as combinations of one or more of these agents.

15. The pharmaceutical composition, bifunctional molecule, nucleic acid or group of nucleic acid molecules, vector, or host cell for use according to claim 12, for use in the treatment of infectious disease, preferably chronic infectious disease, even more preferably chronic viral infections, preferably an infectious disease caused by a virus selected from the group consisting of HIV, hepatitis virus, herpes virus, adenovirus, influenza virus, flaviviruses, echovirus, rhinovirus, coxsackie virus, coronavirus, respiratory syncytial virus, mumps virus, rotavirus, measles virus, rubella virus, parvovirus, vaccinia virus, HTLV virus, dengue virus, papillomavirus, molluscum virus, poliovirus, rabies virus, JC virus and arboviral encephalitis virus.

## Patentansprüche

1. Bifunktionelles Molekül, umfassend oder bestehend aus:
(a) einem humanisierten Anti-Human-PD-1-Antikörper, der Folgendes umfasst:
(i) eine variable Domäne der schweren Kette (VH), die eine Aminosäuresequenz ausgewählt aus der Gruppe bestehend aus SEQ ID NO: 19, 22 oder 24 umfasst oder daraus besteht, und (ii) eine variable Domäne der leichten Kette (VL), die eine Aminosäuresequenz der SEQ ID NO: 28 umfasst oder daraus besteht,
und
(b) ein Immuntherapeutikum oder ein Fragment davon, wobei das Immuntherapeutikum oder ein Fragment davon ein Peptid, ein Polypeptid oder ein Protein ist und eine Größe zwischen 10 kDa und 50 kDa aufweist; wobei das Immuntherapeutikum oder Fragment davon aus der Liste ausgewählt ist, die aus Zytokinen, Zytokinrezeptoren, Chemokinen, Chemokinrezeptoren und humanem transmembranösem Immunprotein vom Typ I oder II, vorzugsweise dessen extrazellulärer Domäne, besteht,
wobei das C-terminale Ende der schweren und/oder leichten Kette(n) des Antikörpers kovalent mit dem N-terminalen Ende des Immuntherapeutikums als Fusionsprotein verknüpft ist, vorzugsweise durch einen Peptidlinker.

2. Das bifunktionelle Molekül nach Anspruch 1, wobei der humanisierte Anti-Human-PD-1 (a) eine VH, umfassend oder bestehend aus einer Aminosäuresequenz der SEQ ID NO: 24, und (b) eine VL, umfassend oder bestehend aus einer Aminosäuresequenz der SEQ ID NO: 28, umfasst.

3. Das bifunktionelle Molekül nach einem der Ansprüche 1-2, wobei der Antikörper ein Antagonist der Bindung von humanem PD-L1 und/oder PD-L2 an humanes PD-1 ist.

4. Das bifunktionelle Molekül nach einem der Ansprüche 1-3, wobei das Immuntherapeutikum ist:
- ein humanes transmembranöses Immunprotein vom Typ I oder ein Fragment davon, ausgewählt aus der Gruppe bestehend aus ICOSL, CD86, B7H4, B7H3, CD28H, PDL2, PDL1, DNAM, CTLA-4, Lag-3, TIGIT, 2B4, BTLA, HVEM, CD101, Nectin-1, Nectin-2, Nectin-3, NELC-5, TLT-2, LFA-3, TIM3, TIM4, LAIR1, SIRPG, IL10R, IL6RA, IL-1R1, IL-1RAcP, IL6RB, TGFBRII, CSF1R, IL22R, VEGFR1, VEGFR2, VEGFR3, CD111, CD112, CD155, CD113, VISTA, CD244, OX40, SIRPalpha, CD80, CD24, Siglec-10, Fas, IL15RA, SIRB1, SIRB2, LTBR,IL21R und GITR; oder
- ein humanes transmembranöses Immunprotein vom Typ II oder ein Fragment davon, ausgewählt aus der Gruppe bestehend aus CD40L, OX40L, FasL, TRAIL, TNF, LIGHT, APRIL, GITRL, CD30, CD70, CD40, CD27, CD30, CD153, RANK, CD96, CLEC1, CLEC2/CLE1B, CLEC3A, CLEC4A, CLEC4E, CLEC4L, CLEC51, CLEC6, CLEC7A, NKG2D, BTL-II, TGFRII, DECTIN-1, DC-SIGN, LT-alpha, LT-beta, 4-1BBL und MINCLE, vorzugsweise einem Mitglied der TNF-Familie; oder
- ein Zytokin oder ein Fragment davon, ausgewählt aus der Gruppe bestehend aus TGFβ, IL-1, IL-2, IL-4, IL-6, IL-7, IL-10, IL-12A, IL-12B, IL-15, IL-21 und IL-18; oder
- ein humanes IL-2 oder eine Mutante davon, vorzugsweise wie in SEQ ID NO: 58 gezeigt, mit den Substitutionen F42A, Y45A und L72G, vorzugsweise T3A, F42A, Y45A, L72G und C125A.

5. Das bifunktionelle Molekül nach einem der Ansprüche 1 bis 4, wobei der Antikörper Folgendes umfasst:
- eine konstante Leichtkettendomäne, abgeleitet von einer humanen konstanten Kappa-Leichtkettendomäne, und eine konstante Schwerkettendomäne, abgeleitet von einer humanen konstanten IgGl-, IgG2-, IgG3- oder IgG4-Schwerkettendomäne; oder
- eine konstante Leichtkettendomäne, abgeleitet von einer humanen konstanten Kappa-Leichtkettendomäne, und eine konstante Schwerkettendomäne, abgeleitet von einer humanen konstanten IgG1-Schwerkettendomäne, optional mit einer Substitution oder einer Kombination von Substitutionen ausgewählt aus der Gruppe bestehend aus T2500/M428L; M252Y/S254T/T256E + H433K/N434F; E233P/L234V/L235A/G236A + A327G/A330S/P331S; E333A; S239D/A330L/I332E; P2571/Q311; K326W/E333S; S239D/I332E/G236A; N297A; L234A/L235A; N297A + M252Y/S254T/T256E; K322A und K444A, vorzugsweise ausgewählt aus der Gruppe bestehend aus N297A, optional in Kombination mit M252Y/S254T/T256E, L234A/L235A, wobei die Nummerierung der Reste der EU-Nummerierung entspricht; oder
- eine konstante Leichtkettendomäne, abgeleitet von einer konstanten Domäne einer humanen Kappa-Leichtkettendomäne, und eine konstante Schwerkettendomäne, abgeleitet von einer konstanten Domäne einer humanen IgG4-Schwerkettendomäne, optional mit einer Substitution oder einer Kombination von Substitutionen, ausgewählt aus der Gruppe bestehend aus S228P; L234A/L235A, S228P + M252Y/S254T/T256E und K444A, wobei die Nummerierung der Reste der EU-Nummerierung entspricht.

6. Isolierte Nukleinsäuresequenz oder Gruppe isolierter Nukleinsäuremoleküle, die das bifunktionelle Molekül nach einem der Ansprüche 1-5 kodiert.

7. Vektor, umfassend die Nukleinsäure oder Gruppe von Nukleinsäuremolekülen nach Anspruch 6.

8. Wirtszelle, umfassend den Vektor nach Anspruch 7 oder die Nukleinsäure oder Gruppe von Nukleinsäuremolekülen nach Anspruch 6.

9. Verfahren zur Herstellung des bifunktionellen Moleküls nach einem der Ansprüche 1-5, umfassend die Kultivierung einer Wirtszelle nach Anspruch 8 und optional einen Schritt der Isolierung des bifunktionellen Moleküls.

10. Pharmazeutische Zusammensetzung, umfassend das bifunktionelle Molekül nach einem der Ansprüche 1-5, die Nukleinsäure oder Gruppe von Nukleinsäuremolekülen nach Anspruch 6, den Vektor nach Anspruch 7 oder die Wirtszelle nach Anspruch 8 und einen pharmazeutisch verträglichen Träger.

11. Die pharmazeutische Zusammensetzung nach Anspruch 10, wobei sie ferner ein zusätzliches therapeutisches Mittel umfasst, vorzugsweise ausgewählt aus der Gruppe bestehend aus Angiogenese-Inhibitoren, Antikörpern, Antimetaboliten, Antimitotika, Antiproliferativa, Virostatika, Aurora-Kinase-Inhibitoren, Apoptose-Promotoren (z. B. Inhibitoren der Bcl-2-Familie), Aktivatoren des Todesrezeptor-Signalwegs, Bcr-Abl-Kinase-Inhibitoren, BiTE-(Bi-Specific T cell Engager) Antikörpern, Antikörper-Wirkstoff-Konjugaten, Modifikatoren der biologischen Reaktion, Bruton-Tyrosinkinase (BTK)-Inhibitoren, Cyclinabhängigen Kinase-Inhibitoren, Zellzyklus-Inhibitoren, Cyclooxygenase-2-Inhibitoren, DVDs, Leukämie-Virus-Onkogen-Homolog (ErbB2)-Rezeptor-Inhibitoren, Wachstumsfaktor-Inhibitoren, Hitzeschockprotein (HSP)-90-Inhibitoren, Histon-Deacetylase (HDAC)-Inhibitoren, Hormontherapien, Immunologika, Inhibitoren von Inhibitoren von Apoptoseproteinen (IAPs), interkalierende Antibiotika, Kinaseinhibitoren, Kinesininhibitoren, Jak2-Inhibitoren, Mammalian Target of Rapamycin-Inhibitoren, microRNAs, Inhibitoren der mitogenaktivierten extrazellulären Signalregulierten Kinase, multivalente Bindungsproteine, nichtsteroidale Antiphlogistika (NSAIDs), Inhibitoren der Poly-ADP (Adenosindiphosphat)-Ribose-Polymerase (PARP), Platin-Chemotherapeutika, Polo-like-Kinase(Plk)-Inhibitoren, Phosphoinositid-3-Kinase (PI3K)-Inhibitoren, Proteasom-Inhibitoren, Purinanaloga, Pyrimidinanaloga, Rezeptor-Tyrosinkinase-Inhibitoren, Retinoide/Deltoide Pflanzenalkaloide, kleine inhibitorische Ribonukleinsäuren (siRNAs), Topoisomerase-Inhibitoren, Ubiquitinligase-Inhibitoren, Hypomethylierungsmittel, Checkpoint-Inhibitoren, Peptidimpfstoffe und dergleichen, Epitope oder Neoepitope von Tumorantigenen sowie Kombinationen aus einem oder mehreren dieser Mittel.

12. Pharmazeutische Zusammensetzung nach Anspruch 10 oder 11 oder bifunktionelles Molekül nach einem der Ansprüche 1-5 oder Nukleinsäure oder Gruppe von Nukleinsäuremolekülen nach Anspruch 6 oder Vektor nach Anspruch 7 oder Wirtszelle nach Anspruch 8 zur Verwendung als Medikament.

13. Die pharmazeutische Zusammensetzung, das bifunktionale Molekül, die Nukleinsäure oder Gruppe von Nukleinsäuremolekülen, der Vektor oder die Wirtszelle zur Verwendung nach Anspruch 12 zur Verwendung bei der Behandlung von Krebs, vorzugsweise einer Krebsart ausgewählt aus der Gruppe bestehend aus einer hämatologischen Malignität oder einem soliden Tumor mit Expression von PD-1 und/oder PD-L1, wie etwa einer Krebsart ausgewählt aus der Gruppe bestehend aus hämatolymphoiden Neoplasien, angioimmunoblastischem T-Zell-Lymphom, myelodysplastischem Syndrom und akuter myeloischer Leukämie, einer durch ein Virus induzierten oder mit einer Immunschwäche verbundenen Krebsart, wie etwa einer Krebsart ausgewählt aus der Gruppe bestehend aus Kaposi-Sarkom (z. B. verbunden mit dem Kaposi-Sarkom-Herpesvirus); Plattenepithelkarzinom des Gebärmutterhalses, des Analbereichs, des Penis und der Vulva sowie Oropharynxkarzinomen (z. B. verbunden mit dem humanen Papillomavirus); B-Zell-Non-Hodgkin-Lymphome (NHL), einschließlich diffusem großzelligem B-Zell-Lymphom, Burkitt-Lymphom, plasmablastischem Lymphom, primärem Lymphom des zentralen Nervensystems, primärem HHV-8-Ergusslymphom, klassischem Hodgkin-Lymphom und lymphoproliferativen Erkrankungen (z. B. assoziiert mit dem Epstein-Barr-Virus (EBV) und/oder dem Kaposi-Sarkom-Herpesvirus); hepatozellulärem Karzinom (z. B. assoziiert mit Hepatitis B- und/oder C-Viren); Merkelzellkarzinom (z. B. assoziiert mit dem Merkelzell-Polyomavirus (MPV)); und Krebs im Zusammenhang mit einer Infektion mit dem humanen Immundefizienzvirus (HIV), und einem Krebs, ausgewählt aus der Gruppe bestehend aus metastasiertem oder nicht metastasiertem Melanom, malignem Mesotheliom, nichtkleinzelligem Lungenkrebs, Nierenzellkarzinom, Hodgkin-Lymphom, Kopf- und Halskrebs, Urothelkarzinom, Dickdarmkrebs, Leberzellkarzinom, kleinzelligem Lungenkrebs, metastasiertem Merkelzellkarzinom, Magen- oder Gastroösophagealkrebs und Gebärmutterhalskrebs.

14. Die pharmazeutische Zusammensetzung, das bifunktionelle Molekül, die Nukleinsäure oder Gruppe von Nukleinsäuremolekülen, der Vektor oder die Wirtszelle zur Verwendung nach einem der Ansprüche 12 oder 13 zur Verwendung in Kombination mit einer Strahlentherapie oder einem zusätzlichen therapeutischen Mittel, vorzugsweise ausgewählt aus der Gruppe bestehend aus Alkylierungsmitteln, Angiogenese-Inhibitoren, Antikörpern, Antimetaboliten, Antimitotika, Antiproliferativa, Virostatika, Aurora-Kinase-Inhibitoren, Apoptose-Promotoren (zum Beispiel Inhibitoren der Bcl-2-Familie), Aktivatoren des Todesrezeptor-Signalwegs, Bcr-Abl-Kinase-Inhibitoren, BiTE- (Bi-Specific T cell Engager) Antikörpern, Antikörper-Wirkstoff-Konjugaten, biologischen Reaktionsmodifikatoren, Bruton-Tyrosinkinase (BTK)-Inhibitoren, Cyclin-abhängigen Kinase-Inhibitoren, Zellzyklus-Inhibitoren, Cyclooxygenase-2-Inhibitoren, DVDs, Leukämie-Virus-Onkogen-Homolog (ErbB2). Rezeptorinhibitoren, Wachstumsfaktorinhibitoren, Hitzeschockprotein (HSP)-90-Inhibitoren, Histondeacetylase (HDAC)-Inhibitoren, Hormontherapien, Immunologika, Inhibitoren von Inhibitoren von Apoptoseproteinen (IAPs), interkalierende Antibiotika, Kinaseinhibitoren, Kinesininhibitoren, Jak2-Inhibitoren, Mammalian Target of Rapamycin-Inhibitoren, microRNAs, mitogenaktivierte extrazelluläre signalregulierte Kinaseinhibitoren, multivalente Bindungsproteine, nichtsteroidale Antiphlogistika (NSAIDs), Poly-ADP (Adenosindiphosphat)-Ribose-Polymerase (PARP)-Inhibitoren, Platin-Chemotherapeutika, Polo-like-Kinase (Plk)-Inhibitoren, Phosphoinositid-3-Kinase (PI3K)-Inhibitoren, Proteasom-Inhibitoren, Purin-Analoga, Pyrimidin-Analoga, Rezeptor-Tyrosin-Kinase-Inhibitoren, Retinoide/Deltoide, Pflanzenalkaloide, kleine inhibitorische Ribonukleinsäuren (siRNAs), Topoisomerase-Inhibitoren, Ubiquitin-Ligase-Inhibitoren, Hypomethylierungsmittel, Checkpoint-Inhibitoren, Peptidimpfstoffe und dergleichen, Epitope oder Neoepitope von Tumorantigenen sowie Kombinationen aus einem oder mehreren dieser Mittel.

15. Die pharmazeutische Zusammensetzung, das bifunktionelle Molekül, die Nukleinsäure oder Gruppe von Nukleinsäuremolekülen, der Vektor oder die Wirtszelle zur Verwendung nach Anspruch 12 zur Verwendung bei der Behandlung von Infektionskrankheiten, vorzugsweise chronischen Infektionskrankheiten, noch bevorzugter chronischen Virusinfektionen, vorzugsweise einer Infektionskrankheit, die durch ein Virus verursacht wird, ausgewählt aus der Gruppe bestehend aus HIV, Hepatitisvirus, Herpesvirus, Adenovirus, Influenzavirus, Flaviviren, Echovirus, Rhinovirus, Coxsackievirus, Coronavirus, Respiratorischem Synzytialvirus, Mumpsvirus, Rotavirus, Masernvirus, Rötelnvirus, Parvovirus, Vacciniavirus, HTLV-Virus, Denguevirus, Papillomavirus, Molluscumvirus, Poliovirus, Tollwutvirus, JC-Virus und Arboviralem Enzephalitisvirus.

## Revendications

1. Molécule bifonctionnelle comprenant ou consistant en :
(a) un anticorps anti-PD-1 humain humanisé, qui comprend :
(i) un domaine variable de chaîne lourde (VH) comprenant ou consistant en une séquence d'acides aminés choisie dans le groupe constitué par les SEQ ID NO : 19, 22 ou 24, et (ii) un domaine variable de chaîne légère (VL) comprenant ou consistant en une séquence d'acides aminés de SEQ ID NO : 28,
et
(b) un agent immunothérapeutique ou un fragment de celui-ci, lequel l'agent immunothérapeutique ou fragment de celui-ci est un peptide, un polypeptide ou une protéine, et a une taille comprise entre 10 kDa et 50 kDa ; lequel agent immunothérapeutique ou fragment de celui-ci est choisi dans la liste constituée par les cytokines, les récepteurs de cytokines, les chimiokines, les récepteurs de chimiokines, et les protéines immunitaires transmembranaire humaine de type I ou II, de préférence leur domaine extracellulaire,
dans laquelle l'extrémité C-terminale de la ou des chaînes lourde et/ou légère de l'anticorps est liée de manière covalente à l'extrémité N-terminale de l'agent immunothérapeutique en tant que protéine de fusion, de préférence par un lieur peptidique.

2. Molécule bifonctionnelle selon la revendication 1, dans lequel l'anti-PD-1 humain humanisé comprend (a) une VH comprenant ou consistant en une séquence d'acides aminés de la SEQ ID NO : 24 ; et (b) une VL comprenant ou consistant en une séquence d'acides aminés de la SEQ ID NO : 28.

3. Molécule bifonctionnelle selon l'une quelconque des revendications 1 et 2, dans laquelle l'anticorps est un antagoniste de la liaison de PD-L1 et/ou PD-L2 humains à PD-1 humain.

4. Molécule bifonctionnelle selon l'une quelconque des revendications 1 à 3, dans laquelle l'agent immunothérapeutique est :
- une protéine immunitaire transmembranaire humaine de type I ou un fragment de celle-ci, choisi dans le groupe constitué par les ICOSL, CD86, B7H4, B7H3, CD28H, PDL2, PDL1, DNAM, CTLA-4, Lag-3, TIGIT, 2B4, BTLA, HVEM, CD101, nectine-1, nectine-2, nectine-3, NELC-5, TLT-2, LFA-3, TIM3, TIM4, LAIR1, SIRPG, IL10R, IL6RA, IL-1R1, IL-1RAcP, IL6RB, TGFBRII, CSF1R, IL22R, VEGFR1, VEGFR2, VEGFR3, CD111, CD112, CD155, CD113, VISTA, CD244, OX40, SIRPalpha, CD80, CD24, Siglec-10, Fas, IL15RA, SIRB1, SIRB2, LTBR, IL21R et GITR ; ou
- une protéine immunitaire transmembranaire humaine de type II ou un fragment de celle-ci, choisi dans le groupe constitué par les CD40L, OX40L, FasL, TRAIL, TNF, LIGHT, APRIL, GITRL, CD30, CD70, CD40, CD27, CD30, CD153, RANK, CD96, CLEC1, CLEC2/CLE1B, CLEC3A, CLEC4A, CLEC4E, CLEC4L, CLEC51, CLEC6, CLEC7A, NKG2D, BTL-II, TGFRII, DECTIN-1, DC-SIGN, LT-alpha, LT-bêta, 4-1BBL et MINCLE, de préférence un membre de la famille des TNF ; ou
- une cytokine ou un fragment de celle-ci, choisi dans le groupe constitué par les TGFβ, IL-1, IL-2, IL-4, IL-6, IL-7, IL-10, IL-12A, IL-12B, IL-15, IL-21 et IL-18 ; ou
- une IL-2 humaine ou un mutant de celle-ci, de préférence tel que présenté dans la SEQ ID NO : 58 avec les substitutions F42A, Y45A et L72G, de préférence T3A, F42A, Y45A, L72G et C125A.

5. Molécule bifonctionnelle selon l'une quelconque des revendications 1 à 4, dans laquelle l'anticorps comprend :
- un domaine constant de chaîne légère dérivé d'un domaine constant de chaîne légère kappa humaine et un domaine constant de chaîne lourde dérivé d'un domaine constant de chaîne lourde d'IgG1, IgG2, IgG3 ou IgG4 ; ou
- un domaine constant de chaîne légère dérivé d'un domaine constant de chaîne légère kappa humaine et un domaine constant de chaîne lourde dérivé d'un domaine constant de chaîne lourde d'IgG1, éventuellement avec une substitution ou une combinaison de substitutions choisie dans le groupe constitué par T250Q/M428L ; M252Y/S254T/T256E + H433K/N434F ; E233P/L234V/L235A/G236A + A327G/A330S/P331S ; E333A; S239D/A330L/I332E ; P257I/Q311 ; K326W/E333S ; S239D/I332E/G236A ; N297A ; L234A/L235A ; N297A + M252Y/S254T/T256E ; K322A et K444A, de préférence choisie dans le groupe constitué par N297A éventuellement en combinaison avec M252Y/S254T/T256E, L234A/L235A., dans lequel la numérotation des résidus est conforme à la numérotation UE ; ou
- un domaine constant de chaîne légère dérivé d'un domaine constant de chaîne légère kappa humaine et un domaine constant de chaîne lourde dérivé d'un domaine constant de chaîne lourde d'IgG4, éventuellement avec une substitution ou une combinaison de substitutions choisie dans le groupe constitué par S228P ; L234A/L235A, S228P + M252Y/S254T/T256E et K444A, dans lequel la numérotation des résidus est conforme à la numérotation UE.

6. Séquence d'acide nucléique isolée ou groupe de molécules d'acide nucléique isolées codant la molécule bifonctionnelle de l'une quelconque des revendications 1 à 5.

7. Vecteur comprenant l'acide nucléique ou le groupe de molécules d'acide nucléique de la revendication 6.

8. Cellule hôte comprenant le vecteur de la revendication 7 ou l'acide nucléique ou le groupe de molécules d'acide nucléique de la revendication 6.

9. Méthode pour produire la molécule bifonctionnelle de l'une quelconque des revendications 1 à 5, comprenant une étape de culture d'une cellule hôte de la revendication 8 et éventuellement une étape d'isolation de la molécule bifonctionnelle.

10. Composition pharmaceutique comprenant la molécule bifonctionnelle de l'une quelconque des revendications 1 à 5, l'acide nucléique ou le groupe de molécules d'acide nucléique de la revendication 6, le vecteur de la revendication 7 ou la cellule hôte de la revendication 8, et un véhicule pharmaceutiquement acceptable.

11. Composition pharmaceutique selon la revendication 10, qui comprend en outre un agent thérapeutique additionnel, de préférence choisi dans le groupe constitué par les agents d'alkylation, les inhibiteurs d'angiogenèse, les anticorps, les antimétabolites, les antimitotiques, les antiprolifératifs, les antiviraux, les inhibiteurs de kinase Aurora, les promoteurs d'apoptose (par exemple les inhibiteurs de la famille de la Bcl-2), les activateurs de la voie des récepteurs de mort, les inhibiteurs de kinase Bcr-Abl, les anticorps BiTE (engageant les cellules T bispécifiques), les conjugués anticorps-médicament, les modificateurs de réponse biologique, les inhibiteurs de tyrosine kinase de Bruton (BTK), les inhibiteurs de kinase dépendant de la cycline, les inhibiteurs du cycle cellulaire, les inhibiteurs de cyclooxygénase-2, les DVD, les inhibiteurs de récepteurs d'homologues aux oncogènes viraux de la leucémie (ErbB2), les inhibiteurs de facteur de croissance, les inhibiteurs de la protéine de choc thermique (HSP)-90, les inhibiteurs de l'histone désacétylase (HDAC), les hormonothérapies, les immunologiques, les inhibiteurs d'inhibiteurs de protéines de l'apoptose (IAP), les antibiotiques d'intercalation, les inhibiteurs de kinase, les inhibiteurs de kinésine, les inhibiteurs de Jak2, les inhibiteurs de cible mammifère de la rapamycine, les microARN, les inhibiteurs de kinases régulées par un signal extracellulaire activé par un mitogène, les protéines de liaison multivalentes, les anti-inflammatoires non stéroïdiens (AINS), les inhibiteurs de poly[ADP (adénosine diphosphate)ribose] polymérase (PARP), les chimiothérapeutiques au platine, les inhibiteurs de kinase Polo-like (Pkl), les inhibiteurs de phosphoinositide-3 kinase (PI3K), les inhibiteurs de protéasome, les analogues de purine, les analogues de pyrimidine, les inhibiteurs de récepteurs de tyrosine kinase, les alcaloïdes végétaux rétinoïdes/deltoïdes, les petits acides ribonucléiques inhibiteurs (ARNsi), les inhibiteurs de topoisomérase, les inhibiteurs d'ubiquitine ligase, les agents d'hypométhylation, les inhibiteurs de point de contrôle, les vaccins peptidiques et analogues, les épitopes ou néoépitopes issus d'antigènes tumoraux, ainsi que les combinaisons d'un ou plusieurs de ces agents.

12. Composition pharmaceutique selon la revendication 10 ou 11, ou molécule bifonctionnelle selon l'une quelconque des revendications 1 à 5, ou acide nucléique ou groupe de molécules d'acide nucléique selon la revendication 6, ou vecteur selon la revendication 7, ou cellule hôte selon la revendication 8, pour son utilisation en tant que médicament.

13. Composition pharmaceutique, molécule bifonctionnelle, acide nucléique ou groupe de molécules d'acide nucléique, vecteur, ou cellule hôte, pour son utilisation selon la revendication 12, pour son utilisation dans le traitement d'un cancer, de préférence d'un cancer choisi dans le groupe constitué par une malignité hématologique ou une tumeur solide avec expression de PD-1 et/ou PD-L1, tel qu'un cancer choisi dans le groupe constitué par les néoplasmes hématolymphoïdes, un lymphome à cellules T angioimmunoblastique, un syndrome myélodysplasique, et une leucémie myéloïde aiguë, un cancer induit par un virus ou associé à une immunodéficience tel qu'un cancer choisi dans le groupe constitué par un sarcome de Kaposi (par exemple associé à un herpès virus lié au sarcome de Kaposi), les cancers à cellules squameuses du col, anaux, péniens et vulvaires et les cancers oropharyngés (par exemple associés au virus du papillome humain) ; les lymphomes non hodgkiniens à cellules B (NHL), y compris un lymphome diffus à grandes cellules B, un lymphome de Burkitt, un lymphome plasmablastique, un lymphome du système nerveux central primaire, un lymphome primitif à épanchement lié au HHV-8, un lymphome hodgkinien classique, et des troubles lymphoprolifératifs (par exemple associés au virus d'Epstein-Barr (EBV) et/ou à l'herpès virus lié au sarcome de Kaposi) ; un carcinome hépatocellulaire (par exemple associé aux virus des hépatites B et/ou C) ; un carcinome à cellules de Merkel (par exemple associé au polyomavirus des cellules de Merkel (MPV)) ; et un cancer associé à une infection par le virus de l'immunodéficience humaine (VIH) ; et un cancer choisi dans le groupe constitué par un mélanome, un mésothéliome malin, un cancer du poumon non à petites cellules, un carcinome à cellules rénales, un lymphome hodgkinien, un cancer de la tête et du cou, un carcinome urothélial, un cancer colorectal, un carcinome hépatocellulaire, un cancer du poumon à petites cellules, un carcinome à cellules de Merkel métastasique, un cancer gastrique ou gastro-œsophagien, et un cancer du col, métastasique ou non métastasique.

14. Composition pharmaceutique, molécule bifonctionnelle, acide nucléique ou groupe de molécules d'acide nucléique, vecteur, ou cellule hôte, pour son utilisation selon la revendication 12 ou 13, pour son utilisation en combinaison avec une radiothérapie ou un agent thérapeutique additionnel de préférence choisi dans le groupe constitué par les agents d'alkylation, les inhibiteurs d'angiogenèse, les anticorps, les antimétabolites, les antimitotiques, les antiprolifératifs, les antiviraux, les inhibiteurs de kinase Aurora, les promoteurs d'apoptose (par exemple les inhibiteurs de la famille de la Bcl-2), les activateurs de la voie des récepteurs de mort, les inhibiteurs de kinase Bcr-Abl, les anticorps BiTE (engageant les cellules T bispécifiques), les conjugués anticorps-médicament, les modificateurs de réponse biologique, les inhibiteurs de tyrosine kinase de Bruton (BTK), les inhibiteurs de kinase dépendant de la cycline, les inhibiteurs du cycle cellulaire, les inhibiteurs de cyclooxygénase-2, les DVD, les inhibiteurs de récepteurs d'homologues aux oncogènes viraux de la leucémie (ErbB2), les inhibiteurs de facteur de croissance, les inhibiteurs de la protéine de choc thermique (HSP)-90, les inhibiteurs de l'histone désacétylase (HDAC), les hormonothérapies, les immunologiques, les inhibiteurs d'inhibiteurs de protéines de l'apoptose (IAP), les antibiotiques d'intercalation, les inhibiteurs de kinase, les inhibiteurs de kinésine, les inhibiteurs de Jak2, les inhibiteurs de cible mammifère de la rapamycine, les microARN, les inhibiteurs de kinases régulées par un signal extracellulaire activé par un mitogène, les protéines de liaison multivalentes, les anti-inflammatoires non stéroïdiens (AINS), les inhibiteurs de poly[ADP (adénosine diphosphate)ribose] polymérase (PARP), les chimiothérapeutiques au platine, les inhibiteurs de kinase Polo-like (Plk), les inhibiteurs de phosphoinositide-3 kinase (PI3K), les inhibiteurs de protéasome, les analogues de purine, les analogues de pyrimidine, les inhibiteurs de récepteurs de tyrosine kinase, les alcaloïdes végétaux rétinoïdes/deltoïdes, les petits acides ribonucléiques inhibiteurs (ARNsi), les inhibiteurs de topoisomérase, les inhibiteurs d'ubiquitine ligase, les agents d'hypométhylation, les inhibiteurs de point de contrôle, les vaccins peptidiques et analogues, les épitopes ou néoépitopes issus d'antigènes tumoraux, ainsi que les combinaisons d'un ou plusieurs de ces agents.

15. Composition pharmaceutique, molécule bifonctionnelle, acide nucléique ou groupe de molécules d'acide nucléique, vecteur, ou cellule hôte, pour son utilisation selon la revendication 12, pour son utilisation dans le traitement d'une maladie infectieuse, de préférence d'une maladie infectieuse chronique, mieux encore d'une infection virale chronique, de préférence d'une maladie infectieuse causée par un virus choisi dans le groupe constitué par un VIH, un virus de l'hépatite, un herpès virus, un adénovirus, un virus grippal, un flavivirus, un échovirus, un rhinovirus, un virus Coxsackie, un coronavirus, un virus respiratoire syncytial, le virus des oreillons, un rotavirus, le virus de la rougeole, le virus de la rubéole, un parvovirus, le virus de la vaccine, le virus HTLV, un virus de la dengue, un papillomavirus, le virus molluscum, le poliovirus, le virus de la rage, le virus JC et un virus d'encéphalite arbovirale.
